# EUROPEAN PATENT SPECIFICATION

(11) **EP 4 090 772 B1**
(45) Date of publication and mention of the grant of the patent: **25.11.2026**
(21) Application number: 21741305.3
(22) Date of filing: 15.01.2021
(51) Int. Cl.: C12Q 1/6886

(54) **METHODS FOR DIAGNOSING HEPATOCELLULAR CARCINOMA**
VERFAHREN ZUR DIAGNOSE VON HEPATOZELLULÄREM KARZINOM
MÉTHODES DE DIAGNOSTIC DU CARCINOME HÉPATOCELLULAIRE

(30) Priority: 17.01.2020 US 202062962437 P
(43) Date of publication of application: 23.11.2022
(73) Proprietor: The Board of Trustees of the Leland Stanford Junior University, Stanford, CA 94305-2038 (US)
(72) Inventor: PLEVRITIS, Sylvia K., Palo Alto, California 94306-2100 (US); WANG, Shan X., Palo Alto, California 94306-2100 (US); PATNAIK, Ritish, Palo Alto, California 94306-2100 (US); YU, Alice, Palo Alto, California 94306-2100 (US)
(74) Representative: Bassil, Nicholas Charles
(86) International application number: PCT/US2021/013656
(87) International publication number: WO 2021/146570

(56) References cited:
- WO-A1-2018/045322
- WO-A2-02/18632
- CA-A1- 3 094 717
- US-A1- 2017 356 051
- US-A1- 2018 066 320
- MIN-AE SONG ET AL: "Elucidating the Landscape of Aberrant DNA Methylation in Hepatocellular Carcinoma", PLOS ONE, vol. 8, no. 2, 20 February 2013 (2013-02-20), pages e55761, XP055173243, DOI: 10.1371/journal.pone.0055761
- ZHANG CHENG ET AL: "Meta-analysis of DNA methylation biomarkers in hepatocellular carcinoma", ONCOTARGET, vol. 7, no. 49, 8 November 2016 (2016-11-08), pages 81255 - 81267, XP093070957, DOI: 10.18632/oncotarget.13221

## Description

### BACKGROUND OF THE INVENTION

90% of hepatocellular carcinomas (HCCs) have underlying cirrhosis, which is often due to increased liver fibrosis from viral hepatitis or non-alcoholic steatohepatitis (NASH) (Zhang & Friedman Hepatology 56, 769-775 (2012)). Surveillance of cirrhosis patients with high-sensitivity, rule-out diagnostic tests is crucial for diagnosing HCCs for earlier treatment with improved outcomes. While current standard-of-care alpha-fetoprotein (AFP) tests demonstrate high specificity (90%) at a clinically-established 20 ng/mL cutoff, they unfortunately also exhibit low sensitivity (59%) (Marrero et al. Gastroenterology 137, 110-118 (2009)). WO 2018/045322 A1 relates to technology for hepatocellular carcinoma screening and to methods, compositions, and related uses for detecting the presence of hepatocellular carcinoma. CA 3094717 A1 describes a cancer assay panel for targeted detection of cancer-specific methylation patterns, including methods of designing, making, and using the cancer assay panel for diagnosis of cancer. WO 02/18632 A2 relates to a method for detecting the degree of methylation of a defined cytosine in the sequence context 5'-CpG-3' of a genomic DNA sample. Min-Ae Song et al (2013), PLoS ONE: 8,2, e55761 relates to the landscape of aberrant DNA methylation in hepatocellular carcinoma. Zhang Cheng et al (2016), Oncotarget: 7, 49, 81255-81267 describes a meta-analysis of DNA methylation biomarkers in hepatocellular carcinoma.

### SUMMARY OF THE INVENTION

Compositions, methods, and kits are provided for diagnosing hepatocellular carcinoma (HCC) in patients. In particular, methylated cell-free DNA biomarkers and methods of using them to determine if a patient has hepatocellular carcinoma are provided. Additionally, the methylated cell-free DNA biomarkers can be used to distinguish between patients with a chronic liver disease such as cirrhosis who do not have hepatocellular carcinoma and those patients with a chronic liver disease who have hepatocellular carcinoma. The identified biomarkers can be used alone or in combination with one or more additional biomarkers or relevant clinical parameters in prognosis, diagnosis, therapy selection, or monitoring treatment of HCC.

In one aspect of the invention, a method of diagnosing and treating hepatocellular carcinoma (HCC) in a patient is provided, the method comprising:
detecting methylation at one or more CpG sites in each of the following genes from circulating free DNA (cfDNA) in a sample: SPINT2, RUNX3, PRDM2, APC, GSTP1, WIF1, SEPT9, HOXA1, PFKP, and AK055957, wherein increased frequency of methylation at the one or more CpG sites in the genes consisting of SPINT2, RUNX3, PRDM2, APC, GSTP1, WIF1, SEPT9, HOXA1, PFKP, and AK055957 in the cfDNA sample from the patient compared to reference value ranges for frequency of methylation at the one or more CpG sites in a control cfDNA sample indicates that the patient has a positive diagnosis for the HCC, wherein the one or more CpG sites are selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof.

In certain embodiments, the patient has a condition or disease that makes the patient more susceptible to developing HCC. In some embodiments, the patient has a liver disease. Exemplary liver diseases include, but are not limited to, liver cirrhosis, fatty liver disease, alcoholic hepatitis, non-alcoholic steatohepatitis, autoimmune hepatitis, drug-induced hepatitis, viral hepatitis, a hepatitis A virus infection, a hepatitis B virus infection, a hepatitis C virus infection, a hepatitis D virus infection, a hepatitis E virus infection, hereditary hemochromatosis, Wilson disease, primary biliary cirrhosis, and α-1-antitrypsin deficiency.

The one or more CpG sites are selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof (locations of these CpG sites are given in the Illumina HumanMethylation450K Manifest). In some embodiments, the method comprises measuring the frequency of methylation at the cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188 CpG sites in the cfDNA.

A patient having a positive diagnosis for HCC based on the frequency of methylation of the CpG sites may be treated with an anti-cancer therapy. Exemplary methods for treating HCC include, without limitation, surgical resection of an HCC tumor, radiofrequency ablation (RFA) of an HCC tumor, cryoablation of an HCC tumor, percutaneous injection of an HCC tumor with ethanol or acetic acid, transcatheter arterial chemoembolization (TACE), selective internal radiation therapy (SIRT), liver transplantation, high intensity focused ultrasound, external beam therapy, portal vein embolization, radionuclide therapy (e.g., yttrium-90, lodine-131, rhenium-188, or holmium-166), chemotherapy (e.g., cisplatin, gemcitabine, oxaliplatin, doxorubicin, 5-fluorouracil, capecitabine, or mitoxantrone), targeted therapy (e.g., sorafenib, regorafenib, lenvatinib, cabozantinib, ramucirumab, nivolumab, or pembrolizumab), immunotherapy, or biologic therapy, or a combination thereof.

Any suitable method may be used for detecting methylation at CpG sites in the cfDNA. Exemplary techniques include, without limitation, methylation-sensitive arbitrarily-primed polymerase chain reaction (MS AP-PCR), methylation-sensitive single nucleotide primer extension (Ms-SNuPE), methylation-specific PCR (MSP), methylation-sensitive DNA restriction enzyme analysis, restriction enzyme-based sequencing, restriction enzyme-based microarray analysis, combined bisulfite restriction analysis (COBRA), methylated CpG island amplification (MCA), methylated CpG island amplification and microarray (MCAM), Hpall tiny fragment enrichment by ligation-mediated PCR (HELP), bisulfite sequencing, bisulfite microarray analysis, methylation-specific pyrosequencing, HELP-sequencing (HELP-seq), TET-assisted pyridine borane sequencing (TAPS), Glal hydrolysis and ligation adapter dependent PCR (GLAD-PCR), methylated DNA immunoprecipitation-sequencing (MeDIP-Seq), or methylated DNA immunoprecipitation-microarray analysis (MeDIP-chip), Southern blotting with methyl-sensitive restriction enzymes, and methylation-specific giant magnetoresistive sensor-based microarray analysis.

In certain embodiments, the method further comprises calculating an HCC risk score based on the methylation frequency at the CpG sites in the SPINT2, RUNX3, PRDM2, APC, GSTP1, WIF1, SEPT9, HOXA1, PFKP, and AK055957 genes of the cfDNA using one or more algorithms. In some embodiments, the method further comprises calculating a geometric mean score for the methylation frequency of the CpG sites in the cfDNA for the SPINT2, RUNX3, PRDM2, APC, GSTP1, WIF1, SEPT9, HOXA1, PFKP, and AK055957 genes and comparing the geometric mean score of the patient to a reference geometric mean score (layered analysis for methylated biomarkers (LAMB)-HCC gene methylation score) for diagnosis of the HCC.

In certain embodiments, the method further comprises measuring blood levels of alpha-fetoprotein (AFP), wherein detection of increased blood levels of AFP in combination with increased frequency of methylation at the one or more CpG sites in the genes consisting of SPINT2, RUNX3, PRDM2, APC, GSTP1, WIF1, SEPT9, HOXA1, PFKP, and AK055957 compared to reference value ranges for blood levels of AFP and frequency of methylation at the one or more CpG sites in the genes consisting of SPINT2, RUNX3, PRDM2, APC, GSTP1, WIF1, SEPT9, HOXA1, PFKP, and AK055957 for a control subject indicate that the patient has a positive diagnosis for the HCC.

In certain embodiments, the cfDNA sample is a blood sample or plasma sample comprising cfDNA.

In another aspect of the invention, a method of monitoring HCC in a patient is provided, the method comprising:
detecting methylation at one or more CpG sites in each of the following genes from circulating free DNA (cfDNA) in a first blood sample at a first time point and a second blood sample at a second time point: SPINT2, RUNX3, PRDM2, APC, GSTP1, WIF1, SEPT9, HOXA1, PFKP, and AK055957, wherein detection of increased frequency of methylation of the CpG sites in the genes consisting of SPINT2, RUNX3, PRDM2, APC, GSTP1, WIF1, SEPT9, HOXA1, PFKP, and AK055957 in the cfDNA of the second blood sample compared to the cfDNA of the first blood sample indicate that the HCC is progressing, and detection of decreased frequency of methylation of the CpG sites in the genes consisting of SPINT2, RUNX3, PRDM2, APC, GSTP1, WIF1, SEPT9, HOXA1, PFKP, and AK055957 in the cfDNA of the second blood sample compared to the cfDNA of the first blood sample indicate that the HCC is not progressing, wherein the one or more CpG sites are selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof. In some embodiments, the method further comprises repeating the method steps.

In certain embodiments, the HCC is a primary tumor, a metastasis, or a recurrence.

In certain embodiments, the first time point is before a treatment of the patient for HCC is started and the second time point is during or after the treatment. For example, the method can be used to monitor the efficacy of a treatment including, without limitation, surgical resection of an HCC tumor, radiofrequency ablation (RFA) of an HCC tumor, cryoablation of an HCC tumor, percutaneous injection of an HCC tumor with ethanol or acetic acid, transcatheter arterial chemoembolization (TACE), selective internal radiation therapy (SIRT), liver transplantation, high intensity focused ultrasound, external beam therapy, portal vein embolization, radionuclide therapy, chemotherapy, targeted therapy, immunotherapy, or biologic therapy, or a combination thereof. In some embodiments, the method further comprises increasing dosage or frequency of a treatment for HCC, changing to a different treatment, or starting palliative care for the patient if the HCC is progressing.

In certain embodiments, the method further comprises measuring blood levels of alpha-fetoprotein (AFP), wherein detection of increased blood levels of AFP in combination with increased frequency of methylation at the one or more CpG sites in the genes consisting of SPINT2, RUNX3, PRDM2, APC, GSTP1, WIF1, SEPT9, HOXA1, PFKP, and AK055957 in the second blood sample compared to the first blood sample indicate that the HCC is progressing; and decreased blood levels of AFP in combination with decreased frequency of methylation at the one or more CpG sites in the genes consisting of SPINT2, RUNX3, PRDM2, APC, GSTP1, WIF1, SEPT9, HOXA1, PFKP, and AK055957 in the second blood sample compared to the first blood sample indicate that the HCC is not progressing.

In another aspect of the invention, a method of monitoring for a recurrence of hepatocellular carcinoma (HCC) in a patient is provided, the method comprising:
a) detecting methylation at one or more CpG sites in promoter regions of biomarker genes in circulating free DNA (cfDNA) from a first cfDNA sample after treatment for a previous occurrence at a first time point when the patient is characterized as cancer-free from imaging or other diagnostic modalities, wherein the biomarker genes comprise each of AK055957, APC, GSTP1, HOXA1, PFKP, PRDM2, RUNX3, SEPTIN9, SPINT2, and WIF1;
b) detecting methylation at the one or more CpG sites in the promoter regions of the biomarker genes in cfDNA from a second cfDNA sample at a second time point during a period of monitoring for the recurrence, wherein the biomarker genes comprise each of AK055957, APC, GSTP1, HOXA1, PFKP, PRDM2, RUNX3, SEPTIN9, SPINT2, and WIF1, wherein increased frequency of methylation at the one or more CpG sites in the promoter regions of the biomarker genes comprising each of AK055957, APC, GSTP1, HOXA1, PFKP, PRDM2, RUNX3, SEPTIN9, SPINT2, and WIF1 in the cfDNA of the second cfDNA sample compared to the cfDNA of the first cfDNA sample indicates that the HCC has recurred; and
c) repeating step b subsequently during the period of monitoring for the recurrence, and wherein the one or more CpG sites are selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof.

In certain embodiments, the patient is treated for the recurrence of HCC by surgical resection of an HCC tumor, radiofrequency ablation (RFA) of an HCC tumor, cryoablation of an HCC tumor, percutaneous injection of an HCC tumor with ethanol or acetic acid, transcatheter arterial chemoembolization (TACE), selective internal radiation therapy (SIRT), liver transplantation, high intensity focused ultrasound, external beam therapy, portal vein embolization, radionuclide therapy, chemotherapy, targeted therapy, immunotherapy, or biologic therapy, or a combination thereof.

In certain embodiments, the method further comprises measuring blood levels of alpha-fetoprotein (AFP) for the patient, wherein increased blood levels of AFP in combination with increased frequency of methylation at the one or more CpG sites in the genes consisting of SPINT2, RUNX3, PRDM2, APC, GSTP1, WIF1, SEPT9, HOXA1, PFKP, and AK055957 in the cfDNA from the patient compared to reference value ranges for blood levels of AFP and frequency of methylation at the one or more CpG sites in the genes consisting of SPINT2, RUNX3, PRDM2, APC, GSTP1, WIF1, SEPT9, HOXA1, PFKP, and AK055957 indicate that the patient has a positive diagnosis for the recurrence of HCC.

In another aspect of the invention, a kit is provided comprising agents for detecting methylation of CpG sites in SPINT2, RUNX3, PRDM2, APC, GSTP1, WIF1, SEPT9, HOXA1, PFKP, and AK055957 genes in cfDNA. The kit may further include instructions for diagnosing hepatocellular carcinoma (HCC) in a patient according to the methods described herein. In certain embodiments, the kit further comprises agents for performing methylation-sensitive arbitrarily-primed polymerase chain reaction (MS AP-PCR), methylation-sensitive single nucleotide primer extension (Ms-SNuPE), methylation-specific PCR (MSP), methylation-sensitive DNA restriction enzyme analysis, restriction enzyme-based sequencing, restriction enzyme-based microarray analysis, combined bisulfite restriction analysis (COBRA), methylated CpG island amplification (MCA), methylated CpG island amplification and microarray (MCAM), Hpall tiny fragment enrichment by ligation-mediated PCR (HELP), bisulfite sequencing, bisulfite microarray analysis, methylation-specific pyrosequencing, HELP-sequencing (HELP-seq), TET-assisted pyridine borane sequencing (TAPS), Glal hydrolysis and ligation adapter dependent PCR (GLAD-PCR), methylated DNA immunoprecipitation-sequencing (MeDIP-Seq), or methylated DNA immunoprecipitation-microarray analysis (MeDIP-chip), Southern blotting with methyl-sensitive restriction enzymes, or methylation-specific giant magnetoresistive sensor-based microarray analysis. In some embodiments, the kit comprises a bisulfite reagent, methylation-sensitive restriction enzymes, PCR primers that selectively amplify DNA regions that contain CpG dinucleotides, methylation-specific primers, methylation-specific probes, or a combination thereof.

In certain embodiments, the kit comprises at least one probe comprising a sequence selected from the group consisting of SEQ ID NOS:1-432.

In another aspect of the invention, an *in vitro* method of diagnosing hepatocellular carcinoma (HCC) in a patient is provided, the method comprising:
detecting methylation at one or more CpG sites in genes from a circulating free DNA (cfDNA) sample, wherein the genes comprise each of SPINT2, RUNX3, PRDM2, APC, GSTP1, WIF1, SEPT9, HOXA1, PFKP, and AK055957, wherein increased frequency of methylation at the one or more CpG sites in the genes comprising each of SPINT2, RUNX3, PRDM2, APC, GSTP1, WIF1, SEPT9, HOXA1, PFKP, and AK055957 from the cfDNA sample from the patient compared to reference value ranges for frequency of methylation at the one or more CpG sites in the cfDNA for a control cfDNA sample indicates that the patient has a positive diagnosis for the HCC, wherein the one or more CpG sites are selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof.

Also described herein is a sample comprising cell-free DNA methylated at one or more CpG sites in each of the following genes: SPINT2, RUNX3, PRDM2, APC, GSTP1, WIF1, SEPT9, HOXA1, PFKP, and AK055957, wherein the one or more CpG sites are selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof for use as a biomarker for diagnosis of hepatocellular carcinoma (HCC) in a patient, detecting recurrence of HCC, or monitoring treatment of HCC.

### BRIEF DESCRIPTION OF THE DRAWINGS

FIG. 1 shows the layered analysis for methylated biomarkers (LAMB). A meta-analysis of 3099 paired HCCs and ANTs was used to identify hypermethylated genes. Candidate gene promoters were screened in microarrays of 153 paired HCCs and ANTs for differentially methylated CpGs by methylation frequency (β) and area under the receiver operating characteristic curve (AUC) - a metric for a biomarker's predictive ability. Sites were then screened in microarrays of 1722 control blood samples and 159 demographically matched HCCs by AUC and β values for CpGs differentially methylated in blood (LAMB-HCC).

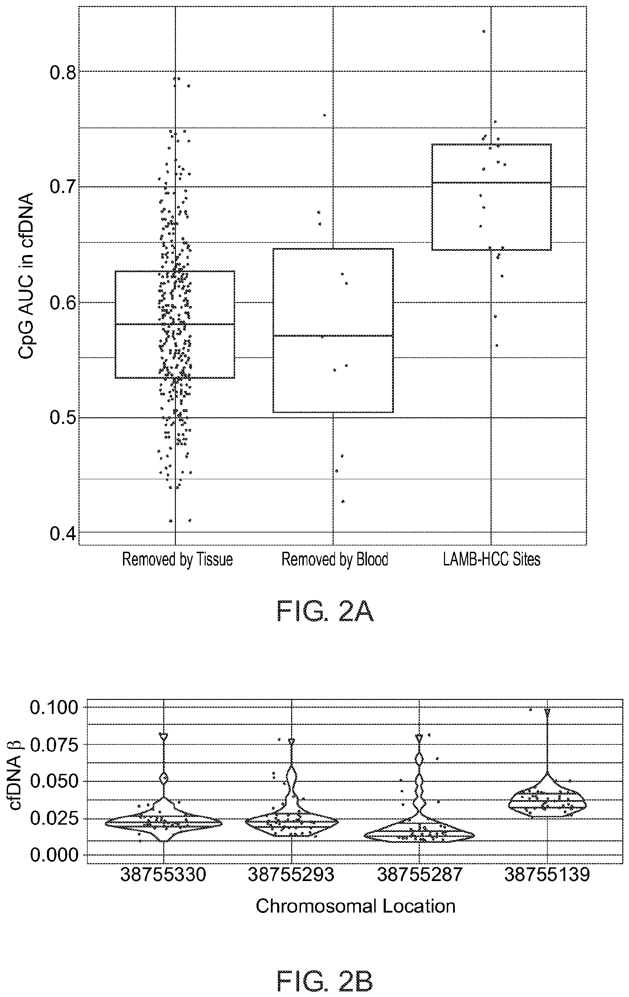

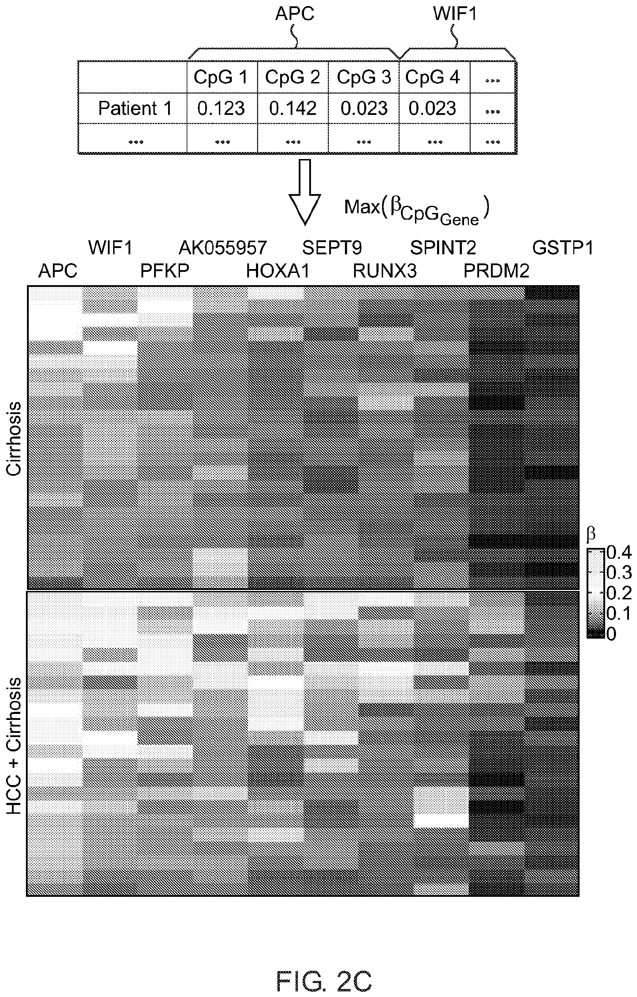

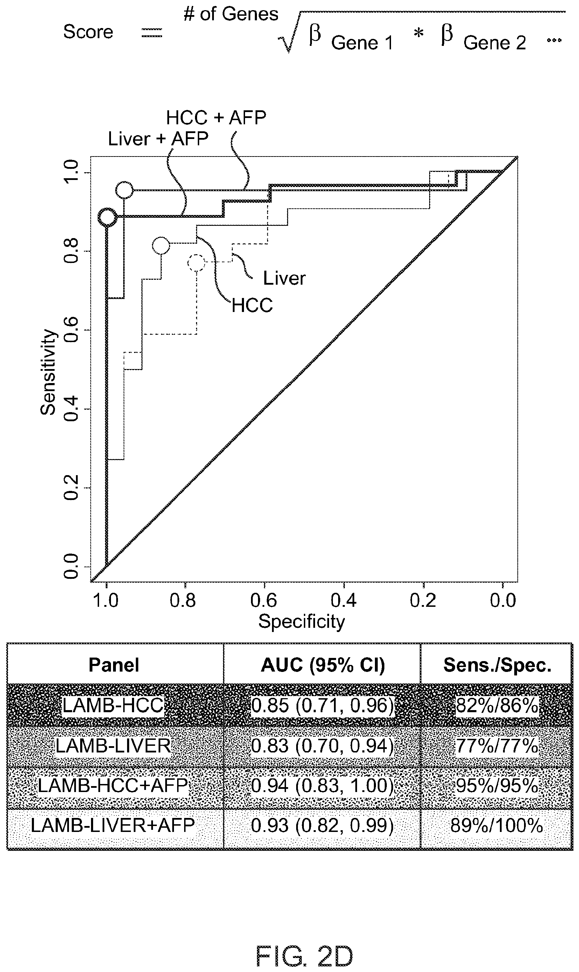

FIGS. 2A-2D show LAMB performance in an external cfDNA validation dataset. FIG. 2A, AUCs of CpGs screened by LAMB (n = 22 cirrhosis, 22 HCC with cirrhosis). FIG. 2B, Methylation frequency (β) distributions for 4 SPINT2 CpGs in a 200 bp span. FIG. 2C, Gene methylation mapping diagram and resulting gene methylation profiles for the 44 cfDNA samples. FIG. 2D, Geometric mean scoring formula, receiver-operating characteristic curves, and performance statistics for tested panels. Circles show reported sensitivities and specificities. The LAMB-LIVER panel contains 6 sites hypomethylated in colorectal, pancreatic, and lung tumors (β < 0.2).

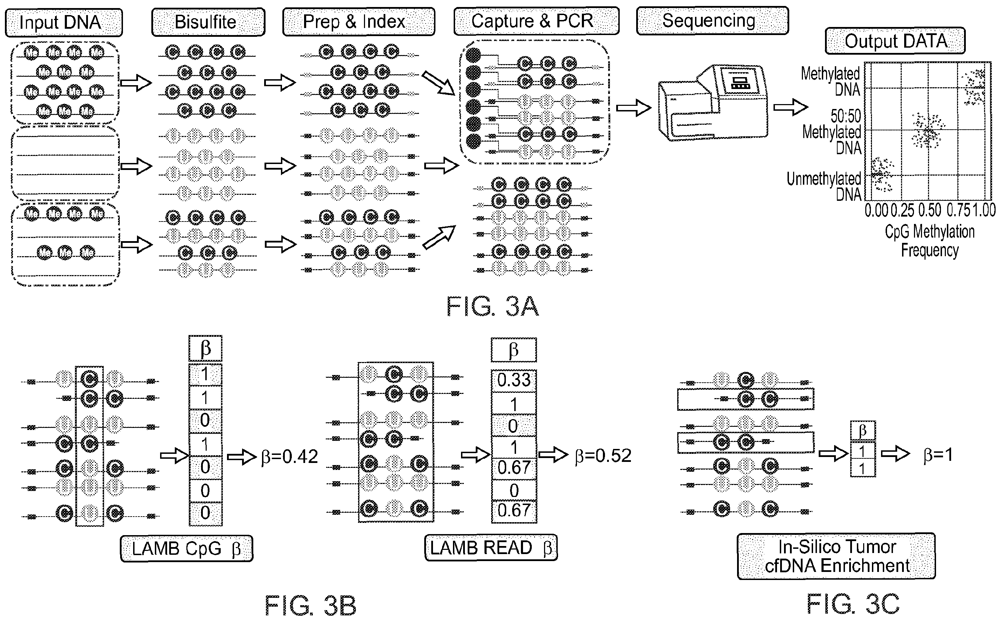

FIGS. 3A-3C show LAMB-HCC Targeted Bisulfite Sequencing Assay. FIG. 3A, The LAMB-HCC targeted bisulfite sequencing assay is comprised of bisulfite conversion, post-bisulfite conversion adaptor tagging (PBAT) library preparation, sample-specific indexing, pooled hybridization capture with probes for flanking regions of LAMB CpGs (total: ~6KB), pooled PCR amplification, and deep sequencing (100X+ depth). Testing methylated, unmethylated, and 50:50 methylated/unmethylated sheared genomic DNA will reveal sequencing bias. FIG. 3B, Sequencing data from patient samples will be analyzed with the methylation profiles shown in Figure 2b and new 10-gene methylation profiles from the methylation frequencies (β) of all CpGs in each read. FIG. 3C, Enrichment for tumor cfDNA via in-silico size selection (90 to 150 bp) will also be tested.
FIG. 4 shows flow diagram for selecting tissue studies for meta-analysis.
FIG. 5 shows Forest Plots and count data for genes identified by meta-analysis.
FIG. 6 shows gene methylation frequencies for HCC and cirrhosis cfDNA.
FIG. 7 shows geometric mean scores for tested panels with optimal cut-offs.
FIG. 8 shows LAMB + AFP screening workflow.

### DETAILED DESCRIPTION OF THE INVENTION

Compositions, methods, and kits are provided for diagnosing hepatocellular carcinoma in patients. In particular, methylated cell-free DNA biomarkers and methods of using them to determine if a patient has hepatocellular carcinoma are provided.

Before the present compositions, methods, and kits are described, it is to be understood that this invention is not limited to particular methods or compositions described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, some potential and preferred methods and materials are now described.

Any recited method can be carried out in the order of events recited or in any other order which is logically possible.

It must be noted that as used herein and in the appended claims, the singular forms "a", "an", and "the" include plural referents unless the context clearly dictates otherwise. Thus, for example, reference to "a biomarker" includes a plurality of such biomarkers and reference to "the cfDNA" includes reference to one or more cfDNAs and equivalents thereof, known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### Definitions

The term "sample" as used herein relates to a material or mixture of materials, typically, although not necessarily, in liquid form, containing one or more analytes of interest.

As used herein, the term "circulating cell-free DNA" refers to DNA that is circulating in the peripheral blood of a patient. The DNA molecules in cell-free DNA may have a median size that is below 1 kb (e.g., in the range of 50 bp to 500 bp, 80 bp to 400 bp, or 100-1,000 bp), although fragments having a median size outside of this range may be present. Cell-free DNA may contain circulating tumor DNA (ctDNA), i.e., tumor DNA circulating freely in the blood of a cancer patient or circulating fetal DNA (if the subject is a pregnant female). cfDNA can be highly fragmented and in some cases can have a mean fragment size about 165-250 bp (Newman et al Nat Med. 2014 20: 548-54). cfDNA can be obtained by centrifuging whole blood to remove all cells, and then isolating the DNA from the remaining plasma or serum. Such methods are well known (see, e.g., Lo et al, Am J Hum Genet 1998; 62:768-75). Circulating cell-free DNA is double-stranded, but can be made single stranded by denaturation.

*Biomarkers.* The term "biomarker" as used herein refers to a compound, such as cfDNA, a protein, a mRNA, a metabolite, or a metabolic byproduct which is differentially expressed or present at different concentrations, levels or frequencies in one sample compared to another, such as a biological sample (e.g., blood or tissue sample) from patients who have cancer compared to a biological sample from healthy control subjects (i.e., subjects not having cancer). Biomarkers include, but are not limited to, hepatocellular carcinoma (HCC) biomarkers including cfDNA methylated at one or more CpG sites in one or more biomarker genes selected from SPINT2, RUNX3, PRDM2, APC, GSTP1, WIF1, SEPT9, HOXA1, PFKP, and AK055957. Biomarkers include cfDNA with an increased frequency or level of methylation at one or more CpG sited selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof (methylated CpG sites for each biomarker gene are listed in Table 2).

In some embodiments, the concentration, frequency, or level of a biomarker is determined before and after the administration of a treatment to a patient. The treatment may comprise, for example, without limitation, surgical resection of an HCC tumor, radiofrequency ablation (RFA) of an HCC tumor, cryoablation of an HCC tumor, percutaneous injection of an HCC tumor with ethanol or acetic acid, transcatheter arterial chemoembolization (TACE), selective internal radiation therapy (SIRT), liver transplantation, high intensity focused ultrasound, external beam therapy, portal vein embolization, radionuclide therapy (e.g., yttrium-90, lodine-131, rhenium-188, or holmium-166), chemotherapy (e.g., cisplatin, gemcitabine, oxaliplatin, doxorubicin, 5-fluorouracil, capecitabine, or mitoxantrone), targeted therapy (e.g., sorafenib, regorafenib, lenvatinib, cabozantinib, ramucirumab, nivolumab, or pembrolizumab), immunotherapy, or biologic therapy, or a combination thereof, if the patient is diagnosed with HCC. The degree of change in the concentration, frequency, or level of a biomarker, or lack thereof, is interpreted as an indication of whether the treatment has the desired effect (e.g., anti-tumor activity). In other words, the concentration or level of a biomarker is determined before and after the administration of the treatment to an individual, and the degree of change, or lack thereof, in the level is interpreted as an indication of whether the individual is "responsive" to the treatment.

A "reference level" or "reference value" of a biomarker means a level of the biomarker that is indicative of a particular disease state, phenotype, or predisposition to developing a particular disease state or phenotype, or lack thereof, as well as combinations of disease states, phenotypes, or predisposition to developing a particular disease state or phenotype, or lack thereof. A "positive" reference level of a biomarker means a level that is indicative of a particular disease state or phenotype. A "negative" reference level of a biomarker means a level that is indicative of a lack of a particular disease state or phenotype. A "reference level" of a biomarker may be an absolute or relative amount or concentration of the biomarker, a presence or absence of the biomarker, a range of amount or concentration of the biomarker, a minimum and/or maximum amount or concentration of the biomarker, a mean amount or concentration of the biomarker, and/or a median amount or concentration of the biomarker; and, in addition, "reference levels" of combinations of biomarkers may also be ratios of absolute or relative amounts or concentrations of two or more biomarkers with respect to each other. Appropriate positive and negative reference levels of biomarkers for a particular disease state, phenotype, or lack thereof may be determined by measuring levels of desired biomarkers in one or more appropriate subjects, and such reference levels may be tailored to specific populations of subjects (e.g., a reference level may be age-matched or gender-matched so that comparisons may be made between biomarker levels in samples from subjects of a certain age or gender and reference levels for a particular disease state, phenotype, or lack thereof in a certain age or gender group). Such reference levels may also be tailored to specific techniques that are used to measure levels of biomarkers in samples (e.g., methylation-specific polymerase chain reaction (PCR), quantitative methylation-specific PCR, methylation-sensitive DNA restriction enzyme analysis, methylation-specific pyrosequencing, or bisulfite genomic sequencing), where the levels of biomarkers may differ based on the specific technique that is used.

A "similarity value" is a number that represents the degree of similarity between two things being compared. For example, a similarity value may be a number that indicates the overall similarity between a patient's biomarker profile using specific phenotype-related biomarkers and reference value ranges for the biomarkers in one or more control samples or a reference profile (e.g., the similarity to an "HCC" cfDNA methylation profile). The similarity value may be expressed as a similarity metric, such as a correlation coefficient, or may simply be expressed as the difference in cfDNA methylation frequency or levels, or geometric mean scores for gene methylation frequency for methylated cfDNA biomarkers in a patient sample compared to a control cfDNA sample or reference cfDNA methylation profile.

The terms "quantity", "amount", and "level" are used interchangeably herein and may refer to an absolute quantification of a molecule or an analyte in a sample, or to a relative quantification of a molecule or analyte in a sample, i.e., relative to another value such as relative to a reference value as taught herein, or to a range of values for the biomarker. These values or ranges can be obtained from a single patient or from a group of patients.

The term "cfDNA sample" with respect to an individual encompasses samples such as blood or plasma samples comprising cfDNA obtained from the individual. The cfDNA samples can be obtained by any suitable method such as by venipuncture. The definition also includes samples that have been manipulated in any way after their procurement, such as by treatment with reagents, washed, centrifuged, or enriched for particular types of molecules (e.g., methylated cfDNA biomarkers).

*Obtaining and assaying a sample.* The term "assaying" is used herein to include the physical steps of manipulating a sample to generate data related to the sample. As will be readily understood by one of ordinary skill in the art, a sample must be "obtained" prior to assaying the sample. Thus, the term "assaying" implies that the sample has been obtained. The terms "obtained" or "obtaining" as used herein encompass the act of receiving an extracted or isolated sample. For example, a testing facility can "obtain" a sample in the mail (or via delivery, etc.) prior to assaying the sample. In some such cases, the sample was "extracted" or "isolated" from an individual by another party prior to mailing (i.e., delivery, transfer, etc.), and then "obtained" by the testing facility upon arrival of the sample. Thus, a testing facility can obtain the sample and then assay the sample, thereby producing data related to the sample.

The terms "obtained" or "obtaining" as used herein can also include the physical extraction or isolation of a sample from a subject. Accordingly, a sample can be isolated from a subject (and thus "obtained") by the same person or same entity that subsequently assays the sample. When a sample is "extracted" or "isolated" from a first party or entity and then transferred (e.g., delivered, mailed, etc.) to a second party, the sample was "obtained" by the first party (and also "isolated" by the first party), and then subsequently "obtained" (but not "isolated") by the second party. Accordingly, in some embodiments, the step of obtaining does not comprise the step of isolating a sample.

In some embodiments, the step of obtaining comprises the step of isolating a sample (e.g., a pre-treatment sample, a post-treatment sample, etc.). Methods and protocols for isolating various samples (e.g., a blood sample, a serum sample, a plasma sample, a biopsy sample, an aspirate, etc.) will be known to one of ordinary skill in the art and any convenient method may be used to isolate a sample.

It will be understood by one of ordinary skill in the art that in some cases, it is convenient to wait until multiple samples (e.g., a pre-treatment sample and a post-treatment sample) have been obtained prior to assaying the samples. Accordingly, in some cases an isolated sample (e.g., a pre-treatment sample, a post-treatment sample, etc.) is stored until all appropriate samples have been obtained. One of ordinary skill in the art will understand how to appropriately store a variety of different types of samples and any convenient method of storage may be used (e.g., refrigeration) that is appropriate for the particular sample. In some embodiments, a pre-treatment sample is assayed prior to obtaining a post-treatment sample. In some cases, a pre-treatment sample and a post-treatment sample are assayed in parallel. In some cases, multiple different post-treatment samples and/or a pre-treatment sample are assayed in parallel. In some cases, samples are processed immediately or as soon as possible after they are obtained.

The terms "determining", "measuring", "evaluating", "assessing," "assaying," and "analyzing" are used interchangeably herein to refer to any form of measurement, and include determining if an element is present or not. These terms include both quantitative and/or qualitative determinations. Assaying may be relative or absolute. For example, "assaying" can be determining whether the methylation level or frequency is less than or "greater than or equal to" a particular threshold, (the threshold can be pre-determined or can be determined by assaying a control sample). On the other hand, "assaying to determine the methylation level" can mean determining a quantitative value (using any convenient metric) that represents the level of methylation at a CpG site. The level of methylation can be expressed in arbitrary units associated with a particular assay (e.g., fluorescence units, e.g., mean fluorescence intensity (MFI)), or can be expressed as an absolute value with defined units (e.g., number of methylated CpG sites in a cfDNA gene, frequency of methylation at a CpG site in cfDNA, etc.). Additionally, the level of methylation at a CpG site can be compared to the methylation level of one or more additional CpG sites to derive a normalized value that represents a normalized methylation level. The specific metric (or units) chosen is not crucial as long as the same units are used (or conversion to the same units is performed) when evaluating multiple samples from the same individual (e.g., samples taken at different points in time from the same individual). This is because the units cancel when calculating a fold-change (i.e., determining a ratio) in the methylation level from one sample to the next (e.g., samples taken at different points in time from the same individual).

As used herein, "methylation" refers to cytosine methylation at positions C5 or N4 of cytosine, the N6 position of adenine, or other types of nucleic acid methylation. *In vitro* amplified DNA is usually unmethylated because typical *in vitro* DNA amplification methods do not retain the methylation pattern of the amplification template. However, "unmethylated DNA" or "methylated DNA" can also refer to amplified DNA whose original template was unmethylated or methylated, respectively.

Accordingly, as used herein a "methylated nucleotide" or a "methylated nucleotide base" refers to the presence of a methyl moiety on a nucleotide base, where the methyl moiety is not present in a recognized typical nucleotide base. For example, cytosine does not contain a methyl moiety on its pyrimidine ring, but 5-methylcytosine contains a methyl moiety at position 5 of its pyrimidine ring. Therefore, cytosine is not a methylated nucleotide and 5-methylcytosine is a methylated nucleotide. In another example, thymine contains a methyl moiety at position 5 of its pyrimidine ring; however, for purposes herein, thymine is not considered a methylated nucleotide when present in DNA since thymine is a typical nucleotide base of DNA.

As used herein, a "methylated nucleic acid molecule" refers to a nucleic acid molecule that contains one or more methylated nucleotides.

As used herein, a "methylation state", "methylation profile", and "methylation status" of a nucleic acid molecule refers to the presence of absence of one or more methylated nucleotide bases in the nucleic acid molecule. For example, a nucleic acid molecule containing a methylated cytosine is considered methylated (e.g., the methylation state of the nucleic acid molecule is methylated). A nucleic acid molecule that does not contain any methylated nucleotides is considered unmethylated.

The methylation state of a particular nucleic acid sequence (e.g., a gene marker or DNA region as described herein) can indicate the methylation state of every base in the sequence or can indicate the methylation state of a subset of the bases (e.g., of one or more cytosines) within the sequence, or can indicate information regarding regional methylation density within the sequence with or without providing precise information of the locations within the sequence the methylation occurs.

The methylation state of a nucleotide locus in a nucleic acid molecule refers to the presence or absence of a methylated nucleotide at a particular locus in the nucleic acid molecule. For example, the methylation state of a cytosine at the 7^{th} nucleotide in a nucleic acid molecule is methylated when the nucleotide present at the 7^{th} nucleotide in the nucleic acid molecule is 5-methylcytosine. Similarly, the methylation state of a cytosine at the 7^{th} nucleotide in a nucleic acid molecule is unmethylated when the nucleotide present at the 7^{th} nucleotide in the nucleic acid molecule is cytosine (and not 5-methylcytosine).

The methylation status can optionally be represented or indicated by a "methylation value" (e.g., representing a methylation frequency, fraction, ratio, percent, etc.). A methylation value can be generated, for example, by quantifying the amount of intact nucleic acid present following restriction digestion with a methylation dependent restriction enzyme or by comparing amplification profiles after bisulfite reaction or by comparing sequences of bisulfite-treated and untreated nucleic acids. Accordingly, a value, e.g., a methylation value, represents the methylation status and can thus be used as a quantitative indicator of methylation status across multiple copies of a locus. This is of particular use when it is desirable to compare the methylation status of a sequence in a sample to a threshold or reference value.

As used herein, "methylation frequency" or "methylation percent (%)" refer to the number of instances in which a molecule or locus is methylated relative to the number of instances the molecule or locus is unmethylated.

As such, the methylation state describes the state of methylation of a nucleic acid (e.g., a genomic sequence). In addition, the methylation state refers to the characteristics of a nucleic acid segment at a particular genomic locus relevant to methylation. Such characteristics include, but are not limited to, whether any of the cytosine (C) residues within this DNA sequence are methylated, the location of methylated C residue(s), the frequency or percentage of methylated C throughout any particular region of a nucleic acid, and allelic differences in methylation due to, e.g., difference in the origin of the alleles. The terms "methylation state", "methylation profile", and "methylation status" also refer to the relative concentration, absolute concentration, or pattern of methylated C or unmethylated C throughout any particular region of a nucleic acid in a biological sample. For example, if the cytosine (C) residue(s) within a nucleic acid sequence are methylated it may be referred to as "hypermethylated" or having "increased methylation", whereas if the cytosine (C) residue(s) within a DNA sequence are not methylated it may be referred to as "hypomethylated" or having "decreased methylation". Likewise, if the cytosine (C) residue(s) within a nucleic acid sequence are methylated as compared to another nucleic acid sequence (e.g., from a different region or from a different individual, etc.) that sequence is considered hypermethylated or having increased methylation compared to the other nucleic acid sequence. Alternatively, if the cytosine (C) residue(s) within a DNA sequence are not methylated as compared to another nucleic acid sequence (e.g., from a different region or from a different individual, etc.) that sequence is considered hypomethylated or having decreased methylation compared to the other nucleic acid sequence. Additionally, the term "methylation pattern" as used herein refers to the collective sites of methylated and unmethylated nucleotides over a region of a nucleic acid. Two nucleic acids may have the same or similar methylation frequency or methylation percent but have different methylation patterns when the number of methylated and unmethylated nucleotides are the same or similar throughout the region but the locations of methylated and unmethylated nucleotides are different. Sequences are said to be "differentially methylated" or as having a "difference in methylation" or having a "different methylation state" when they differ in the extent (e.g., one has increased or decreased methylation relative to the other), frequency, or pattern of methylation. The term "differential methylation" refers to a difference in the level or pattern of nucleic acid methylation in a cancer positive sample as compared with the level or pattern of nucleic acid methylation in a cancer negative sample. It may also refer to the difference in levels or patterns between patients that have recurrence of cancer after surgery versus patients who not have recurrence. Differential methylation and specific levels or patterns of DNA methylation are prognostic and predictive biomarkers, e.g., once the correct cut-off or predictive characteristics have been defined.

The methylation state frequency can be used to describe a population of individuals or a sample from a single individual. For example, a nucleotide locus having a methylation state frequency of 50% is methylated in 50% of instances and unmethylated in 50% of instances. Such a frequency can be used, for example, to describe the degree to which a nucleotide locus or nucleic acid region is methylated in a population of individuals or a collection of nucleic acids. Thus, when methylation in a first population or pool of nucleic acid molecules is different from methylation in a second population or pool of nucleic acid molecules, the methylation state frequency of the first population or pool will be different from the methylation state frequency of the second population or pool. Such a frequency also can be used, for example, to describe the degree to which a nucleotide locus or nucleic acid region is methylated in a single individual. For example, such a frequency can be used to describe the degree to which a group of cells from a tissue sample are methylated or unmethylated at a nucleotide locus or nucleic acid region.

As used herein a "nucleotide locus" refers to the location of a nucleotide in a nucleic acid molecule. A nucleotide locus of a methylated nucleotide refers to the location of a methylated nucleotide in a nucleic acid molecule.

Typically, methylation of human DNA occurs on a dinucleotide sequence including an adjacent guanine and cytosine where the cytosine is located 5' of the guanine (also termed CpG dinucleotide sequences). Most cytosines within the CpG dinucleotides are methylated in the human genome, however some remain unmethylated in specific CpG dinucleotide rich genomic regions, known as CpG islands (see, e.g., Antequera et al. (1990) Cell 62: 503-514).

As used herein, a "CpG island" refers to a G:C-rich region of genomic DNA containing an increased number of CpG dinucleotides relative to total genomic DNA. A CpG island can be at least 100, 200, or more base pairs in length, where the G:C content of the region is at least 50% and the ratio of observed CpG frequency over expected frequency is 0.6; in some instances, a CpG island can be at least 500 base pairs in length, where the G:C content of the region is at least 55%) and the ratio of observed CpG frequency over expected frequency is 0.65. The observed CpG frequency over expected frequency can be calculated according to the method provided in Gardiner-Garden et al (1987) J. Mol. Biol. 196: 261-281. For example, the observed CpG frequency over expected frequency can be calculated according to the formula R=(A×B)/(C×D), where R is the ratio of observed CpG frequency over expected frequency, A is the number of CpG dinucleotides in an analyzed sequence, B is the total number of nucleotides in the analyzed sequence, C is the total number of C nucleotides in the analyzed sequence, and D is the total number of G nucleotides in the analyzed sequence. Methylation state is typically determined in CpG islands, e.g., at promoter regions. It will be appreciated though that other sequences in the human genome are prone to DNA methylation such as CpA and CpT (see, e.g., Ramsahoye (2000) Proc. Natl. Acad. Sci. USA 97: 5237-5242; Salmon and Kaye (1970) Biochim. Biophys. Acta. 204: 340-351; Grafstrom (1985) Nucleic Acids Res. 13: 2827-2842; Nyce (1986) Nucleic Acids Res. 14: 4353-4367; Woodcock (1987) Biochem. Biophys. Res. Commun. 145: 888-894).

As used herein, a reagent that modifies a nucleotide of the nucleic acid molecule as a function of the methylation state of the nucleic acid molecule, or a methylation-specific reagent, refers to a compound or composition or other agent that can change the nucleotide sequence of a nucleic acid molecule in a manner that reflects the methylation state of the nucleic acid molecule. Methods of treating a nucleic acid molecule with such a reagent can include contacting the nucleic acid molecule with the reagent, coupled with additional steps, if desired, to accomplish the desired change of nucleotide sequence. Such a change in the nucleic acid molecule's nucleotide sequence can result in a nucleic acid molecule in which each methylated nucleotide is modified to a different nucleotide. Such a change in the nucleic acid nucleotide sequence can result in a nucleic acid molecule in which each unmethylated nucleotide is modified to a different nucleotide. Such a change in the nucleic acid nucleotide sequence can result in a nucleic acid molecule in which each of a selected nucleotide which is unmethylated (e.g., each unmethylated cytosine) is modified to a different nucleotide. Use of such a reagent to change the nucleic acid nucleotide sequence can result in a nucleic acid molecule in which each nucleotide that is a methylated nucleotide (e.g., each methylated cytosine) is modified to a different nucleotide. As used herein, use of a reagent that modifies a selected nucleotide refers to a reagent that modifies one nucleotide of the four typically occurring nucleotides in a nucleic acid molecule (C, G, T, and A for DNA and C, G, U, and A for RNA), such that the reagent modifies the one nucleotide without modifying the other three nucleotides. In one exemplary embodiment, such a reagent modifies an unmethylated selected nucleotide to produce a different nucleotide. In another exemplary embodiment, such a reagent can deaminate unmethylated cytosine nucleotides. An exemplary reagent is bisulfite.

As used herein, the term "bisulfite reagent" refers to a reagent comprising in some embodiments bisulfite, disulfite, hydrogen sulfite, or combinations thereof to distinguish between methylated and unmethylated cytidines, e.g., in CpG dinucleotide sequences.

The term "methylation assay" refers to any assay or method for determining the methylation state of one or more CpG dinucleotide sequences within a sequence of a nucleic acid. Exemplary methylation assays include, without limitation, methylation-sensitive arbitrarily-primed polymerase chain reaction (MS AP-PCR), methylation-sensitive single nucleotide primer extension (Ms-SNuPE), methylation-specific PCR (MSP), methylation-sensitive DNA restriction enzyme analysis, restriction enzyme-based sequencing, restriction enzyme-based microarray analysis, combined bisulfite restriction analysis (COBRA), methylated CpG island amplification (MCA), methylated CpG island amplification and microarray (MCAM), Hpall tiny fragment enrichment by ligation-mediated PCR (HELP), bisulfite sequencing, bisulfite microarray analysis, methylation-specific pyrosequencing, HELP-sequencing (HELP-seq), TET-assisted pyridine borane sequencing (TAPS), Glal hydrolysis and ligation adapter dependent PCR (GLAD-PCR), methylated DNA immunoprecipitation-sequencing (MeDIP-Seq), or methylated DNA immunoprecipitation-microarray analysis (MeDIP-chip), Southern blotting with methyl-sensitive restriction enzymes, and methylation-specific giant magnetoresistive sensor-based microarray analysis.

Bisulfite sequencing uses bisulfite treatment of DNA before sequencing to detect methylation sites. Treatment of DNA with bisulfite converts cytosine residues to uracil, but does not affect methylated cytosine residues. After bisulfite treatment, the only cytosines remaining in the DNA are methylated cytosines. Thus, sequencing the DNA after bisulfite treatment reveals the methylation status of individual cytosine residues at single-nucleotide resolution.

The MS AP-PCR assay uses methylation-sensitive restriction enzymes to digest DNA and PCR with CG-rich primers to selectively amplify regions that contain CpG dinucleotides (see, e.g., Gonzalgo et al. (1997) Cancer Research 57: 594-599).

The MethyLight assay uses bisulfite-dependent, quantitative fluorescence-based real-time PCR with methylation-specific priming and methylation-specific fluorescent probing for detection of DNA methylation. Digital MethyLight combines the MethyLight assay with digital PCR to allow detection of individual methylated molecules (see, e.g., Eads et al. (1999) Cancer Res. 59: 2302-2306, Campan et al. (2018) Methods Mol. Biol. 1708:497-513).

The HeavyMethy assay uses methylation specific blocking probes (also referred to herein as blockers) covering CpG positions between, or covered by, amplification primers to enable methylation-specific selective amplification of a nucleic acid sample.

The HeavyMethyl MethyLight assay is a variation of the MethyLight^{™} assay, wherein the MethylLight^{™} assay is combined with methylation specific blocking probes covering CpG positions between the amplification primers.

The Ms-SNuPE assay uses bisulfite treatment of DNA combined with PCR using primers designed to hybridize immediately upstream of the CpG site(s) and electrophoresis of amplicons on polyacrylamide gels for visualization and quantitation. Treatment of DNA (genomic or cfDNA) with sodium bisulfite causes unmethylated cytosines to be converted to uracils, During the PCR step, uracil is replicated as thymine, and methylcytosine is replicated as cytosine during amplification. The ratio of methylated versus unmethylated cytosine (C versus T) at the original CpG sites can be determined by incubating the gel-isolated PCR product, primer(s), and Taq polymerase with either [³²P]dCTP or [³²P]TTP followed by denaturing polyacrylamide gel electrophoresis and phosphorimage analysis. Ms-SNuPE primers can also be designed to incorporate either [³²P]dATP or [³²P]dGTP into the opposite strand to assess methylation status depending on which CpG site is analyzed (see, e.g., Gonzalgo & Jones (1997) Nucleic Acids Res. 25: 2529-2531; Gonzalgo et al. (2007) Nat. Protoc. 2(8):1931-6).

The MSP assay uses bisulfite treatment of DNA for conversion of non-methylated cytosines to uracils and subsequent amplification with primers specific for methylated versus unmethylated DNA (see, e.g., Herman et al. (1996) Proc. Natl. Acad. Sci. USA 93: 9821-9826, and U.S. Pat. No. 5,786,146).

The COBRA assay uses bisulfite treatment of DNA for conversion of non-methylated cytosines to uracils, locus-specific PCR amplification of the bisulfite-converted DNA, restriction digestion, electrophoresis an analysis of restriction patterns on a gel (see, e.g., Xiong & Laird (1997) Nucleic Acids Res. 25: 2532-2534; Bilichak et al. (2017) Methods Mol. Biol. 1456:63-71).

The MCA assay uses methylation-sensitive restriction enzymes to digest DNA, followed by adaptor ligation and PCR to selectively amplify methylated CpG-rich sequences (see, e.g., Toyota et al. (1999) Cancer Res. 59: 2307-12, and WO 00/26401A1).

The MCAM assay uses MCA in combination with a CpG island microarray to detect DNA methylation in a high-throughput fashion (see, e.g., Estecio et al. (2007) Genome Res. 17(10):1529-1536).

The HELP assay uses the methylation-sensitive restriction enzyme, Hpall, to cut DNA, and a methylation-insensitive isoschizomer, Mspl, as a control. Microarray analysis is performed with microarrays containing probes designed to detect the Hpall/Mspl fragments. HELP-seq combines the HELP assay with massively parallel sequencing of DNA methylation sites (see, e.g., Greally (2018) Methods Mol. Biol. 1708:191-207; Suzuki et al. (2010) Methods 52(3):218-22).

The GLAD-PCR assay uses a site-specific methyl-directed DNA-endonucleases that cleave only methylated DNA, followed by ligation of DNA fragments to universal adapters for high-throughput PCR (see, e.g., Malyshev et al. (2020) Acta Naturae 12(3):124-133; Russian Patent RU 2525710).

The MeDIP assay uses an antibody against 5-methylcytosine to immunoprecipitate methylated DNA fragments. This technique can be combined with high-throughput DNA detection methods using microarray hybridization (MeDIP-chip) or next-generation sequencing (MeDIP-seq). See, e.g., Weber et al. (2005) Nat. Genet. 37 (8): 853-862, Palmke et al. (2011) Methods 53(2):175-184, Quackenbush et al. (2008) Cancer Res. 68(6):1786-1796, Zhu et al. (2019) Analyst 144(6):1904-1915, Yang et al. (2014) Life Sci. 113(1-2):45-54.

TET-assisted pyridine borane sequencing (TAPS) uses the ten-eleven translocation (TET) enzyme to catalyze oxidation of 5-methylcytosine and 5-hydroxymethylcytosine to 5-carboxylcytosine, followed by pyridine borane reduction to produce dihydrouracil. Unmodified cytosine is not affected. See, e.g., Liu et al. (2019) Nat Biotechnol. 37:424-429.

Methylation-specific giant magnetoresistive sensor-based microarray analysis combines methylation specific PCR and melt curve analysis on a giant magnetoresistive (GMR) biosensor. The GMR biosensor comprises synthetic DNA probes that target methylated or unmethylated CpG sites in the PCR amplicons. After hybridization of the PCR amplicons to the GMR biosensor, the difference in melting temperature (Tm) between the two types of probes is measured. See, e.g., Rizzi et al. (2017) ACS Nano. 11(9): 8864-8870, Nesvet et al. (2019) Biosens Bioelectron 124-125:136-142.

Southern Blotting can also be used to detect DNA methylation. The DNA is first digested with methylation-sensitive restriction enzymes, and the restriction fragments are analyzed by Southern Blot.

As used herein, a "selected nucleotide" refers to one nucleotide of the four typically occurring nucleotides in a nucleic acid molecule (C, G, T, and A for DNA and C, G, U, and A for RNA), and can include methylated derivatives of the typically occurring nucleotides (e.g., when C is the selected nucleotide, both methylated and unmethylated C are included within the meaning of a selected nucleotide), whereas a methylated selected nucleotide refers specifically to a methylated typically occurring nucleotide and an unmethylated selected nucleotides refers specifically to an unmethylated typically occurring nucleotide.

The terms "methylation-specific restriction enzyme" or "methylation-sensitive restriction enzyme" refers to an enzyme that selectively digests a nucleic acid dependent on the methylation state of its recognition site. In the case of a restriction enzyme that specifically cuts if the recognition site is not methylated or is hemimethylated, the cut will not take place or will take place with a significantly reduced efficiency if the recognition site is methylated. In the case of a restriction enzyme that specifically cuts if the recognition site is methylated, the cut will not take place or will take place with a significantly reduced efficiency if the recognition site is not methylated. Preferred are methylation-specific restriction enzymes, the recognition sequence of which contains a CG dinucleotide (for instance a recognition sequence such as CGCG or CCCGGG). Further preferred for some embodiments are restriction enzymes that do not cut if the cytosine in this dinucleotide is methylated at the carbon atom C5.

As used herein, a "different nucleotide" refers to a nucleotide that is chemically different from a selected nucleotide, typically such that the different nucleotide has Watson-Crick base-pairing properties that differ from the selected nucleotide, whereby the typically occurring nucleotide that is complementary to the selected nucleotide is not the same as the typically occurring nucleotide that is complementary to the different nucleotide. For example, when C is the selected nucleotide, U or T can be the different nucleotide, which is exemplified by the complementarity of C to G and the complementarity of U or T to A. As used herein, a nucleotide that is complementary to the selected nucleotide or that is complementary to the different nucleotide refers to a nucleotide that base-pairs, under high stringency conditions, with the selected nucleotide or different nucleotide with higher affinity than the complementary nucleotide's base-paring with three of the four typically occurring nucleotides. An example of complementarity is Watson-Crick base pairing in DNA (e.g., AT and C-G) and RNA (e.g., A-U and C-G). Thus, for example, G base-pairs, under high stringency conditions, with higher affinity to C than G base-pairs to G, A, or T and, therefore, when C is the selected nucleotide, G is a nucleotide complementary to the selected nucleotide.

As used herein, the "sensitivity" of a given marker refers to the percentage of samples that report a DNA methylation value above a threshold value that distinguishes between neoplastic and non-neoplastic samples. In some embodiments, a positive is defined as a histology-confirmed neoplasia that reports a DNA methylation value above a threshold value (e.g., the range associated with disease), and a false negative is defined as a histology-confirmed neoplasia that reports a DNA methylation value below the threshold value (e.g., the range associated with no disease). The value of sensitivity, therefore, reflects the probability that a DNA methylation measurement for a given marker obtained from a known diseased sample will be in the range of disease-associated measurements. As defined here, the clinical relevance of the calculated sensitivity value represents an estimation of the probability that a given marker would detect the presence of a clinical condition when applied to a subject with that condition.

As used herein, the "specificity" of a given marker refers to the percentage of non-neoplastic samples that report a DNA methylation value below a threshold value that distinguishes between neoplastic and non-neoplastic samples. In some embodiments, a negative is defined as a histology-confirmed non-neoplastic sample that reports a DNA methylation value below the threshold value (e.g., the range associated with no disease) and a false positive is defined as a histology-confirmed non-neoplastic sample that reports a DNA methylation value above the threshold value (e.g., the range associated with disease). The value of specificity, therefore, reflects the probability that a DNA methylation measurement for a given marker obtained from a known non-neoplastic sample will be in the range of non-disease associated measurements. As defined here, the clinical relevance of the calculated specificity value represents an estimation of the probability that a given marker would detect the absence of a clinical condition when applied to a patient without that condition.

The term "AUC" as used herein is an abbreviation for the "area under a curve". In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, the optimum is 1; a random test would have a ROC curve lying on the diagonal with an area of 0.5; for reference: J. P. Egan. (1975) Signal Detection Theory and ROC Analysis, Academic Press, New York).

"Diagnosis" as used herein generally includes determination as to whether a subject is likely affected by a given disease, disorder or dysfunction. The skilled artisan often makes a diagnosis on the basis of one or more diagnostic indicators, i.e., a biomarker, the presence, absence, frequency, or amount of which is indicative of the presence or absence of the disease, disorder or dysfunction.

"Prognosis" as used herein generally refers to a prediction of the probable course and outcome of a clinical condition or disease. A prognosis of a patient is usually made by evaluating factors or symptoms of a disease that are indicative of a favorable or unfavorable course or outcome of the disease. It is understood that the term "prognosis" does not necessarily refer to the ability to predict the course or outcome of a condition with 100% accuracy. Instead, the skilled artisan will understand that the term "prognosis" refers to an increased probability that a certain course or outcome will occur; that is, that a course or outcome is more likely to occur in a patient exhibiting a given condition, when compared to those individuals not exhibiting the condition.

The terms "treatment", "treating", "treat" and the like are used herein to generally refer to obtaining a desired pharmacologic and/or physiologic effect. The effect can be prophylactic in terms of completely or partially preventing a disease or symptom(s) thereof and/or may be therapeutic in terms of a partial or complete stabilization or cure for a disease and/or adverse effect attributable to the disease. The term "treatment" encompasses any treatment of a disease in a mammal, particularly a human, and includes: (a) preventing the disease and/or symptom(s) from occurring in a subject who may be predisposed to the disease or symptom but has not yet been diagnosed as having it; (b) inhibiting the disease and/or symptom(s), i.e., arresting their development; or (c) relieving the disease symptom(s), i.e., causing regression of the disease and/or symptom(s). Those in need of treatment include those already inflicted (e.g., those with HCC) as well as those in which prevention is desired (e.g., those with chronic liver disease such as liver cirrhosis or hepatitis having an increased susceptibility or an increased likelihood of HCC, those suspected of having HCC, etc.).

A therapeutic treatment is one in which the subject is inflicted prior to administration and a prophylactic treatment is one in which the subject is not inflicted prior to administration. In some embodiments, the subject has an increased likelihood of becoming inflicted or is suspected of being inflicted prior to treatment. In some embodiments, the subject is suspected of having an increased likelihood of becoming inflicted.

The term "about," particularly in reference to a given quantity, is meant to encompass deviations of plus or minus five percent.

The terms "recipient", "individual", "subject", "host", and "patient", are used interchangeably herein and refer to any mammalian subject for whom diagnosis, treatment, or therapy is desired, particularly humans. "Mammal" for purposes of treatment refers to any animal classified as a mammal, including humans, domestic and farm animals, and zoo, sports, or pet animals, such as dogs, horses, cats, cows, sheep, goats, pigs, etc. Preferably, the mammal is human.

A "therapeutically effective dose" or "therapeutic dose" is an amount sufficient to effect desired clinical results (i.e., achieve therapeutic efficacy). A therapeutically effective dose can be administered in one or more administrations.

By "isolated" is meant, when referring to a protein, polypeptide, or peptide, that the indicated molecule is separate and discrete from the whole organism with which the molecule is found in nature or is present in the substantial absence of other biological macro molecules of the same type. The term "isolated" with respect to a polynucleotide is a nucleic acid molecule devoid, in whole or part, of sequences normally associated with it in nature; or a sequence, as it exists in nature, but having heterologous sequences in association therewith; or a molecule disassociated from the chromosome.

"Providing an analysis" is used herein to refer to the delivery of an oral or written analysis (i.e., a document, a report, etc.). A written analysis can be a printed or electronic document. A suitable analysis (e.g., an oral or written report) provides any or all of the following information: identifying information of the subject (name, age, etc.), a description of what type of sample(s) was used and/or how it was used, the technique used to assay the sample, the results of the assay (e.g., the level or frequency of cfDNA CpG methylation as measured and/or the fold-change of a level or frequency of cfDNA CpG methylation over time or in a post-treatment assay compared to a pre-treatment assay), the assessment as to whether the individual is determined to have HCC or a recurrence of HCC, a recommendation for treatment (e.g., a particular anti-cancer therapy), and/or to continue or alter therapy, a recommended strategy for additional therapy, etc. The report can be in any format including, but not limited to printed information on a suitable medium or substrate (e.g., paper); or electronic format. If in electronic format, the report can be in any computer readable medium, e.g., diskette, compact disk (CD), flash drive, and the like, on which the information has been recorded. In addition, the report may be present as a website address which may be used via the internet to access the information at a remote site.

### Methylated cfDNA Biomarkers and Diagnostic Methods

Hypermethylation of CpG-islands in regulatory regions of promoters and/or the first exons in a variety of genes is associated with a variety of cancers. Layered analysis of methylated biomarkers (LAMB) was used to identify methylated cell-free DNA (cfDNA) biomarkers associated with HCC (see Examples). The identified HCC biomarkers include cfDNA methylated at one or more CpG sites in promoter regions of the genes, SPINT2, RUNX3, PRDM2, APC, GSTP1, WIF1, SEPT9, HOXA1, PFKP, and AK05595. Increased frequency or levels of methylation at CpG sites in these biomarker genes are commonly found in HCC tumors. In particular, increased frequency or levels of methylation at one or more CpG sites selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, cg26397188 (locations of these CpG sites are given in the Illumina HumanMethylation450K Manifest), and CpG sites located within 200 nucleotides thereof is associated with HCC. Accordingly, monitoring the frequency or levels of methylation of these CpG sites is useful for prognosis, diagnosis, therapy selection, and monitoring treatment of HCC.

In certain embodiments, a panel of methylated cfDNA biomarkers for use in diagnosis of HCC is provided. Biomarker panels of any size can be used in the practice of the subject methods. Biomarker panels for diagnosing HCC typically comprise at least 2 methylated cfDNA biomarkers and up to 20 methylated cfDNA biomarkers, including any number of biomarkers in between, such as 2, 3, 4, 5, 6, 7, 8, 9, 10, 11, 12, 13, 14, 15, 16, 17, 18, 19, or 20 methylated cfDNA biomarkers. In certain embodiments, the biomarker panel comprises at least 2, or at least 3, or at least 4, or at least 5, or at least 6, or at least 7, or at least 8, or at least 9, or at least 10, or at least 11, or at least 12, or at least 13, or at least 14, or at least 5, or at least 16, or at least 17, or at least 18, or at least 19, or at least 20 or more methylated cfDNA biomarkers. In some embodiments, the biomarker panel comprises or consists of cfDNA biomarkers with methylation at one or more CpG sites selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof. In some embodiments, the biomarker panel comprises or consists of cfDNA biomarkers with methylation at the CpG sites: cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188. Although smaller biomarker panels are usually more economical, larger biomarker panels (i.e., greater than 20 biomarkers) have the advantage of providing more detailed information and can also be used in the practice of the subject methods.

A sample comprising methylated cfDNA (i.e., a "cfDNA sample") is obtained from the subject. The sample is typically a blood or plasma sample comprising cfDNA taken from the subject. A "control" sample, as used herein, refers to a cfDNA sample from a subject that is not diseased. That is, a control sample is obtained from a normal or healthy subject (e.g., an individual known to not have HCC). A cfDNA sample can be obtained from a subject by conventional techniques. For example, blood samples can be obtained by venipuncture according to methods well known in the art.

When analyzing the frequency or levels of methylation at CpG sites in a cfDNA sample from a subject, the reference value ranges used for comparison can represent the frequency or levels of DNA methylation at CpG sites in a cfDNA sample from one or more subjects without HCC (i.e., normal or healthy control). Alternatively, the reference values can represent the frequency or levels of methylation at CpG sites in cfDNA samples from one or more subjects with HCC, wherein similarity to the reference value ranges indicates the subject has HCC.

In some cases, combinations of methylated cfDNA biomarkers are used in the subject methods. In some such cases, the levels of all measured biomarkers must change (as described above) in order for the diagnosis to be made. In some embodiments, only some biomarkers are used in the methods described herein. For example, a single biomarker, 2 biomarkers, 3 biomarkers, 4 biomarkers, 5 biomarkers, 6 biomarkers, 7 biomarkers, 8 biomarkers, 9 biomarkers, 10 biomarkers, 11 biomarkers, 12 biomarkers, 13 biomarkers, 14 biomarkers, 15 biomarkers, 16 biomarkers, 17 biomarkers, 18 biomarkers, 19 biomarkers, or 20 biomarkers can be used in any combination. In other embodiments, all the biomarkers are used. The quantitative values may be combined in linear or non-linear fashion to calculate one or more risk scores for HCC for the individual. In some embodiments, a geometric mean score is calculated from the gene methylation frequency profile for 2, 3, 4, 5, 6, 7, 8, 9, or all 10 of the SPINT2, RUNX3, PRDM2, APC, GSTP1, WIF1, SEPT9, HOXA1, PFKP, and AK05595 genes, wherein the geometric mean score indicates whether or not the individual has HCC. The geometric mean score may further distinguish between a subject who has HCC versus a subject who does not have HCC.

The methods described herein may be used to determine an appropriate treatment regimen for a patient and, in particular, whether a patient should be treated for HCC. For example, a patient is selected for treatment for HCC if the patient has a positive diagnosis for HCC based on a cfDNA methylation profile, as described herein. In some cases, the diagnostic methods described herein may be used by themselves or combined with medical imaging to confirm the diagnosis and further evaluate the extent of cancerous disease (how far and where the cancer has spread) to aid in determining prognosis and evaluating optimal strategies for treatment (e.g., surgery, radionuclide therapy, chemotherapy, targeted therapy, immunotherapy, biologic therapy, etc.). Exemplary medical imaging techniques include, without limitation, magnetic resonance imaging (MRI), positron emission tomography (PET), single photon emission computed tomography (SPECT), computed tomography (CT), ultrasound imaging (UI), optical imaging (OI), photoacoustic imaging (PI), fluoroscopy, and fluorescence imaging.

In some embodiments, the methylated cfDNA biomarkers are used in combination with other biomarkers for diagnosing HCC, such as alpha-fetoprotein (AFP) or des-gamma carboxyprothrombin (DCP). For example, blood levels of AFP or DCP or a combination thereof can be monitored in addition to methylation of the cfDNA biomarkers. Blood levels of AFP greater than 20 ng/ml in combination with increased frequency of methylation at the one or more CpG sites in the genes consisting of SPINT2, RUNX3, PRDM2, APC, GSTP1, WIF1, SEPT9, HOXA1, PFKP, and AK055957 in a cfDNA sample from a patient compared to reference value ranges for frequency of methylation at the one or more CpG sites in a control cfDNA sample indicate that a patient has a positive diagnosis for the HCC. Increasing levels of AFP and/or DCP indicate that HCC is progressing, whereas decreasing levels of AFP and/or DCP indicate that HCC is responding to a treatment.

Exemplary treatments for HCC include, without limitation, tumor surgical resection, radiofrequency ablation (RFA), cryoablation, percutaneous ethanol or acetic acid injection, transcatheter arterial chemoembolization (TACE), selective internal radiation therapy (SIRT), high intensity focused ultrasound, or external beam therapy, liver transplantation, portal vein embolization, or administering anti-cancer therapeutic agents such as chemotherapeutic agents (e.g., cisplatin, gemcitabine, oxaliplatin, doxorubicin, 5-fluorouracil, capecitabine, or mitoxantrone), targeted therapeutic agents (e.g., sorafenib, regorafenib, lenvatinib, or cabozantinib), immunotherapeutic agents (e.g., ramucirumab, nivolumab, or pembrolizumab), or radioisotopes (e.g., Yttrium-90, lodine-131, Rhenium-188, or Holmium-166), or a combination thereof.

The cfDNA biomarkers can be used for monitoring HCC in a patient. For example, a first cfDNA sample can be obtained from the patient at a first time point and a second cfDNA sample can be obtained from the patient at a second time point. In some embodiments, the patient is monitored for HCC by detecting methylation at one or more CpG sites in genes of the cfDNA in the first cfDNA sample and the second cfDNA sample, wherein the genes consist of SPINT2, RUNX3, PRDM2, APC, GSTP1, WIF1, SEPT9, HOXA1, PFKP, and AK055957, wherein detection of increased frequency of methylation of the CpG sites in the genes consisting of SPINT2, RUNX3, PRDM2, APC, GSTP1, WIF1, SEPT9, HOXA1, PFKP, and AK055957 in the second cfDNA sample compared to the first cfDNA sample indicate that the HCC is progressing, and detection of decreased frequency of methylation of the CpG sites in the genes consisting of SPINT2, RUNX3, PRDM2, APC, GSTP1, WIF1, SEPT9, HOXA1, PFKP, and AK055957 in the second cfDNA sample compared to the first cfDNA sample indicate that the HCC is not progressing. In some embodiments, the patient is monitored over a period of time by repeatedly collecting cfDNA samples at intervals and analyzing the cfDNA to determine whether or not the HCC is progressing. HCC at any stage of progression can be monitored, including primary tumors, metastases, or recurrences.

The subject methods are especially useful for diagnosing or monitoring a patient, as described herein, if the patient has an underlying condition or disease such as chronic liver disease, liver inflammation, or liver damage that makes the patient susceptible to developing HCC. Exemplary liver diseases that increase susceptibility to HCC include, but are not limited to, liver cirrhosis, fatty liver disease, hepatitis (e.g., alcoholic hepatitis, non-alcoholic steatohepatitis, autoimmune hepatitis, drug-induced hepatitis, or viral hepatitis), a hepatitis A virus infection, a hepatitis B virus infection, a hepatitis C virus infection, a hepatitis D virus infection, a hepatitis E virus infection, hereditary hemochromatosis, Wilson disease, primary biliary cirrhosis, and α-1-antitrypsin deficiency.

The subject methods may also be used for assaying pre-treatment and post-treatment cfDNA samples obtained from an individual to determine whether the individual is responsive or not responsive to a treatment. For example, a first cfDNA sample can be obtained from a subject before the subject undergoes the therapy, and a second cfDNA sample can be obtained from the subject after the subject undergoes the therapy. In one embodiment, the efficacy of a treatment of a patient for HCC is monitored by measuring the frequency or levels of methylation at one or more CpG sites selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof, in the first cfDNA sample and the second cfDNA sample; and evaluating the efficacy of the treatment, wherein detection of increased frequency or levels of methylation at one or more CpG sites selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof, in the second cfDNA sample compared to the first cfDNA sample indicate that the patient is worsening or not responding to the treatment, and detection of decreased frequency or levels of methylation at one or more CpG sites selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof, in the second cfDNA sample compared to the first cfDNA sample indicate that the patient is improving.

The frequency or level of methylation of a cfDNA biomarker gene in a pre-treatment sample can be referred to as a "pre-treatment value" because the first sample is isolated from the individual prior to the administration of the therapy (i.e., "pre-treatment"). The frequency or level of methylation of a cfDNA biomarker gene in the pre-treatment sample can also be referred to as a "baseline value" because this value is the value to which "post-treatment" values are compared. In some cases, the baseline value (i.e., "pre-treatment value") is determined by determining the frequency or level of methylation of a cfDNA biomarker gene in multiple (i.e., more than one, e.g., two or more, three or more, for or more, five or more, etc.) pre-treatment samples. In some cases, the multiple pre-treatment samples are isolated from an individual at different time points in order to assess natural fluctuations in biomarker levels prior to treatment. As such, in some cases, one or more (e.g., two or more, three or more, for or more, five or more, etc.) pre-treatment samples are isolated from the individual. In some embodiments, all of the pre-treatment samples will be the same type of sample (e.g., a blood sample). In some cases, two or more pre-treatment samples are pooled prior to determining the level of the biomarker in the samples. In some cases, the frequency or level of methylation of a cfDNA biomarker gene is determined separately for two or more pre-treatment samples and a "pre-treatment value" is calculated by averaging the separate measurements.

A post-treatment sample is isolated from an individual after the administration of a therapy. Thus, the frequency or level of methylation of a cfDNA biomarker gene in a post-treatment sample can be referred to as a "post-treatment value". In some embodiments, the frequency or level of methylation of a cfDNA biomarker gene is measured in additional post-treatment samples (e.g., a second, third, fourth, fifth, etc. post-treatment sample). Because additional post-treatment samples are isolated from the individual after the administration of a treatment, the levels of a biomarker in the additional samples can also be referred to as "post-treatment values."

The term "responsive" as used herein means that the treatment is having the desired effect such as an anti-tumor effect. For example, a positive therapeutic response would refer to one or more of the following improvements in the disease: (1) reduction in tumor size; (2) reduction in the number of cancer cells; (3) inhibition (i.e., slowing to some extent, preferably halting) of tumor growth; (4) inhibition (i.e., slowing to some extent, preferably halting) of cancer cell infiltration into peripheral organs; (5) inhibition (i.e., slowing to some extent, preferably halting) of tumor metastasis; and (6) some extent of relief from one or more symptoms associated with the cancer. When the individual does not improve in response to the treatment, it may be desirable to seek a different therapy or treatment regime for the individual.

The determination that an individual has HCC is an active clinical application of the correlation between the frequency or level of methylation of the cfDNA biomarker genes described herein and the disease. For example, "determining" requires the active step of reviewing the data, which is produced during the active assaying step(s), and resolving whether an individual does or does not have HCC or is responding or not responding to a therapy for treatment of HCC. Additionally, in some cases, a decision is made to proceed with the current treatment (i.e., therapy), or instead to alter the treatment. In some cases, the subject methods include the step of continuing therapy or altering therapy.

The term "continue treatment" (i.e., continue therapy) is used herein to mean that the current course of treatment (e.g., continued administration of a therapy) is to continue. If the current course of treatment is not effective in treating HCC, the treatment may be altered. "Altering therapy" is used herein to mean "discontinuing therapy" or "changing the therapy" (e.g., changing the type of treatment, changing the particular dose and/or frequency of administration of medication, e.g., increasing the dose and/or frequency). In some cases, therapy can be altered until the individual is deemed to be responsive. In some embodiments, altering therapy means changing which type of treatment is administered, discontinuing a particular treatment altogether, etc.

As a non-limiting illustrative example, a patient may be initially treated with a chemotherapeutic agent. Then to "continue treatment" would be to continue with this type of treatment. If the current course of treatment is not effective, the treatment may be altered, e.g., increasing dosage or frequency of a treatment for HCC, changing to a different treatment, or starting palliative care for the patient. Switching treatment might involve, for example, administering a different chemotherapeutic agent or administering a different type of anti-cancer therapy such as surgery, radiation therapy, immunotherapy, etc.

In other words, the frequency or level of methylation of the cfDNA biomarker genes defined herein may be monitored in order to determine when to continue therapy and/or when to alter therapy. As such, a post-treatment cfDNA sample can be isolated after any of the administrations and the cfDNA sample can be assayed to determine the frequency or level of methylation of the cfDNA biomarker genes. Accordingly, the subject methods can be used to determine whether an individual being treated for HCC is responsive or is maintaining responsiveness to a treatment.

The therapy can be administered to an individual any time after a pre-treatment cfDNA sample is isolated from the individual, but it is preferable for the therapy to be administered simultaneous with or as soon as possible (e.g., about 7 days or less, about 3 days or less, e.g., 2 days or less, 36 hours or less, 1 day or less, 20 hours or less, 18 hours or less, 12 hours or less, 9 hours or less, 6 hours or less, 3 hours or less, 2.5 hours or less, 2 hours or less, 1.5 hours or less, 1 hour or less, 45 minutes or less, 30 minutes or less, 20 minutes or less, 15 minutes or less, 10 minutes or less, 5 minutes or less, 2 minutes or less, or 1 minute or less) after a pre-treatment cfDNA sample is isolated (or, when multiple pre-treatment cfDNA samples are isolated, after the final pre-treatment cfDNA sample is isolated).

In some cases, more than one type of therapy may be administered to the individual. For example, a subject who has HCC may undergo surgical resection of a tumor followed by administration of a chemotherapeutic agent or biologic agent. Systemic therapy may be administered if the cancer spreads beyond the liver or undergoes metastasis.

In some embodiments, the methylated cfDNA biomarkers are used for monitoring for a recurrence of hepatocellular carcinoma (HCC) in a patient. For example, a first cfDNA can be obtained from the patient after treatment for a previous occurrence of HCC at a first time point when the patient is characterized as cancer-free from imaging or other diagnostic modalities. The levels or frequency of methylation at one or more CpG sites in promoter regions of biomarker genes in cfDNA from the first cfDNA sample can be measured, wherein the biomarker genes consist of AK055957, APC, GSTP1, HOXA1, PFKP, PRDM2, RUNX3, SEPTIN9, SPINT2, and WIF1. A second cfDNA sample can be obtained from the patient at a second time point during a period of monitoring for the recurrence. The levels or frequency of methylation at one or more CpG sites in promoter regions of biomarker genes in cfDNA from the second cfDNA sample can also be measured, wherein the biomarker genes consist of AK055957, APC, GSTP1, HOXA1, PFKP, PRDM2, RUNX3, SEPTIN9, SPINT2, and WIF1, wherein increased levels or frequency of methylation at the one or more CpG sites in the promoter regions of the biomarker genes consisting of AK055957, APC, GSTP1, HOXA1, PFKP, PRDM2, RUNX3, SEPTIN9, SPINT2, and WIF1 in the cfDNA of the second cfDNA sample compared to the cfDNA of the first cfDNA sample indicate that the HCC has recurred. If the patient has a positive diagnosis for the recurrence of the HCC based on the levels or frequency of methylation of the one or more CpG sites, the patient should be treated for the recurrence of the HCC. In some embodiments, the patient is monitored for a recurrence over a period of time by repeatedly collecting cfDNA samples at intervals and analyzing the cfDNA to determine whether or not the patient has a recurrence of HCC. In some embodiments, the patient is monitored for a recurrence repeatedly over a period of 1 month, 2 months, 4 months, 6 months, 8 month, 1 year, 2 years, 3 years, 4 years, 5 years, or longer by the methods described herein.

In some embodiments, the subject methods include providing an analysis indicating whether the individual is determined to have HCC or a recurrence of HCC. The analysis may further provide an analysis of whether an individual is responsive or not responsive to a treatment, or whether the individual is determined to be maintaining responsiveness or not maintaining responsiveness to a treatment for HCC. As described above, an analysis can be an oral or written report (e.g., written or electronic document). The analysis can be provided to the subject, to the subject's physician, to a testing facility, etc. The analysis can also be accessible as a website address via the internet. In some such cases, the analysis can be accessible by multiple different entities (e.g., the subject, the subject's physician, a testing facility, etc.).

### Detecting Methylation of cfDNA

Any suitable method known in the art can be used for detecting methylation at CpG sites in cfDNA. Exemplary techniques for detecting methylation include, without limitation, methylation-sensitive arbitrarily-primed polymerase chain reaction (MS AP-PCR), methylation-sensitive single nucleotide primer extension (Ms-SNuPE), methylation-specific PCR (MSP), methylation-sensitive DNA restriction enzyme analysis, restriction enzyme-based sequencing, restriction enzyme-based microarray analysis, combined bisulfite restriction analysis (COBRA), methylated CpG island amplification (MCA), methylated CpG island amplification and microarray (MCAM), Hpall tiny fragment enrichment by ligation-mediated PCR (HELP), bisulfite sequencing, bisulfite microarray analysis, methylation-specific pyrosequencing, HELP-sequencing (HELP-seq), TET-assisted pyridine borane sequencing (TAPS), Glal hydrolysis and ligation adapter dependent PCR (GLAD-PCR), methylated DNA immunoprecipitation-sequencing (MeDIP-Seq), or methylated DNA immunoprecipitation-microarray analysis (MeDIP-chip), Southern blotting with methyl-sensitive restriction enzymes, and methylation-specific giant magnetoresistive sensor-based microarray analysis.

Bisulfite sequencing uses bisulfite treatment of DNA before sequencing to detect methylation sites. Treatment of DNA with bisulfite converts cytosine residues to uracil, but does not affect methylated cytosine residues. After bisulfite treatment, the only cytosines remaining in the DNA are methylated cytosines. Thus, sequencing the DNA after bisulfite treatment reveals the methylation status of individual cytosine residues at single-nucleotide resolution (Reinders et al. (2010) Epigenomics 2(2):209-20, Chatterjee et al. (2012) Nucleic Acids Research 40(10): e79, Wreczycka et al. (2017) J. Biotechnol. 261:105-115, Shafi et al. (2018) Brief Bioinform. 19(5):737-753).

The MS AP-PCR assay uses methylation-sensitive restriction enzymes to digest DNA and PCR with CG-rich primers to selectively amplify regions that contain CpG dinucleotides (see, e.g., Gonzalgo et al. (1997) Cancer Research 57: 594-599).

The MethyLight assay uses bisulfite-dependent, quantitative fluorescence-based real-time PCR with methylation-specific priming and methylation-specific fluorescent probing for detection of DNA methylation. Digital MethyLight combines the MethyLight assay with digital PCR to allow detection of individual methylated molecules (see, e.g., Eads et al. (1999) Cancer Res. 59: 2302-2306, Campan et al. (2018) Methods Mol. Biol. 1708:497-513).

The HeavyMethy assay uses methylation specific blocking probes (also referred to herein as blockers) covering CpG positions between, or covered by, amplification primers to enable methylation-specific selective amplification of a nucleic acid sample.

The HeavyMethyl MethyLight assay is a variation of the MethyLight^{™} assay, wherein the MethylLight^{™} assay is combined with methylation specific blocking probes covering CpG positions between the amplification primers.

The Ms-SNuPE assay uses bisulfite treatment of DNA combined with PCR using primers designed to hybridize immediately upstream of the CpG site(s) and electrophoresis of amplicons on polyacrylamide gels for visualization and quantitation. Treatment of DNA (genomic or cfDNA) with sodium bisulfite causes unmethylated cytosines to be converted to uracils, During the PCR step, uracil is replicated as thymine, and methylcytosine is replicated as cytosine during amplification. The ratio of methylated versus unmethylated cytosine (C versus T) at the original CpG sites can be determined by incubating the gel-isolated PCR product, primer(s), and Taq polymerase with either [³²P]dCTP or [³²P]TTP followed by denaturing polyacrylamide gel electrophoresis and phosphorimage analysis. Ms-SNuPE primers can also be designed to incorporate either [³²P]dATP or [³²P]dGTP into the opposite strand to assess methylation status depending on which CpG site is analyzed (see, e.g., Gonzalgo & Jones (1997) Nucleic Acids Res. 25: 2529-2531; Gonzalgo et al. (2007) Nat. Protoc. 2(8):1931-6).

The MSP assay uses bisulfite treatment of DNA for conversion of non-methylated cytosines to uracils and subsequent amplification with primers specific for methylated versus unmethylated DNA (see, e.g., Herman et al. (1996) Proc. Natl. Acad. Sci. USA 93: 9821-9826, and U.S. Pat. No. 5,786,146).

The COBRA assay uses bisulfite treatment of DNA for conversion of non-methylated cytosines to uracils, locus-specific PCR amplification of the bisulfite-converted DNA, restriction digestion, electrophoresis an analysis of restriction patterns on a gel (see, e.g., Xiong & Laird (1997) Nucleic Acids Res. 25: 2532-2534; Bilichak et al. (2017) Methods Mol. Biol. 1456:63-71).

The MCA assay uses methylation-sensitive restriction enzymes to digest DNA, followed by adaptor ligation and PCR to selectively amplify methylated CpG-rich sequences (see, e.g., Toyota et al. (1999) Cancer Res. 59: 2307-12, and WO 00/26401A1).

The MCAM assay uses MCA in combination with a CpG island microarray to detect DNA methylation in a high-throughput fashion (see, e.g., Estecio et al. (2007) Genome Res. 17(10):1529-1536).

The HELP assay uses the methylation-sensitive restriction enzyme, Hpall, to cut DNA, and a methylation-insensitive isoschizomer, Mspl, as a control. Microarray analysis is performed with microarrays containing probes designed to detect the Hpall/Mspl fragments. HELP-seq combines the HELP assay with massively parallel sequencing of DNA methylation sites (see, e.g., Greally (2018) Methods Mol. Biol. 1708:191-207; Suzuki et al. (2010) Methods 52(3):218-22).

The GLAD-PCR assay uses a site-specific methyl-directed DNA-endonucleases that cleave only methylated DNA, followed by ligation of DNA fragments to universal adapters for high-throughput PCR (see, e.g., Malyshev et al. (2020) Acta Naturae 12(3):124-133; Russian Patent RU 2525710).

The MeDIP assay uses an antibody against 5-methylcytosine to immunoprecipitate methylated DNA fragments. This technique can be combined with high-throughput DNA detection methods using microarray hybridization (MeDIP-chip) or next-generation sequencing (MeDIP-seq). See, e.g., Weber et al. (2005) Nat. Genet. 37 (8): 853-862, Palmke et al. (2011) Methods 53(2):175-184, Quackenbush et al. (2008) Cancer Res. 68(6):1786-1796, Zhu et al. (2019) Analyst 144(6):1904-1915, Yang et al. (2014) Life Sci. 113(1-2):45-54.

TET-assisted pyridine borane sequencing (TAPS) uses the ten-eleven translocation (TET) enzyme to catalyze oxidation of 5-methylcytosine and 5-hydroxymethylcytosine to 5-carboxylcytosine, followed by pyridine borane reduction to produce dihydrouracil. Unmodified cytosine is not affected. See, e.g., Liu et al. (2019) Nat Biotechnol. 37:424-429.

Methylation-specific giant magnetoresistive sensor-based microarray analysis combines methylation specific PCR and melt curve analysis on a giant magnetoresistive (GMR) biosensor. The GMR biosensor comprises synthetic DNA probes that target methylated or unmethylated CpG sites in the PCR amplicons. After hybridization of the PCR amplicons to the GMR biosensor, the difference in melting temperature (Tm) between the two types of probes is measured. See, e.g., Rizzi et al. (2017) ACS Nano. 11(9): 8864-8870, Nesvet et al. (2019) Biosens Bioelectron 124-125:136-142.

Southern Blotting can also be used to detect DNA methylation. The DNA is first digested with methylation-sensitive restriction enzymes, and the restriction fragments are analyzed by Southern Blot.

Sequences are said to be "differentially methylated" or as having a "difference in methylation" or having a "different methylation state" when they differ in the extent (e.g., one has increased or decreased methylation relative to the other), frequency, or pattern of methylation. The term "differential methylation" refers to a difference in the level or pattern of nucleic acid methylation in a cancer positive sample as compared with the level or pattern of nucleic acid methylation in a cancer negative sample. It may also refer to the difference in levels or patterns between patients that have recurrence of cancer after surgery versus patients who do not have recurrence.

Methylation status can optionally be represented or indicated by a "methylation value" (e.g., representing a methylation frequency, fraction, ratio, percent, etc.). A methylation value can be generated, for example, by quantifying the amount of intact nucleic acid present following restriction digestion with a methylation dependent restriction enzyme or by comparing amplification profiles after bisulfite reaction or by comparing sequences of bisulfite-treated and untreated nucleic acids. Accordingly, a value, e.g., a methylation value, represents the methylation status and can thus be used as a quantitative indicator of methylation status across multiple copies of a locus. This is of particular use when it is desirable to compare the methylation status of a sequence in a sample to a threshold or reference value.

The methylation state may be expressed in terms of a fraction or percentage of individual strands of DNA that is methylated at a particular site relative to the total population of DNA in the sample comprising that particular site. As used herein, "methylation frequency" or "methylation percent (%)" refer to the number of instances in which a molecule or locus is methylated relative to the number of instances the molecule or locus is unmethylated.

### Data Analysis

In some embodiments, one or more pattern recognition methods can be used in analyzing the data for cfDNA methylation. The quantitative values may be combined in linear or non-linear fashion to calculate one or more risk scores for HCC for an individual. In some embodiments, measurements for a methylated cfDNA biomarker or combinations of biomarkers are formulated into linear or non-linear models or algorithms (e.g., a 'biomarker signature') and converted into a likelihood score. This likelihood score indicates the probability that a cfDNA sample is from a patient who has no evidence of disease or a patient who has HCC. A likelihood score can also be used to distinguish among different stages of cancer progression. The models and/or algorithms can be provided in machine readable format, and may be used to correlate the frequency or levels of methylation at CpG sites in cfDNA biomarker genes or a biomarker profile with a disease state, and/or to designate a treatment modality for a patient or class of patients.

Analyzing the levels of a plurality of biomarkers may comprise the use of an algorithm or classifier. In some embodiments, a machine learning algorithm is used to classify a patient as having HCC. The machine learning algorithm may comprise a supervised learning algorithm. Examples of supervised learning algorithms may include Average One-Dependence Estimators (AODE), Artificial neural network (e.g., Backpropagation), Bayesian statistics (e.g., Naive Bayes classifier, Bayesian network, Bayesian knowledge base), Case-based reasoning, Decision trees, Inductive logic programming, Gaussian process regression, Group method of data handling (GMDH), Learning Automata, Learning Vector Quantization, Minimum message length (decision trees, decision graphs, etc.), Lazy learning, Instance-based learning Nearest Neighbor Algorithm, Analogical modeling, Probably approximately correct learning (PAC) learning, Ripple down rules, a knowledge acquisition methodology, Symbolic machine learning algorithms, Subsymbolic machine learning algorithms, Support vector machines, Random Forests, Ensembles of classifiers, Bootstrap aggregating (bagging), and Boosting. Supervised learning may comprise ordinal classification such as regression analysis and Information fuzzy networks (IFN). Alternatively, supervised learning methods may comprise statistical classification, such as AODE, Linear classifiers (e.g., Fisher's linear discriminant, Logistic regression, Naive Bayes classifier, Perceptron, and Support vector machine), quadratic classifiers, k-nearest neighbor, Boosting, Decision trees (e.g., C4.5, Random forests), Bayesian networks, and Hidden Markov models.

The machine learning algorithms may also comprise an unsupervised learning algorithm. Examples of unsupervised learning algorithms may include artificial neural network, Data clustering, Expectation-maximization algorithm, Self-organizing map, Radial basis function network, Vector Quantization, Generative topographic map, Information bottleneck method, and IBSEAD. Unsupervised learning may also comprise association rule learning algorithms such as Apriori algorithm, Eclat algorithm and FP-growth algorithm. Hierarchical clustering, such as Single-linkage clustering and Conceptual clustering, may also be used. Alternatively, unsupervised learning may comprise partitional clustering such as K-means algorithm and Fuzzy clustering.

In some instances, the machine learning algorithms comprise a reinforcement learning algorithm. Examples of reinforcement learning algorithms include, but are not limited to, temporal difference learning, Q-learning and Learning Automata. Alternatively, the machine learning algorithm may comprise Data Pre-processing.

Preferably, the machine learning algorithms may include, but are not limited to, Average One-Dependence Estimators (AODE), Fisher's linear discriminant, Logistic regression, Perceptron, Multilayer Perceptron, Artificial Neural Networks, Support vector machines, Quadratic classifiers, Boosting, Decision trees, C4.5, Bayesian networks, Hidden Markov models, High-Dimensional Discriminant Analysis, and Gaussian Mixture Models. The machine learning algorithm may comprise support vector machines, Naïve Bayes classifier, k-nearest neighbor, high-dimensional discriminant analysis, or Gaussian mixture models. In some instances, the machine learning algorithm comprises Random Forests.

### Kits

Also provided are kits that can be used to detect the methylated cfDNA biomarkers described herein. Such kits can be used to diagnose a subject with HCC, detect a recurrence of HCC, therapy selection, or monitoring responses to treatment. The kit may include one or more agents for detection of methylated cfDNA biomarkers, a container for holding a biological sample comprising cfDNA (e.g., blood or plasma) isolated from a human subject suspected of having HCC; and printed instructions for reacting agents with the biological sample or a portion of the biological sample to detect the frequency or level of methylation at one or more CpG sites in cfDNA in the biological sample. The agents may be packaged in separate containers. The kit may further comprise one or more control reference samples and reagents for performing a methylation assay (e.g., bisulfite sequencing, MS AP-PCR, MethyLight^{™}, Digital MethyLight^{™}, HeavyMethyl^{™}, HeavyMethyl^{™} MethyLight^{™}, Ms-SNuPE, MSP, COBRA, MCA, MCAM, HELP, HELP-seq, GLAD-PCR, MeDIP-Seq, MeDIP-chip, and the like). For example, the subject kits may include agents for determining the frequency or level of methylation such as a bisulfite reagent, methylation-sensitive restriction enzymes, PCR primers that selectively amplify DNA regions that contain CpG dinucleotides, methylation-specific primers, methylation-specific probes, or a combination thereof.

For example, the kits can be used to detect methylation of one or more of the biomarkers described herein, which show increased frequency of methylation in cfDNA samples from patients who have HCC compared to healthy control subjects or subjects without cancer. In some embodiments, a kit comprises agents for determining the frequency or level of methylation at one or more CpG sites in promoter regions of the genes: SPINT2, RUNX3, PRDM2, APC, GSTP1, WIF1, SEPT9, HOXA1, PFKP, and AK05595. In some embodiments, the kit comprises agents for determining the frequency or level of methylation at one or more CpG sites selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof. In some embodiments, the kit comprises agents for determining the frequency or level of methylation at the CpG sites: cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188.

The kit can comprise one or more containers for compositions contained in the kit. Compositions can be in liquid form or can be lyophilized. Suitable containers for the compositions include, for example, bottles, vials, syringes, and test tubes. Containers can be formed from a variety of materials, including glass or plastic.

In addition to the above components, the subject kits may further include (in certain embodiments) instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, and the like. Yet another form of these instructions is a computer readable medium, e.g., diskette, compact disk (CD), DVD, flash drive, and the like, on which the information has been recorded. Yet another form of these instructions that may be present is a website address which may be used via the internet to access the information at a removed site.

### EXPERIMENTAL

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g., amounts, temperature, *etc.)* but some experimental errors and deviations should be accounted for. Unless indicated otherwise, parts are parts by weight, molecular weight is weight average molecular weight, temperature is in degrees Centigrade, and pressure is at or near atmospheric.

The present invention has been described in terms of particular embodiments found or proposed by the present inventor to comprise preferred modes for the practice of the invention. It will be appreciated by those of skill in the art that, in light of the present disclosure, numerous modifications and changes can be made in the particular embodiments exemplified without departing from the intended scope of the invention. For example, due to codon redundancy, changes can be made in the underlying DNA sequence without affecting the protein sequence. Moreover, due to biological functional equivalency considerations, changes can be made in protein structure without affecting the biological action in kind or amount. All such modifications are intended to be included within the scope of the appended claims.

### Example 1

### Integrating large-scale meta-analysis and CpG microarray data identifies promising cell-free DNA biomarkers for hepatocellular carcinomas in cirrhosis patients

Cancer development has been linked to the functional silencing of tumor suppressor genes through methylation of CpG dinucleotides in their promoter regions³. Detecting methylated HCC DNA in cell-free DNA (cfDNA) from cirrhosis patients has shown promising accuracy⁴⁻⁶. These studies' biomarkers have been discovered through statistical analyses (multiple-hypothesis testing, regression analysis, etc.) of samples from a small group of patients (<50). Statistical methods do not account for a biomarker's biological relevance. This limitation, in combination with the small discovery sample sizes, may cause such methods to identify biomarkers with low predictive power in other patient cohorts. To mitigate these risks, we created a physiologically-inspired biomarker discovery methodology, Layered Analysis for Methylated Biomarkers (LAMB), that incorporates methylation data from 3411 HCC patients and 1722 healthy controls to screen tumor suppressors for biologically-rooted cfDNA biomarkers.

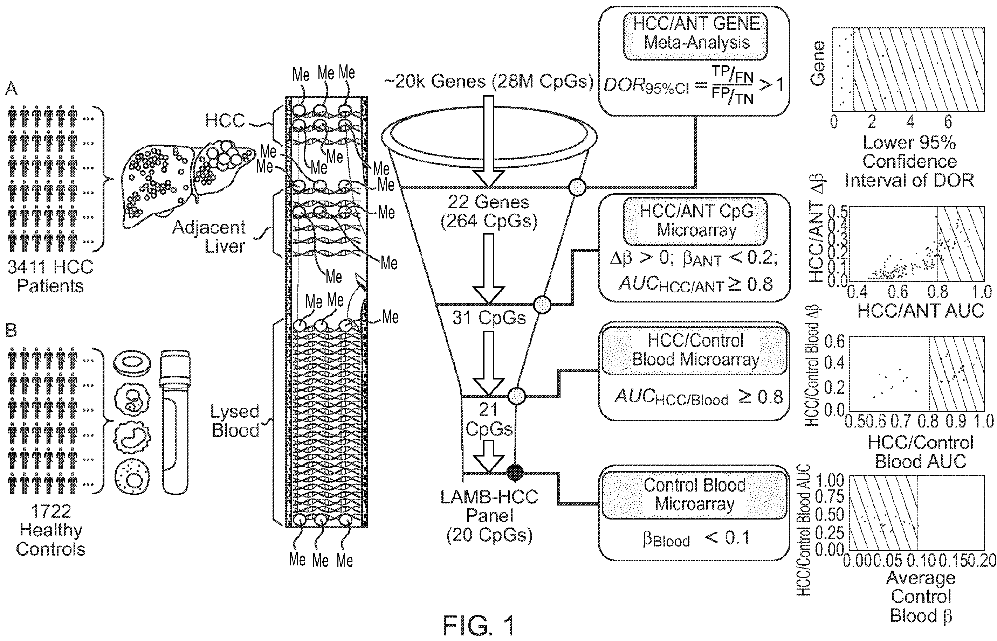

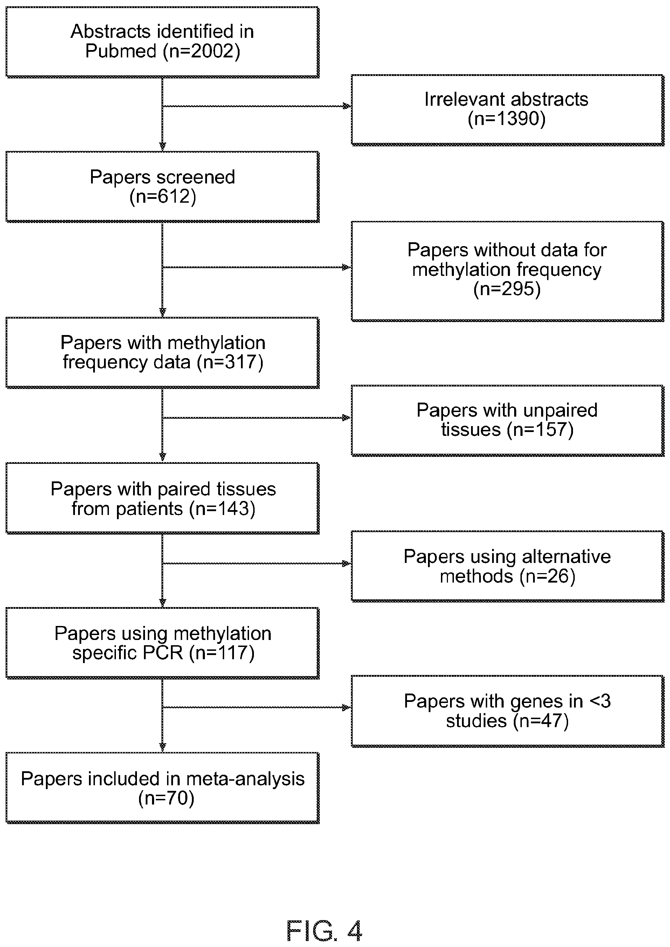

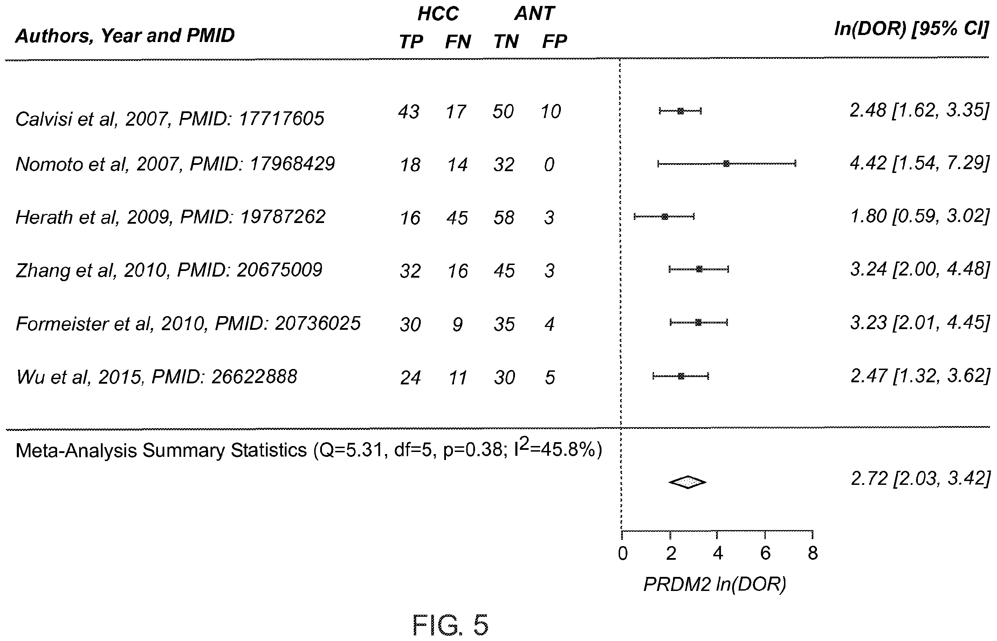

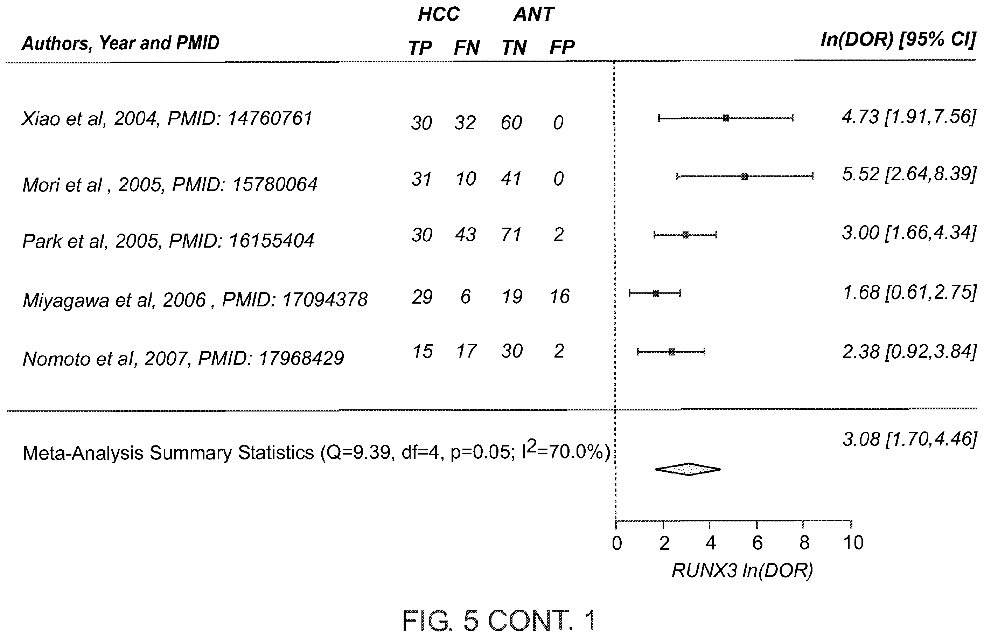

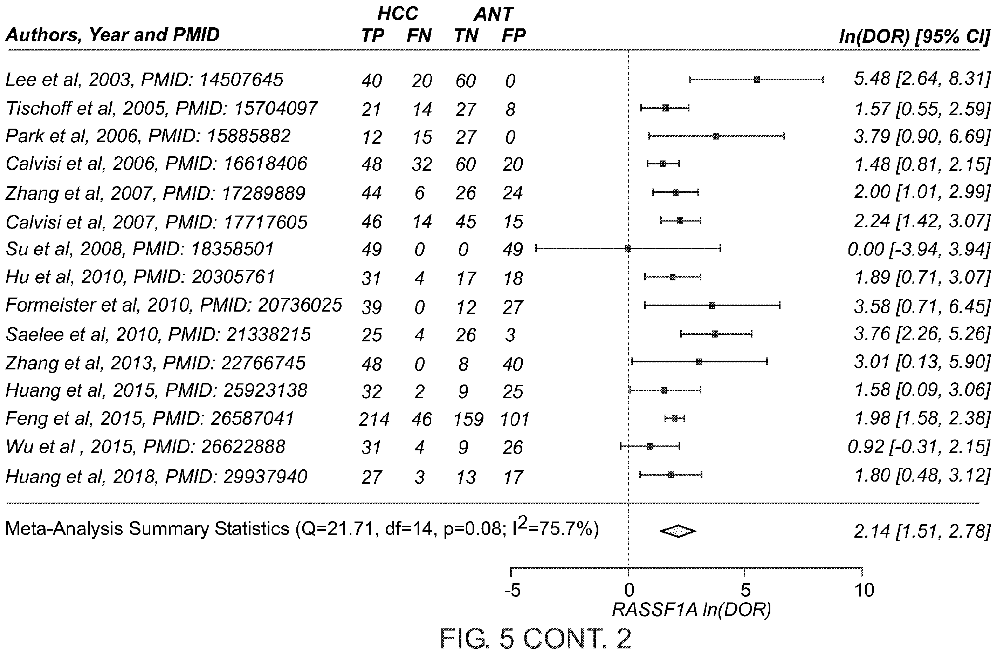

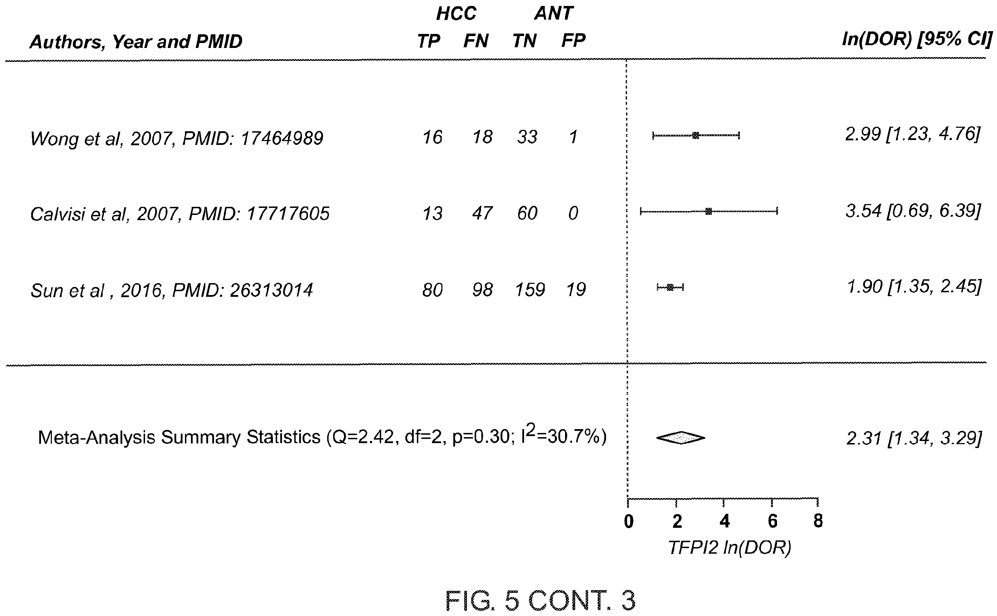

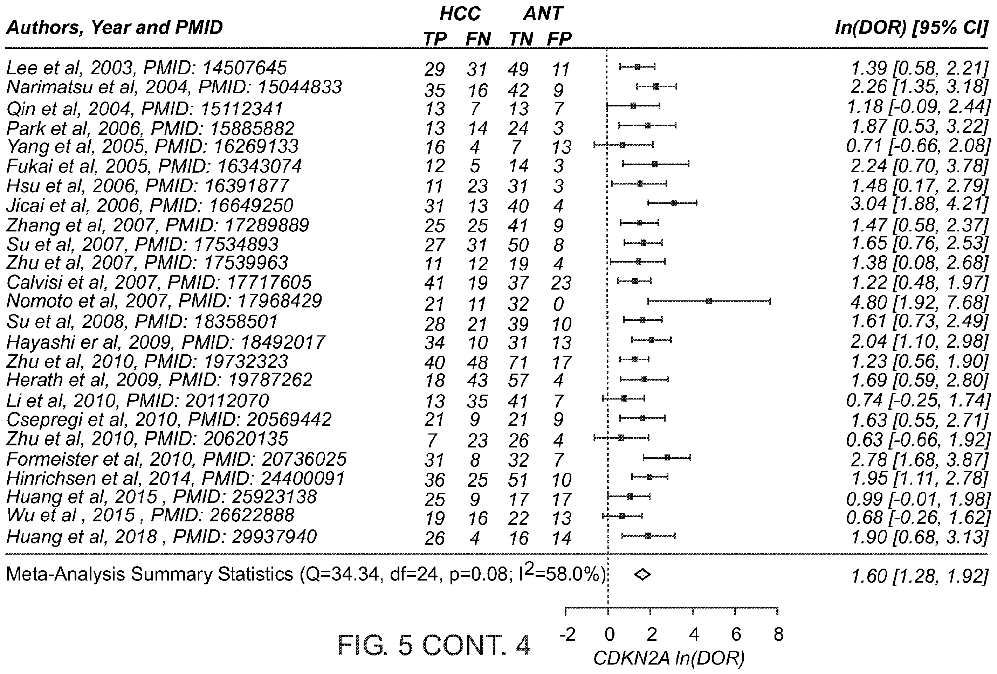

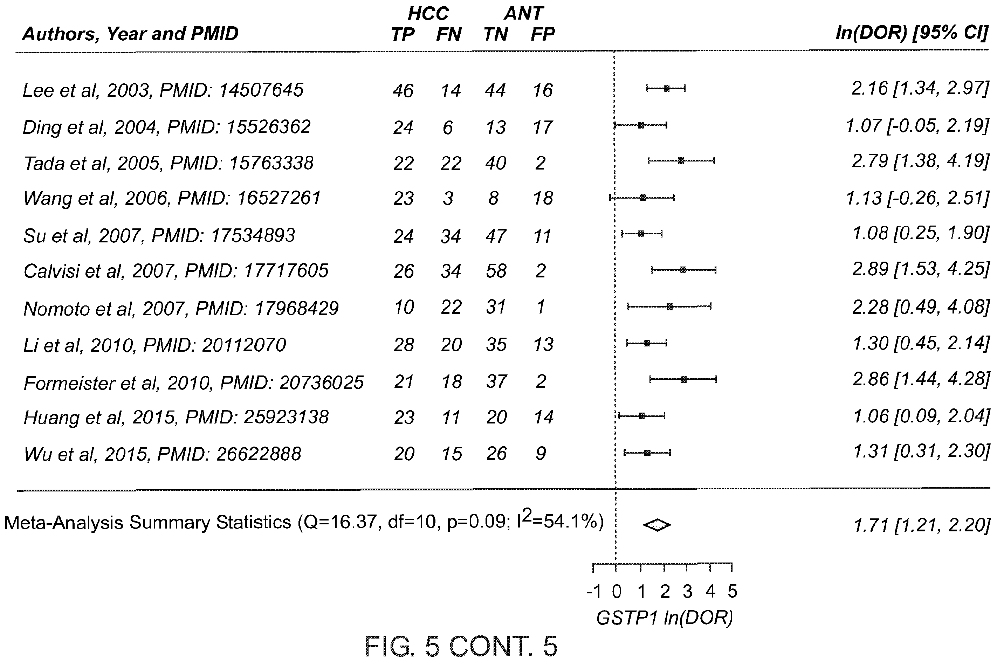

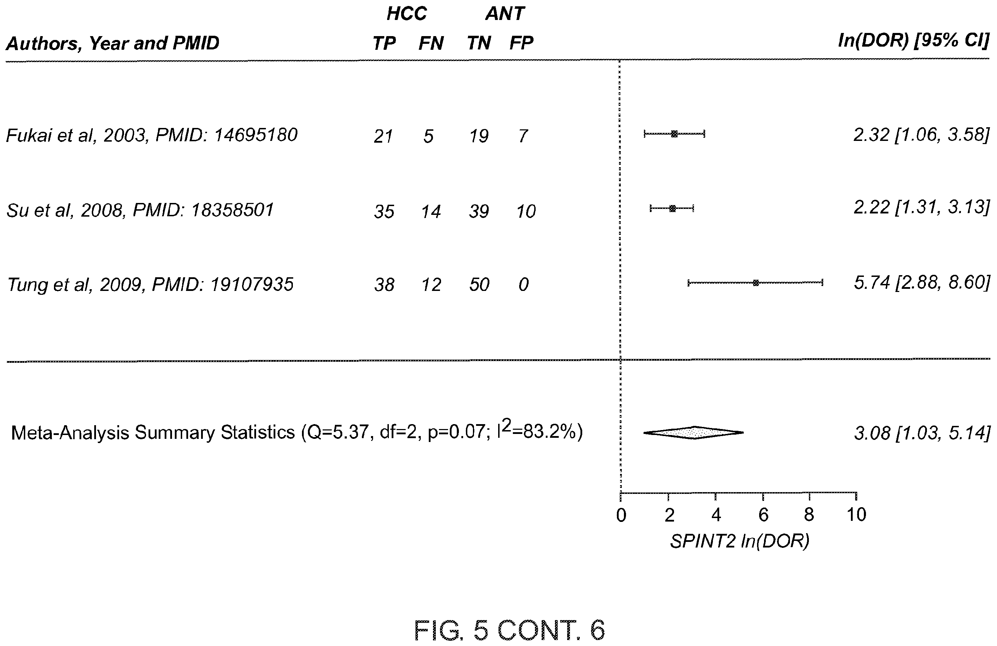

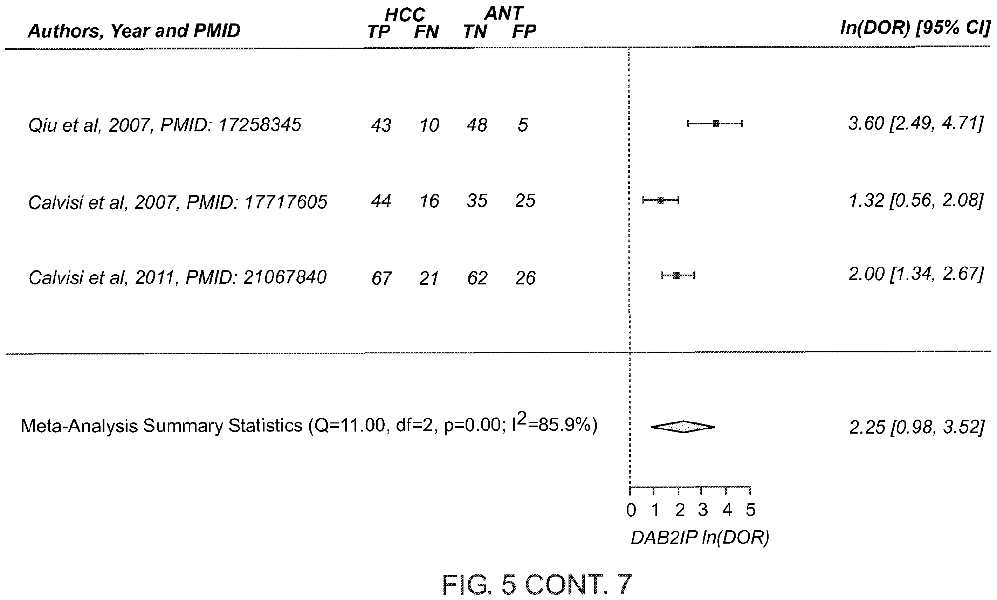

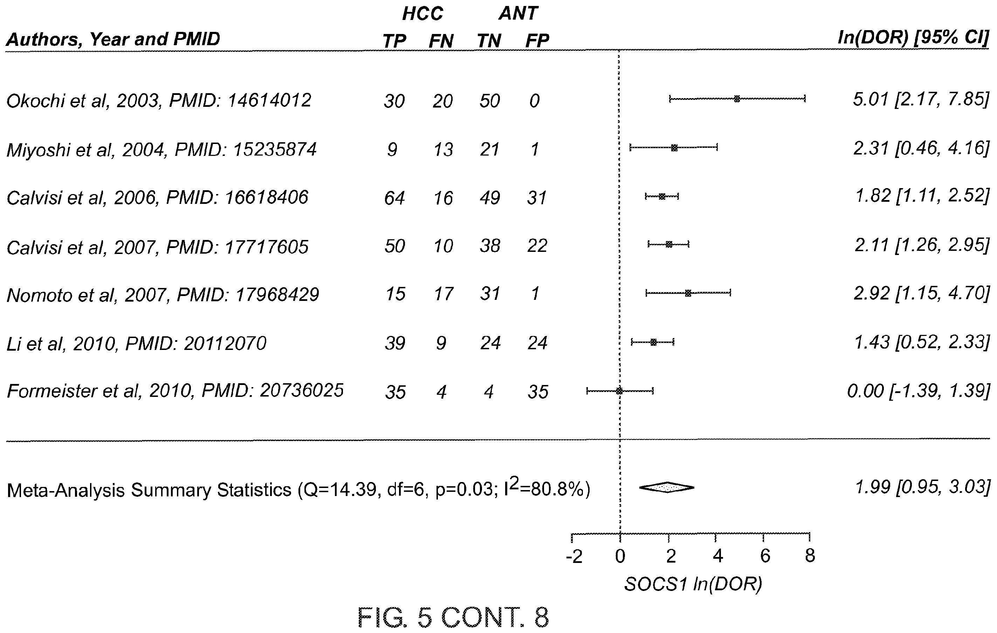

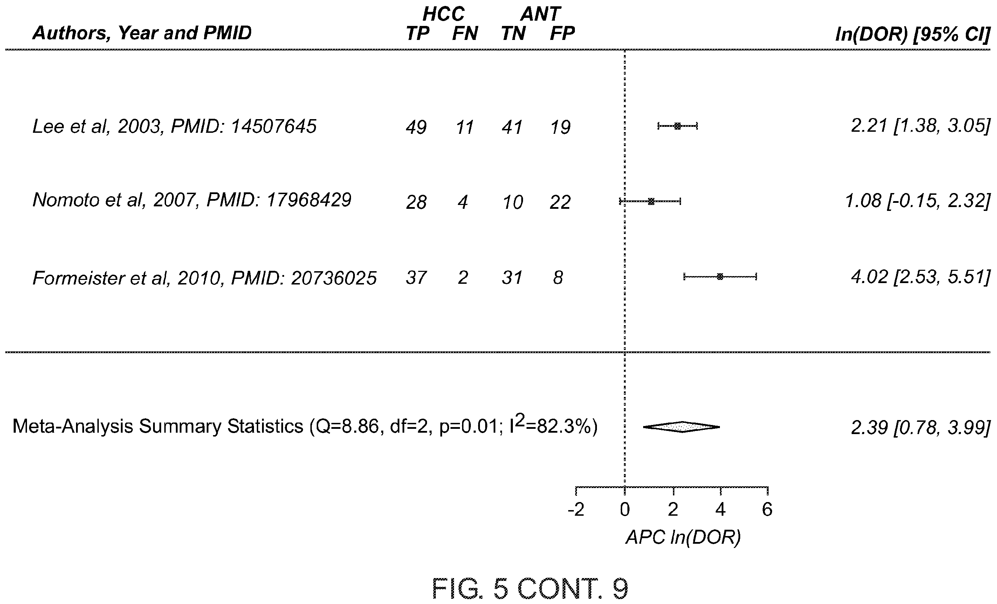

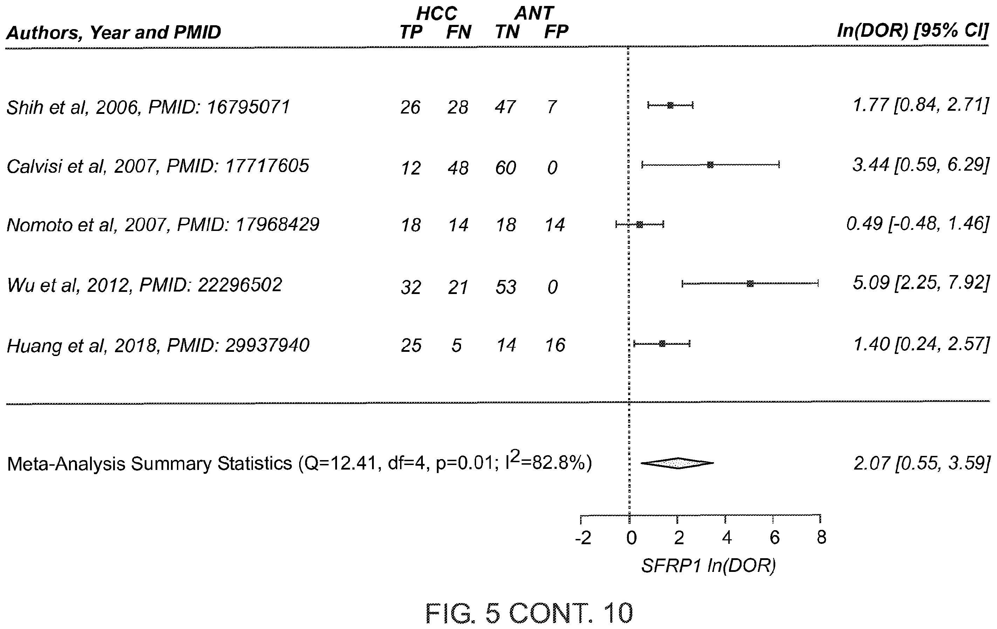

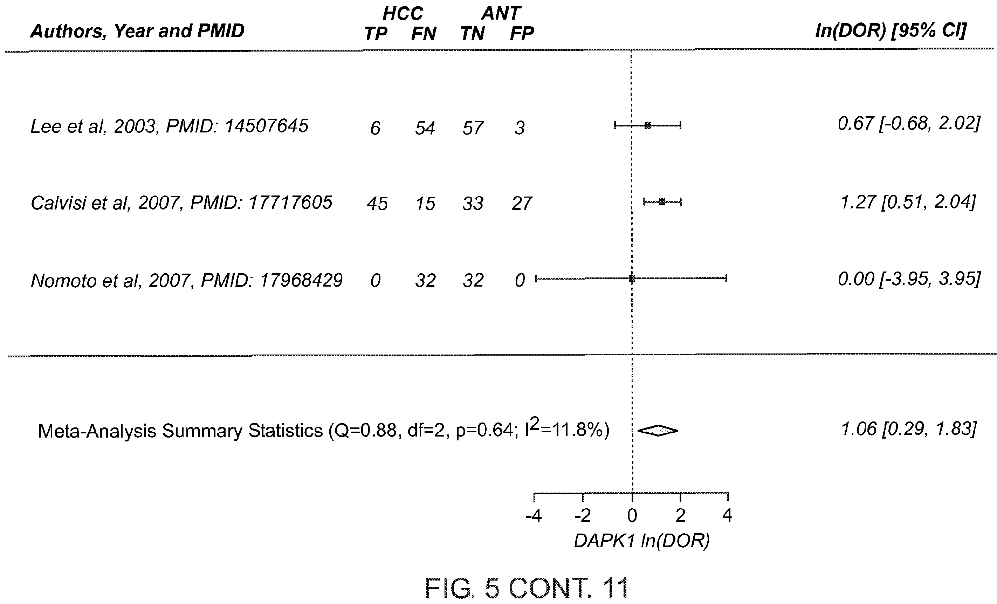

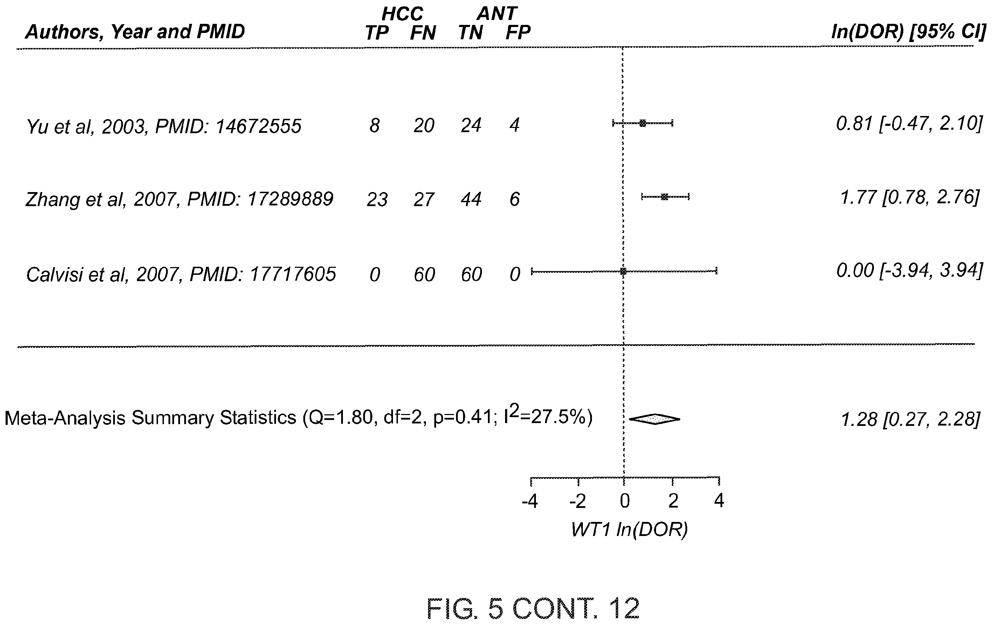

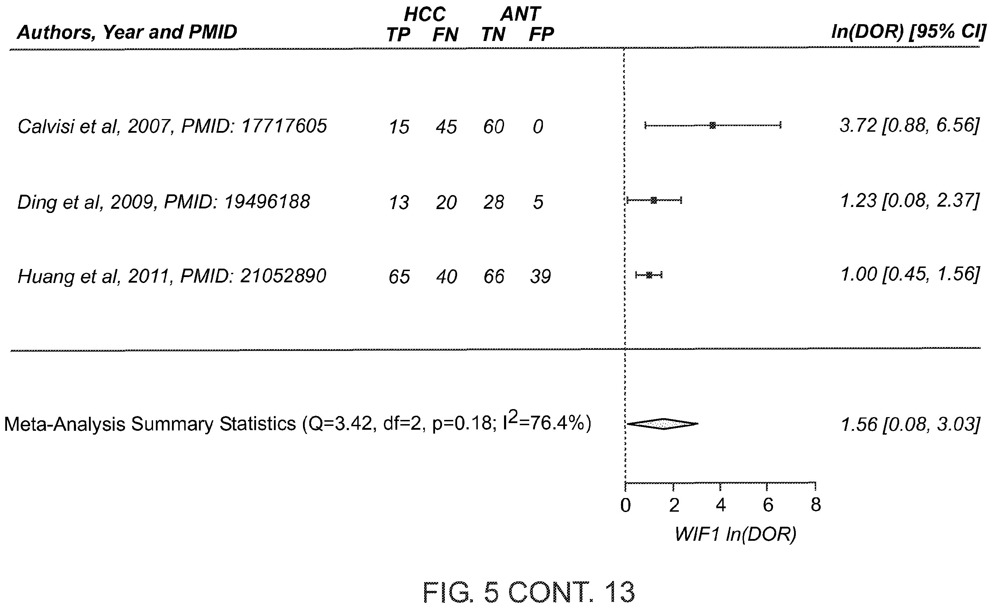

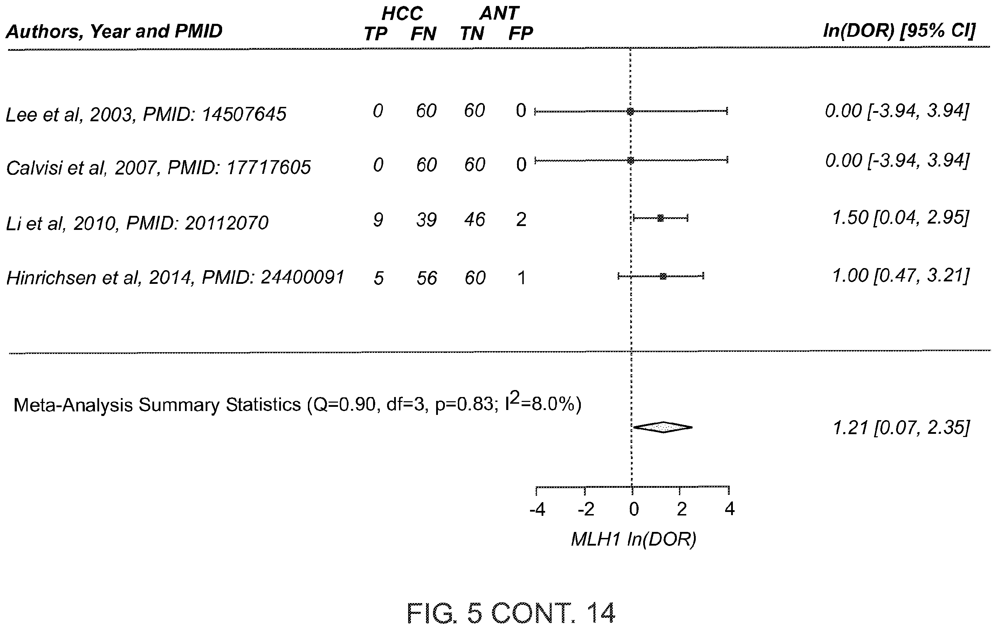

LAMB identifies hypermethylated tumor suppressors in tissues through meta-analysis data and differentially methylated CpGs in the tumor suppressors' promoters through microarray data. Gene hypermethylation count data of paired HCCs and adjacent noncancerous tissues (ANTs) was collected from 117 studies (FIG. 1A, FIG. 4). Diagnostic odds ratios (DORs) of genes in each study were calculated. A random-effects meta-analysis computed adjusted DORs for genes in three or more studies (FIG. 5). The meta-analysis identified 15 tumor suppressors, which were combined with 7 tumor suppressors showing high predictive power between HCC and cirrhosis cfDNA in binary classification models (AUC > 0.8)5-6 (FIG. 1A, Table 1).

Methylation frequencies (β) from Illumina HumanMethylation450 (450K) microarray data for CpGs in candidate tumor suppressor promoters was extracted from paired HCCs and ANTs of 153 patients from The Cancer Genome Atlas (TCGA) and two other studies⁷⁻⁹. CpGs with lower mean methylation in HCCs than ANTs, with hypermethylation in ANTs, or with low predictive power between HCCs and ANTs in a univariate logistic regression model were removed (FIG. 1A).

The majority of cfDNA comes from hematopoietic cell death with hepatocytes accounting for ~1-10% of cfDNA in healthy controls¹⁰⁻¹¹. To select for methylated CpGs distinguishable from hematopoietic cfDNA, sites were screened against 1722 lysed blood samples from healthy controls (FIG. 1B). 450K blood data was combined from five studies¹²⁻¹⁶. 159 TCGA HCCs were matched to blood by age, gender, and race. CpGs demonstrating low predictive power between HCC tissue and control blood in a univariate logistic regression model were removed. To identify biomarkers with low background methylation, CpGs were filtered by methylation frequency in the remaining 1563 blood samples.

The LAMB pipeline identified a cfDNA biomarker panel (LAMB-HCC) of 20 CpGs from 10 tumor suppressors (Table 2). 6 tumor suppressors were identified by the tissue meta-analysis (SPINT2, RUNX3, PRDM2, APC, GSTP1, WIF1), and 4 were found from plasma studies (SEPT9, HOXA1, PFKP, AK055957), underscoring the value of incorporating genes from both study types. While excluded genes show differential methylation in their studies, this effect was not shown in the 450K data, suggesting that these genes may not be population-wide predictors of HCC in cirrhosis patients.

The LAMB-HCC panel was then evaluated on an independent 450K validation dataset of cfDNA from 22 HCC patients with underlying cirrhosis and 22 cirrhosis patients matched by liver function and fibrosis⁴. CpG AUCs in univariate logistic regression models demonstrated increased predictive power from the CpGs excluded by the tissue and blood analyses to LAMB-HCC CpGs, underscoring the impact of LAMB's physiologically-inspired screening (FIG. 2A).

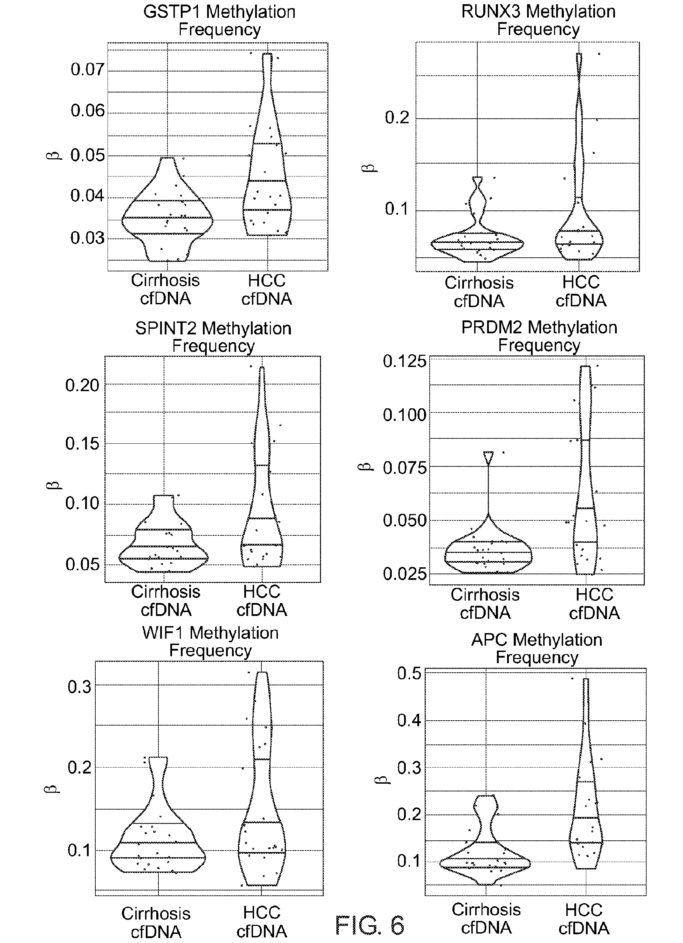

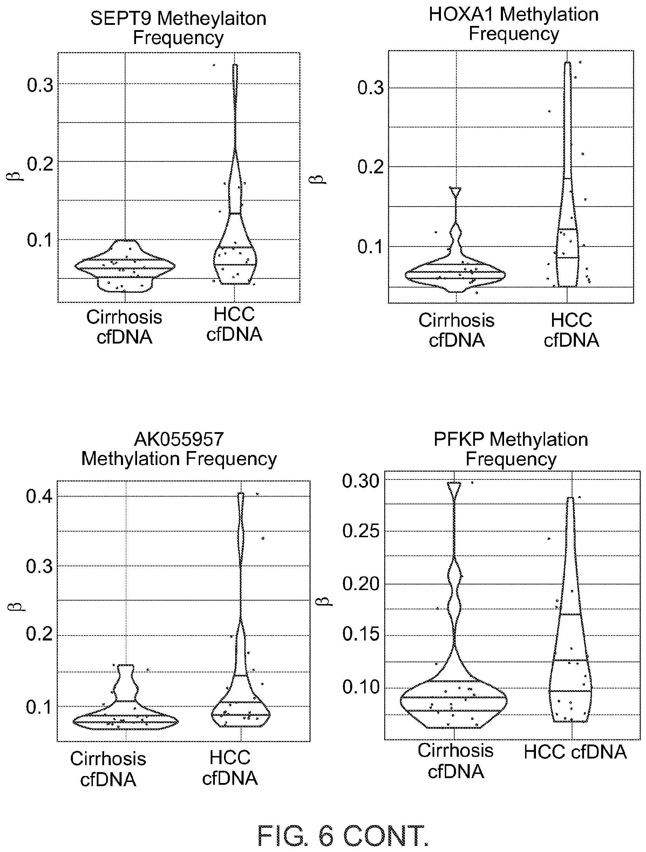

Due to increased, localized cfDNA contributions by cirrhotic livers, we hypothesized that cfDNA is not uniformly distributed in the vasculature of cirrhosis patients. Accordingly, a CpG's methylation frequency may fluctuate among blood samples from the same patient. In the validation dataset, cfDNA CpG methylation frequencies showed similar distributions in closely-situated sites (~50 bases), but distributions deviated with greater distances, even for sites in the same gene (FIG. 2B). To account for this variation, we viewed the LAMB-HCC panel as 20 CpGs that captured the methylation profile of 10 genes. We mapped the maximum methylation frequency of LAMB-HCC CpGs in the promoter of a gene to that gene, resulting in 10-gene methylation frequency profiles for the 44 cfDNA patients (FIG. 6, FIG. 2C).

To test the LAMB-HCC panel on the validation data in an unbiased manner, we calculated geometric mean scores for each patient through their 10-gene methylation frequency profiles (FIG. 2D). The geometric mean formula normalizes input methylation frequencies so that no frequency dominates weighting of the output score. LAMB-HCC gene methylation scores demonstrated high predictive power between HCC and cirrhosis cfDNA (AUC: 0.85, FIG. 2D).

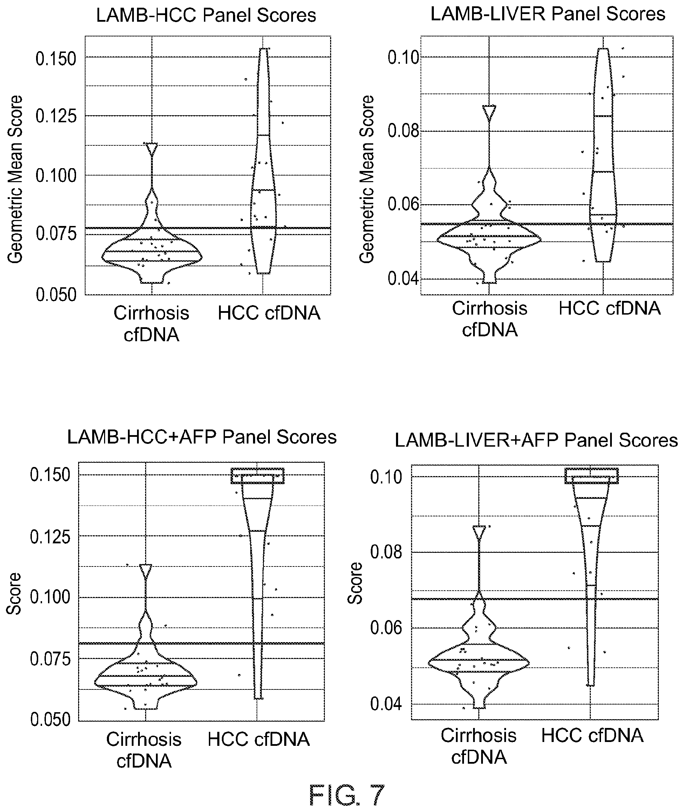

Hepatitis and NASH cirrhosis patients exhibit an increased risk for colorectal, pancreatic, and lung cancer¹⁷⁻¹⁸. We examined LAMB-HCC CpG methylation frequencies in 411 colorectal, 184 pancreatic, and 843 lung tumors from TCGA, identifying 6 CpGs within 4 genes that were not hypermethylated (βₘₑₐₙ < 0.2) in the non-liver cancers (Table 2). Geometric mean scores from this LAMB-LIVER panel exhibited high predictive power between HCC and cirrhosis cfDNA despite a slight decrease in AUC, suggesting that the LAMB method could identify cancer-specific biomarkers (FIG. 2D, FIG. 7).

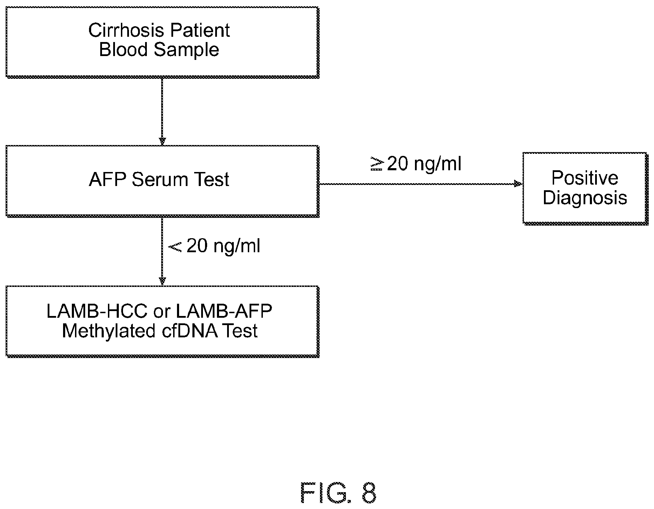

We next examined how LAMB panels perform in combination with AFP screening. At the 20 ng/mL clinical cutoff, 13/22 HCC patients tested positive (59% sensitivity), while all cirrhosis patients with AFP test values tested negative (100% specificity). These values are similar to what is observed in other patient cohorts (sensitivity: 59%, specificity: 90%)². To identify misdiagnosed HCC patients, we tested the 9 HCC and all 22 cirrhosis patients with AFP values below 20 ng/mL with the LAMB-HCC and LAMB-LIVER panels (FIG. 8). LAMB-HCC+AFP and LAMB-LIVER+AFP showed excellent predictive power (AUC: 0.93 and 0.92, respectively, FIG. 2D), highlighting the panels' ability to complement AFP screening (FIG. 7).

We created the LAMB method with the goal of mining two rich methylation data sources, published studies and 450K data, to find population-wide methylated cfDNA biomarkers. Utilizing both tissue and plasma studies identifies new tumor suppressor genes that complement published cfDNA biomarkers. By incorporating this information into 450K data in a physiologically-inspired manner, the LAMB method tests biomarkers across multiple data types, sample types, and patient populations in an effort to recapitulate the epigenetic diversity of tumors and people. Selectively collecting methylation count and frequency data from paired tissue samples and matching tumors to blood samples by demographic information provides the balance of cases and controls necessary to use DORs and AUCs as unbiased metrics to screen biomarkers. As a result, the LAMB pipeline identified a 10-gene cfDNA panel for HCC in cirrhosis patients that showed high predictive power in an external validation dataset. Additional validation of the panel with AFP and CpG methylation detection technologies is needed for eventual clinical adoption. Nevertheless, the LAMB method coupled with these validation results may fuel the creation of other physiologically-inspired data mining approaches to identify population-wide, biologically-rooted biomarkers for other diseases.

### References

1. Zhang, D.Y. & Friedman, S. L. Hepatology. 56, 769-775 (2012).
2. Marrero, J.A. et al. Gastroenterology. 137, 110-118 (2009).
3. Das, P.M. & Singal, R. J. Clin. Oncol. 22, 4632-4642 (2004).
4. Hlady, R.A. et al. Theranostics. 9, 7239-7250 (2019).
5. Kisiel, J.B. et al. Hepatology. 69, 1180-1192 (2019).
6. Oussalah, A. et al. EBioMedicine. 30, 138-147 (2018).
7. The Cancer Genome Atlas Network. Cell. 169, 1327-1341 (2017).
8. Kuramoto, J. et al. Carcinogenesis. 38, 261-270 (2017).
9. Shen, J. et al. Epigenetics. 8, 34-43 (2013).
10. Moss, J. et al. Nat. Commun. 9, 5068 (2018).
11. Sun, K. et al. Proc. Natl. Acad. Sci. U.S.A. 112, E5503-E5512 (2015).
12. Chuang, Y. et al. Genome Med. 9, 76 (2017).
13. Hannon E. et al. Genome Biol. 17, 176 (2016).
14. Horvath, S. et al. Genome Biol. 17, 171 (2016).
15. Cho. S.H. et al. J. Neurosci. 35, 807-818 (2015).
16. Hannum, G. et al. Mol. Cell. 49, 359-367 (2013).
17. Kalaitzakis, E. et al. Clin. Gastroenterol. Hepatol. 9, 168-174 (2011).
18. Sorensen, H.T. et al. Hepatology. 28, 921-925 (1998).

### Example 2

### Methods

### Meta-Analysis of Tissue Methylation Studies

We conducted a PubMed search for "((((hepatocellular carcinoma) OR HCC) OR hepatoma) OR hepatocarcinoma) AND methylation" that resulted in 2002 abstracts (as of October 1, 2019). The abstracts were screened for relevance to HCC tissue methylation, resulting in 612 pertinent tissue methylation papers (FIG. 4). These papers were screened for methylation frequency data, resulting in the inclusion of 317 studies. The remaining papers were first screened for testing HCCs and ANTs from the same patients and then screened for usage of methylation-specific PCR, leading to 117 papers. Count data was collated for the 117 papers with hypermethylated promoters for HCCs and ANTs treated as true positives and false positives, respectively. Using the "metafor" library in R, a random-effects meta-analysis utilizing the Hartung-Knapp-Sidik-Jonkman method was conducted on genes tested in three or more studies, resulting in adjusted natural logs of DORs (diagnostic odds ratios) with 95% confidence intervals (Table 1). Genes with log confidence intervals below 0 were discarded. Forest plots were generated for genes identified by the meta-analysis (FIG. 5). R.P., A.Y., and P.B.R. independently conducted the screening of abstracts/papers and collected count data from the resulting papers. Differences in identified papers and count data were discussed and all three agreed to final decisions. Plasma methylation studies of HCC and cirrhosis patients were identified during this search and examined for genes showing high predictive ability through logistic prediction models (AUC > 0.8). All 3 individuals agreed to the plasma studies and genes5-6 carried forward for the 450K tissue analysis.

### Promoter CpG Methylation Analysis in HCCs and ANTs

450K methylation frequency data was downloaded from the Cancer Genome Atlas (TCGA-LIHC)7. Recurrent tumors were removed and data for 50 patients with paired primary HCCs and ANTs was used. 450K data for 66 patients with paired tissue (GSE54503) and 37 patients with paired tissue (GSE89852) was downloaded from GEO8-9. These datasets were selected because they had public demographic information, which can be found in their associated studies. All three datasets were combined into a single dataset, and any CpG that was missing in a tissue sample was removed, leading to 326,322 CpGs for 153 patients. The data was logit-transformed, the "ComBat" package in R was utilized to correct for batch effects among the datasets, and the data was inverse logit-transformed. Remaining CpGs were coupled to genes, features, and chromosomal locations through Illumina's 450K annotation file. Methylation frequency data for the CpGs in the TSS1500 and TSS200 for the 22 genes from the tissue meta-analysis and plasma study analysis was extracted for all 153 patients, as well as a cohort of 67 patients with 61 early-stage tumors. Through the R "pROC" library, mean HCC methylation, mean ANT methylation, and univariate CpG site AUCs between HCCs and ANTs were calculated for all of the 153 patients and the 67-patient cohort (Table 2). CpGs with a lower mean methylation in HCC than ANT, with relative hypermethylation in ANT (methylation frequency greater than 0.2), or with an AUC less than 0.8 in a univariate logistic regression model of all patients were excluded from 450K blood analysis.

### Promoter CpG Methylation Analysis in Whole Blood

450K methylation frequency data was downloaded for 305, 127, 622, 272, 236, and 160 healthy controls from GEO (GSE84727, GSE80417, GSE40279, GSE72773, GSE111629, GSE53740), combined, and logit-transformed¹²⁻¹⁶. The datasets analyzed lysed whole blood and were structured such that healthy controls were easy to identify. The data was corrected for batch effects through the "ComBat" package in Python and then inverse logit-transformed. Methylation frequency data for CpGs identified in the 450K HCC/ANT analysis was extracted from the combined blood data. To perform a differential methylation analysis of HCC tissue to blood, 450K data for 163 separate TCGA HCCs was downloaded. 159 of these tumors were matched to whole blood samples based on the patients' age, gender, and race. The ratio of matched blood samples by their original dataset mirrored the ratio of total blood samples by their original dataset. CpGs with an AUC less than 0.8 from a univariate logistic regression model were discarded (Table 2). The mean methylation frequencies of remaining CpGs in the remaining 1563 blood samples were calculated. CpGs with a methylation frequency less than 0.1 were identified as LAMB CpGs (Table 2).

### LAMB-HCC Panel Analysis in Cell-Free DNA

450K methylation frequency data was downloaded for 22 HCC patients with underlying cirrhosis and 22 cirrhosis patients (GSE129374)4. As of October 2019, GSE129374 is the only methylation frequency data available for cell-free DNA from HCC and cirrhosis patients; these patients were matched by liver function and fibrosis. Univariate AUCs through logistic regression models were calculated for all CpGs analyzed by LAMB's 450K filters. Data for LAMB CpGs was extracted, and the maximum methylation frequency of LAMB CpGs in a gene promoter was mapped to that gene to create 10-gene methylation profiles for all 44 patients (FIG. 2C, FIG. 6). The geometric mean of each of the 10-gene methylation profiles was calculated (FIG. 2D). With the "pROC" package in R, a ROC curve was created, and an AUC was found from the geometric mean scores. 95% AUC confidence intervals were computed with 1000-iteration bootstrapping.

### LAMB-LIVER Panel Analysis in Cell-Free DNA

450K data for 411 colorectal, 184 pancreatic, and 843 lung primary tumors was downloaded from TCGA (TCGA-COAD, TCGA-READ, TCGA-PAAD, TCGA-LUSC, TCGA-LUAD). The mean methylation frequencies for LAMB CpGs were extracted for each cancer type. 6 CpGs with mean methylation frequencies below 0.2 in these cancers were selected for the LAMB-LIVER panel. The maximum methylation frequency of LAMB-LIVER CpGs in a gene promoter was mapped to that gene to create 4-gene methylation profiles for all 44 cfDNA samples. The geometric mean of each of the 4-gene methylation profiles was calculated (FIG. 2D). In the "pROC" package in R, a ROC curve was created, and an AUC was found from the geometric mean scores. 95% AUC confidence intervals were computed with 1000-iteration bootstrapping.

### LAMB-HCC+AFP and LAMB-LIVER+AFP Panel Analysis in Cell-Free DNA

AFP serum levels were found for patients in the 450K cfDNA validation dataset from the paper's supplement⁴. The AFP levels of 3 out of 22 cirrhosis patients was not available. For the remaining patients, 9 out of 22 HCC and all 19 cirrhosis patients had AFP levels below the clinical cutoff of 20 ng/mL. The 13 HCC patients with AFP levels above 20 ng/mL were classified as positive, and the LAMB-HCC and LAMB-LIVER geometric mean scores for the 9 remaining HCC patients and all 22 cirrhosis patients were tested. In the "pROC" package in R, ROC curves were created, and AUCs were found from the geometric mean scores. 95% AUC confidence intervals were computed with 1000-iteration bootstrapping.

**TABLE 1. DIAGNOSTIC ODDS RATIOS (DOR) OF GENES ANALYZED BY META-ANALYSIS**

| **Gene** | **DOR (95% CI)** | **n** |
|---|---|---|
| PRDM2 | 15.2 (7.6, 30.4) | 275 |
| RUNX3 | 21.7 (5.5, 86.4) | 242 |
| RASSF1A | 8.5 (4.5, 16.0) | 871 |
| TFPI2 | 10.1 (3.8, 26.8) | 272 |
| CDKN2A (p16) | 5.0 (3.6, 6.8) | 1045 |
| GSTP1 | 5.5 (3.4, 9.0) | 465 |
| SPINT2 | 21.8 (2.8, 170.6) | 125 |
| DAB2IP | 9.5 (2.7, 33.7) | 201 |
| SOCS1 | 7.3 (2.6, 20.8) | 331 |
| APC | 10.9 (2.2, 54.1) | 131 |
| SFRP1 | 7.9 (1.7, 36.1) | 229 |
| DAPK1 | 2.9 (1.3, 6.2) | 152 |
| WT1 | 3.6 (1.3, 9.8) | 138 |
| WIF1 | 4.7 (1.1, 20.7) | 198 |
| MLH1 | 3.4 (1.1, 10.5) | 229 |
| TP73 | 4.5 (0.9, 23.0) | 136 |
| EDNRB | 5.6 (0.8, 37.7) | 142 |
| RB1 | 1.5 (0.7, 3.3) | 181 |
| SOCS3 | 3.9 (0.7, 21.9) | 138 |
| CDKN2A (p14) | 1.5 (0.7, 3.3) | 500 |
| TP53 | 3.4 (0.6, 19.1) | 107 |
| CDH1 | 1.2 (0.4, 3.1) | 340 |
| CDKN2B (p15) | 1.2 (0.4, 3.6) | 300 |
| MGMT | 1.1 (0.4, 3.3) | 387 |
| CAV1 | 8.1 (0.3, 213.4) | 104 |
| STK11 | 2.1 (0.3, 17.3) | 136 |

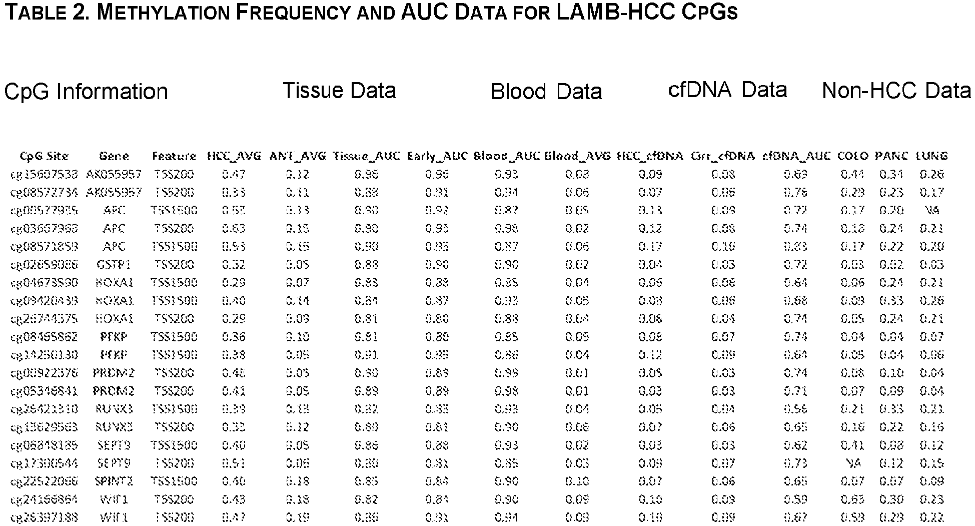

**TABLE 3. LAMB-HCC-PROBE-SEQUENCES**

| Sequence Name | Sequence | Chrom osome | Start | Stop | Orient ation | Group Name | Request ID | GC Percent |
|---|---|---|---|---|---|---|---|---|
| 657302_29427299_PRDM 2_1_Unmethyl | CAACAACTACATCTATCTACCTAAAATTAAATCTTTTTAATATAAATCCAAACATAAAAATATAAAATCT ACATTTATATACTTATCAAATATTTAATAACCTCCAACAATAAACATACC (SEQ ID NO:1) | 1 | 1402 6288 | 1402 6408 | + | PRDM 2 | 657302 | 42.5 |
| 657302_29427299_PRDM 2_2_Unmethyl | TATAAAATCTACATTTATATACTTATCAAATATTTAATAACCTCCAACAATAAACATACCCCCCCAACAC CTCCTTCAATCTTTTTAATTCTTTAAAAATCTTTAAACCACACAAAATCC (SEQ ID NO:2) | 1 | 1402 6348 | 1402 6468 | + | PRDM 2 | 657302 | 41.67 |
| 657302_29427299_PRDM 2_3_Unmethyl | CCCCCAACACCTCCTTCAATCTTTTTAATTCTTTAAAAATCTTTAAACCACACAAAATCCTAACCCTAAAA TACAATCAAACTTACCACAACACCAACCACCAAAATTCCTTCCCAAAAC (SEQ ID NO:3) | 1 | 1402 6408 | 1402 6528 | + | PRDM 2 | 657302 | 60 |
| 657302_29427299_PRDM 2_4_Unmethyl | TAACCCTAAAATACAATCAAACTTACCACAACACCAACCACCAAAATTCCTTCCCAAAACACCACCATA AAATCCACAATAACCTACAAAAAACAACTAAATAATAACCATTAAACAACA (SEQ ID NO:4) | 1 | 1402 6468 | 1402 6588 | + | PRDM 2 | 657302 | 73.33 |
| 657302_29427299_PRDM 2_5_Unmethyl | ACCACCATAAAATCCACAATAACCTACAAAAAACAACTAAATAATAACCATTAAACAACAACACAAAAT CAAAAACCAAACTCTAAAATCATAAAAAAACCAAAAATCCCAAAACCCCCC (SEQ ID NO:5) | 1 | 1402 6528 | 1402 6648 | + | PRDM 2 | 657302 | 75.83 |
| 657302_29427299_PRDM 2_6_Unmethyl | ACACAAAATCAAAAACCAAACTCTAAAATCATAAAAAAACCAAAAATCCCAAAACCCCCCAACCCCAC AAACCACTACCTCACCACCCAACATCACTTCCAACTAAAATCAAAATAACAA (SEQ ID NO:6) | 1 | 1402 6588 | 1402 6708 | + | PRDM 2 | 657302 | 74.17 |
| 657302_29427299_PRDM 2_7_Unmethyl | AACCCCACAAACCACTACCTCACCACCCAACATCACTTCCAACTAAAATCAAAATAACAACAACACAAC CACAAACACCAAAACCAACAAAACAACAACAACAACAACAACCCTCAATAC (SEQ ID NO:7) | 1 | 1402 6648 | 1402 6768 | + | PRDM 2 | 657302 | 80 |
| 657302_29427299_PRDM 2_8_Unmethyl | CAACACAACCACAAACACCAAAACCAACAAAACAACAACAACAACAACAACCCTCAATACTCTAAAAC ACTAACACAACAAACACAAATAAAAAATAACACAAATAAACAACCTCTAAAC (SEQ ID NO:8) | 1 | 1402 6708 | 1402 6828 | + | PRDM 2 | 657302 | 85 |
| 657302_29427299_PRDM 2_9_Unmethyl | TCTAAAACACTAACACAACAAACACAAATAAAAAATAACACAAATAAACAACCTCTAAACTCACACCAA AACACTAATCCCTCCCCCCAAAAAACACAACAACAACAACAACAACAACAC (SEQ ID NO:9) | 1 | 1402 6768 | **1402** 6888 | + | PRDM 2 | 657302 | 81.67 |
| 657302_29427300_RUNX 3_1_Unmethyl | TAAACCACAACAAAACCCACACAAAACTATACCACTACCACCACCTCCCACCCCAAAACTCACCCACAA CCACCCCAACTCCACAACCACAACCCCAAAACAAATCCTCCAAAATCAAAT (SEQ ID NO:10) | 1 | 2525 6644 | 2525 6764 | + | RUNX3 | 657302 | 78.33 |
| 657302_29427300_RUNX 3_2_Unmethyl | CACCCACAACCACCCCAACTCCACAACCACAACCCCAAAACAAATCCTCCAAAATCAAATAACAAAACC ACAACCACCCACACAAAATTAATACCCCCAAAACCCACAAAACAAAACTAA (SEQ ID NO:11) | 1 | 2525 6704 | 2525 6824 | + | RUNX3 | 657302 | 80 |
| 657302_29427300_RUNX 3_3_Unmethyl | AACAAAACCACAACCACCCACACAAAATTAATACCCCAAAACCCACAAAACAAAACTAACAAAACAA CACATCACACAACCAATCAACAAAACCCCCATCACAAACACCTCAATAACAT (SEQ ID NO:12) | 1 | 2525 6764 | 2525 6884 | + | RUNX3 | 657302 | 82.5 |
| 657302_29427300_RUNX 3_4_Unmethyl | CAAAACAACACATCACACAACCAATCAACAAAACCCCCATCACAAACACCTCAATAACATTCACAAAAA AAAACAAAACCTACCAAAAACCACCCAACAAAAAAAAACAAAAAACACCAC (SEQ ID NO:13) | 1 | 2525 6824 | 2525 6944 | + | RUNX3 | 657302 | 77.5 |
| 657302_29427300_RUNX 3_5_Unmethyl | TCACAAAAAAAAACAAAACCTACCAAAAACCACCCAACAAAAAAAAACAAAAAACACCACCCCACAAA AAAAACCCCAATACCACAACCCAAAACCCCCAAAAACTCTAAAAACCCAAAA (SEQ ID NO:14) | 1 | 2525 6884 | 2525 7004 | + | RUNX3 | 657302 | 80 |
| 657302_29427300_RUNX 3_6_Unmethyl | CCCACAAAAAAAACCCCAATACCACAACCCAAAACCCCCAAAAACTCTAAAAACCCAAAACAAATACT AAAAATTTACTTAAAACATCCAAATCCCACAAAAAACACCCTTACCACCCTC (SEQ ID NO:15) | 1 | 2525 6944 | 2525 7064 | + | RUNX3 | 657302 | 69.17 |
| 657302_29427300_RUNX 3_7_Unmethyl | CAAATACTAAAAATTTACTTAAAACATCCAAATCCCACAAAAAACACCCTTACCACCCTCTCTCAAATCA TAACTCCCTAACACTAAAACACAACCCCTTCACTCCTCCTCCCCACTAAC (SEQ ID NO:16) | 1 | 2525 7004 | 2525 7124 | + | RUNX3 | 657302 | 65 |
| 657302_29427300_RUNX 3_8_Unmethyl | TCTCAAATCATAACTCCCTAACACTAAAACACAACCCCTTCACTCCTCCTCCCCACTAACCACAACCAAA CTTCCCCAACTCTTACTACTTCAAACCTATAACTTCTACAACCCCAAACT (SEQ ID NO:17) | 1 | 2525 7064 | 2525 7184 | + | RUNX3 | 657302 | 70.83 |
| 657302_29427300_RUNX 3_9_Unmethyl | CACAACCAAACTTCCCCAACTCTTACTACTTCAAACCTATAACTTCTACAACCCCAAACTAAAAACCACA AAATCTCAAAACCAATAACACCACACTAAAAACCACCCCAAAAAAATTAC (SEQ ID NO:18) | 1 | 2525 7124 | 2525 7244 | + | RUNX3 | 657302 | 72.5 |
| 657302_29427300_RUNX 3_10_Unmethyl | AAAAACCACAAAATCTCAAAACCAATAACACCACACTAAAAACCACCCCAAAAAAATTACTCACCTCCC TCATCCCACACATTATTCTAACCCAAAAACCTCCACCCCACACAAAATTTT (SEQ ID NO:19) | 1 | 2525 7184 | 2525 7304 | + | RUNX3 | 657302 | 65.83 |
| 657302_29427300_RUNX 3_11_Unmethyl | TCACCTCCCTCATCCCACACATTATTCTAACCCAAAAACCTCCACCCCACACAAAATTTTACACATCATCC ACACCCAACCAACAACCTTTACTACTCCCAACCCTACACAACTTTAATC (SEQ ID NO:20) | 1 | 2525 7244 | 2525 7364 | + | RUNX3 | 657302 | 66.67 |
| 657302_29427301_PFKP.1 _1_Unmethyl | CCAAACCCCAAAACTCCCAACACCACCCCAAAAAAAATCTTACAAACCACTAAAAACTATACACACAAC CAAACTCAACCCCCACAACAACCCAAACAACCAAACCTACCACAAACCTCC (SEQ ID NO:21) | 10 | 3109 032 | 3109 152 | + | PFKP | 657302 | 78.33 |
| 657302_29427301_PFKP.1 _2_Unmethyl | ACACACAACCAAACTCAACCCCCACAACAACCCAAACAACCAAACCTACCACAAACCTCCACATCCAAA TAAAATCTCCACACCCCCATTCCACCCCTCCCCACCATCAACACTCCCCAC (SEQ ID NO:22) | 10 | 3109 092 | 3109 212 | + | PFKP | 657302 | 82.5 |
| 657302_29427301_PFKP.1 _3_Unmethyl | ACATCCAAATAAAATCTCCACACCCCCATTCCACCCCTCCCCACCATCAACACTCCCCACAACTACAAAA TTTCCACCCACCCCAACACTCACACCCACTCACAATCTCCCTCACCCCCC (SEQ ID NO:23) | 10 | 3109 152 | 3109 272 | + | PFKP | 657302 | 77.5 |
| 657302_29427301_PFKP.1 _4_Unmethyl | AACTACAAAATTTCCACCCACCCCAACACTCACACCCACTCACAATCTCCCTCACCCCCCCAAAAAATAC TCCCAACATTCTATCCCACCCCACCCTAACACACCCCACAAACAATATAA (SEQ ID NO:24) | 10 | 3109 212 | 3109 332 | + | PFKP | 657302 | 74.17 |
| 657302_29427301_PFKP.1 _5_Unmethyl | CAAAAAATACTCCCAACATTCTATCCCACCCCACCCTAACACACCCCACAAACAATATAACCCCAACCCC CTACAATCTCCCCTCCTCCAAACACTACCCCTACCCCACCTATAACACTC (SEQ ID NO:25) | 10 | 3109 272 | 3109 392 | + | PFKP | 657302 | 75.83 |
| 657302_29427301_PFKP.1 _6_Unmethyl | CCCCAACCCCCTACAATCTCCCCTCCTCCAAACACTACCCCTACCCCACCTATAACACTCCCATCATCTCT AAAACCCCCAACCAAAATCCTCCAACCCACCCACAAAACTCACACACCT (SEQ ID NO:26) | 10 | 3109 332 | 3109 452 | + | PFKP | 657302 | 74.17 |
| 657302_29427301_PFKP.1 _7_Unmethyl | CCATCATCTCTAAAACCCCCAACCAAAATCCTCCAACCCACCCACAAAACTCACACACCTACAATACAAC CCCACTAACAAAAACCCACCCACCTATAACACTACCCCTACCTCTACCAC (SEQ ID NO:27) | 10 | 3109 392 | 3109 512 | + | PFKP | 657302 | 71.67 |
| 657302_29427301_PFKP.1 _8_Unmethyl | ACAATACAACCCCACTAACAAAAACCCACCCACCTATAACACTACCCCTACCTCTACCACCTCCCACACC ACTAACACAACCCCCACCTACAACAACCCCCACATAAATACTCCTACCCA (SEQ ID NO:28) | 10 | 3109 452 | 3109 572 | + | PFKP | 657302 | 74.17 |
| 657302_29427301_PFKP.1 _9_Unmethyl | CTCCCACACCACTAACACAACCCCCACCTACAACAACCCCCACATAAATACTCCTACCCACACCCACCCC AACCCCAACCCCTACCCCTAACCACACCCACACCCCCTCCCCCACAACCC (SEQ ID NO:29) | 10 | 3109 512 | 3109 632 | + | PFKP | 657302 | 80.83 |
| 657302_29427301_PFKP.1 _10_Unmethyl | CACCCACCCCAACCCCAACCCCTACCCCTAACCACACCCACACCCCCTCCCCCACAACCCCTCCCATACA CTCAAACCCCCACCCATCACCACCCATTAACACTAAAACCAAACAAAAAC (SEQ ID NO:30) | 10 | 3109 572 | 3109 691 | + | PFKP | 657302 | 84.87 |
| 657302_29427302_GSTP1 .1_1_Unmethyl | ATAAAATAAAATAAAATAAAACAATTTCCTTTCCTCTAAACAACCTCCACCCCTCTCCCCTACCCTATAA AACAAATATACAAACTCCAAAATCACAACAATCTTAAAAAATTTCCCCCC (SEQ ID NO:31) | 11 | 6735 0856 | 6735 0976 | + | GSTP1 | 657302 | 51.67 |
| 657302_29427302_GSTP1 .1_2_Unmethyl | TACCCTATAAAACAAATATACAAACTCCAAAATCACAACAATCTTAAAAAATTTCCCCCCACAATATCCC AACACACCAATTCACTACACACACTTCACTACAATCCTCTTCCTACTATC (SEQ ID NO:32) | 11 | 6735 0916 | 6735 1036 | + | GSTP1 | 657302 | 65.83 |
| 657302_29427302_GSTP1 .1_3_Unmethyl | ACAATATCCCAACACACCAATTCACTACACACACTTCACTACAATCCTCTTCCTACTATCTATTTACTCCC TAAACCCCACTAAAAACCTAAAAAAAAAAAAAAAACTTCCCCAACCAAC (SEQ ID NO:33) | 11 | 6735 0976 | 6735 1096 | + | GSTP1 | 657302 | 66.67 |
| 657302_29427302_GSTP1 .1_4_Unmethyl | TATTTACTCCCTAAACCCCACTAAAAACCTAAAAAAAAAAAAAAAACTTCCCCAACCAACTACACAACA ACTCCAAAAACTCCAAAACACCCCTCTACAACCAACACCCAAAATACAACA (SEQ ID NO:34) | 11 | 6735 1036 | 6735 1156 | + | GSTP1 | 657302 | 73.33 |
| 657302_29427302_GSTP1 .1_5_Unmethyl | TACACAACAACTCCAAAAACTCCAAAACACCCCTCTACAACCAACACCCAAAATACAACAACCACCAAA ACTAAAACCAACAAAAATCCACAAAACCCTCCAAAAAAACAACCAACACCA (SEQ ID NO:35) | 11 | 6735 1096 | 6735 1216 | + | GSTP1 | 657302 | 83.33 |
| 657302_29427302_GSTP1 .1_6_Unmethyl | ACCACCAAAACTAAAACCAACAAAAATCCACAAAACCCTCCAAAAAAACAACCAACACCATAACTCAA CACTAAAACAAAACAAAACAAAACCACCCTTATAAAACTCAAAAACCACAAA (SEQ ID NO:36) | 11 | 6735 1156 | 6735 1276 | + | GSTP1 | 657302 | 77.5 |
| 657302_29427303_WIF1. 1_1_Unmethyl | CAAAACCTCCTCACTACCAAAAAAACTCCTCATACCACACCTACACAACCTAACACCATCAAATACTCTA CTACACTACAACTCCCTCAACCAAAACTATTCCCATTTAAACAACTAAAC (SEQ ID NO:37) | 12 | 6551 4977 | 6551 5097 | + | WIF1 | 657302 | 65.83 |
| 657302_29427303_WIF1. 1_2_Unmethyl | AAATACTCTACTACACTACAACTCCCTCAACCAAAACTATTCCCATTTAAACAACTAAACACATCACCTC CCAACCTAAATACCAAAAAACCTACAAAAACAAAAAAAAAAAACAAACA (SEQ ID NO:38) | 12 | 6551 5037 | 6551 5156 | + | WIF1 | 657302 | 68.91 |
| 657302_29427304_WIF1. 2_1_Unmethyl | ACAAAAAAAAAAAAAAAAAAACCAACAAACACAAATCACACAAAAAAATAAAACACAAACAAAAATAA AAACAACAAAAACATAAAACTAACAAAACTAACAAAACCAACAATCAACAAAA (SEQ ID NO:39) | 12 | 6551 5160 | 6551 5280 | + | WIF1 | 657302 | 73.33 |
| 657302_29427304_WIF1. 2_2_Unmethyl | AAAATAAAAACAACAAAAACATAAAACTAACAAAACTAACAAAACCAACAATCAACAAAACAAATAAA ACAAAAACAAAAAAACAAAAAAAACTAACACAAACAAAATACAAACAAAAAA (SEQ ID NO:40) | 12 | 6551 5220 | 6551 5340 | + | WIF1 | 657302 | 65.83 |
| 657302_29427304_WIF1. 2_3_Unmethyl | CAAATAAAACAAAAACAAAAAAACAAAAAAAACTAACACAAACAAAATACAAACAAAAAATAAAACC CACAAAACCTAAACAACCACCACTTAAAAACACTATAAAACCCCCCCAAAAAC (SEQ ID NO:41) | 12 | 6551 5280 | 6551 5400 | + | WIF1 | 657302 | 75 |
| 657302_29427304_WIF1. 2_4_Unmethyl | TAAAACCCACAAAACCTAAACAACCACCACTTAAAAACACTATAAAACCCCCCAAAAACAAAACCACA AAAAACCCCCAAAACTACATTCACAATACAATACACCCAATAAAACACCCA (SEQ ID NO:42) | 12 | 6551 5340 | 6551 5460 | + | WIF1 | 657302 | 81.67 |
| 657302_29427304_WIF1. 2_5_Unmethyl | AAAACCACAAAAAACCCCCAAAACTACATTCACAATACAATACACCCAATAAAACACCCACAAAATAA ACATATAAAACTAACAACCCAAAAAAAAACACTCAAAAACCCCTAAAACAT (SEQ ID NO:43) | 12 | 6551 5400 | 6551 5520 | + | WIF1 | 657302 | 71.67 |
| 657302_29427304_WIF1. 2_6_Unmethyl | CAAAATAAACATATAAAACTAACAAACCCAAAAAAAAACACTCAAAAACCCCTAAAACATCACTCACC ACCTCCTCAAACCAAAAAAAAACTTCAAAAAAAAAAAAATAAAAAAAACAAACA (SEQ ID NO:44) | 12 | 6551 5460 | 6551 5580 | + | WIF1 | 657302 | 66.67 |
| 657302_29427305_AK055 957.1_1_Unmethyl | AAAAACCACCACACCCAACCTCAAACATTTTTCTCTAAATAAAACCACAAAATAAACCAACCCCTATAA AAATTTAAAAATAACAACATCCATCAATTTCTATATAAAAAATATACAAAT (SEQ ID NO:45) | 12 | 1.33E +08 | 1.33E +08 | + | AK055 957 | 657302 | 50.83 |
| 657302_29427305_AK055 957.1_2_Unmethyl | CCCCTATAAAAATTTAAAAATAACAACATCCATCAATTTCTATATAAAAAATATACAAATCATATAACAC ATCTTCTAAAAATAAAAATCTACTAAACTCACCATCACAAATAAATAAAC (SEQ ID NO:46) | 12 | 1.33E +08 | 1.33E +08 | + | AK055 957 | 657302 | 43.33 |
| 657302_29427305_AK055 957.1_3_Unmethyl | CATATAACACATCTTCTAAAAATAAAAATCTACTAAACTCACCATCACAAATAAATAAACACATTTAAA ATTTATAATTAAAAAAAAAACAACACCAACAACACAAATACCCCAAACACT (SEQ ID NO:47) | 12 | 1.33E +08 | 1.33E +08 | + | AK055 957 | 657302 | 45.83 |
| 657302_29427305_AK055 957.1_4_Unmethyl | ACATTTAAAATTTATAATTAAAAAAAAAACAACACCAACAACACAAATACCCCAAACACTAAATAAAAC AACAACCTAAAATAAAACTAACCTAAAACAAAAACCAAAACACCCACCCAA (SEQ ID NO:48) | 12 | 1.33E +08 | 1.33E +08 | + | AK055 957 | 657302 | 55 |
| 657302_29427305_AK055 957.1_5_Unmethyl | AAATAAAACAACAACCTAAAATAAAACTAACCTAAAACAAAAACCAAAACACCCACCCAAACAAAAAC AAACAAAAAAAACCCAAACCAAAATCAACCACCCCACACAACACACTACACAA (SEQ ID NO:49) | 12 | 1.33E +08 | 1.33E +08 | + | AK055 957 | 657302 | 70 |
| 657302_29427305_AK055 957.1_6_Unmethyl | ACAAAAACAAACAAAAAAACCCAAACCAAAATCAACCACCCCACACAACACACTACACAAAAACCACA CAACTCCAAATCTCACCTACACCAACACATCCCTCACAAACACACTCCTCCC (SEQ ID NO:50) | 12 | 1.33E +08 | 1.33E +08 | + | AK055 957 | 657302 | 74.17 |
| 657302_29427305_AK055 957.1_7_Unmethyl | AAACCACACAACTCCAAATCTCACCTACACCAACACATCCCTCACAAACACACTCCTCCCACACAAAAA CCCCTTCCCCAAAACACACACCTAAAACTACACACCACAACCCACCCTATC (SEQ ID NO:51) | 12 | 1.33E +08 | 1.33E +08 | + | AK055 957 | 657302 | 75.83 |
| 657302_29427305_AK055 957.1_8_Unmethyl | ACACACAAAAACCCCTTCCCCAAAACACACACCTAAAACTACACACCACAACCCACCCTATCTAACTAAAA CACAAACCCAAATAAATCCCAAAAAAACAAATCAAAACAAAAAACCACACC (SEQ ID NO:52) | 12 | 1.33E +08 | 1.33E +08 | + | AK055 957 | 657302 | 75.83 |
| 657302_29427305_AK055 957.1_9_Unmethyl | TAACTAAAACACAAACCCAAATAAATCCCAAAAAAACAAATCAAAACAAAAAACCACACCCAAAAACA ATAAAAACCACACACTACTAAACAACATACTATCTCCCAACAACATAAAAAC (SEQ ID NO:53) | 12 | 1.33E +08 | 1.33E +08 | + | AK055 957 | 657302 | 69.17 |
| 657302_29427305_AK055 957.1_10_Unmethyl | CAAAAACAATAAAAACCACACACTACTAAACAACATACTATCTCCCAACAACATAAAAACAAAAAATTC ACCAACATATCTAAAACAAACAACAATTTTAACCAACAACTACATAAACCT (SEQ ID NO:54) | 12 | 1.33E +08 | 1.33E +08 | + | AK055 957 | 657302 | 57.5 |
| 657302_29427305_AK055 957.1_11_Unmethyl | AAAAAATTCACCAACATATCTAAAACAAACAACAATTTTAACCAACAACTACATAAACCTTAAACATCC TAACCAATAAAAATAATAAAAAACCTATCACTAAAAAACCTATCCCTAAAC (SEQ ID NO:55) | 12 | 1.33E +08 | 1.33E +08 | + | AK055 957 | 657302 | 55.83 |
| 657302_29427305_AK055 957.1_12_Unmethyl | TAAACATCCTAACCAATAAAAATAATAAAAAACCTATCACTAAAAAACCTATCCCTAAACAACACATAA AAAAAATAAAACCCAACAACACTAACTAAACCACCACAAAATACCAAAACA (SEQ ID NO:56) | 12 | 1.33E +08 | 1.33E +08 | + | AK055 957 | 657302 | 62.5 |
| 657302_29427305_AK055 957.1_13_Unmethyl | AACACATAAAAAAAATAAAACCCAACAACACTAACTAAACCACCACAAAATACCAAAACATATTACCTA AAAACCAAATTTCAAAACATCATTTATCTTCCCAAACCCACCAACTTTCAA (SEQ ID NO:57) | 12 | 1.33E +08 | 1.33E +08 | + | AK055 957 | 657302 | 58.33 |
| 657302_29427305_AK055 957.1_14_Unmethyl | TATTACCTAAAAACCAAATTTCAAAACATCATTTATCTTCCCAAACCCACCAACTTTCAACATTCTCCAAC CTCACCACACCCCCACCTCAAACCATCCCCACCCAAACACTTATCCCCA (SEQ ID NO:58) | 12 | 1.33E +08 | 1.33E +08 | + | AK055 957 | 657302 | 63.33 |
| 657302_29427305_AK055 957.1_15_Unmethyl | CATTCTCCAACCTCACCACACCCCCACCTCAAACCATCCCCACCCAAACACTTATCCCCACAAAAAACTA AACACACTAAAATAAACCCAACAACTACCACCAAAAAAAAAACCCAACTC (SEQ ID NO:59) | 12 | 1.33E +08 | 1.33E +08 | + | AK055 957 | 657302 | 75 |
| 657302_29427305_AK055 957.1_16_Unmethyl | CAAAAAACTAAACACACTAAAATAAACCCAACAACTACCACCAAAAAAAAAACCCAACTCTAAACACA ACCATCAAAAAACTCAAAAACTACAACACCACTTCTACATTAATTTCCACTT (SEQ ID NO:60) | 12 | 1.33E +08 | 1.33E +08 | + | AK055 957 | 657302 | 66.67 |
| 657302_29427306_SEPT.1 _1_Unmethyl | ACTCCTTCCTTCACACCTTCCTTCAAAAACATCTACTCCTAACAAAATCTACTTCCTACTCTCAAAAAACC CTTATTATAAAAAAAAAAAAACATCACCCATCCCTAACTTCTCTAACAA (SEQ ID NO:61) | 17 | 7536 8596 | 7536 8716 | + | SEPT | 657302 | 58.33 |
| 657302_29427306_SEPT.1 _2_Unmethyl | CTCAAAAAACCCTTATTATAAAAAAAAAAAAACATCACCCATCCCTAACTTCTCTAACAACCATATTCCA TCCCCACCCTATACCCCTTCTCCCAAACAATACCTTCTCCAAAACTCACC (SEQ ID NO:62) | 17 | 7536 8656 | 7536 8776 | + | SEPT | 657302 | 65.83 |
| 657302_29427306_SEPT.1 _3_Unmethyl | CCATATTCCATCCCCACCCTATACCCCTTCTCCCAAACAATACCTTCTCCAAAACTCACCCAAAAAAATA CAACAATAACCCCCAAAACAATAATCATAATAAAAATATTAACTACAAAA (SEQ ID NO:63) | 17 | 7536 8716 | 7536 8836 | + | SEPT | 657302 | 71.67 |
| 657302_29427306_SEPT.1 _4_Unmethyl | CAAAAAAATACAACAATAACCCCCAAAACAATAATCATAATAAAAATATTAACTACAAAAATACCCTCA ATAAATAAAAATTAATAACCTCTCACTAATACCATAAAACTCACATATTCA (SEQ ID NO:64) | 17 | 7536 8776 | 7536 8896 | + | SEPT | 657302 | 70.83 |
| 657302_29427306_SEPT.1 _5_Unmethyl | ATACCCTCAATAAATAAAAATTAATAACCTCTCACTAATACCATAAAACTCACATATTCACCCTACACCC CTCAACTCTTAAACCCACAAACCAAAATCCTACCTACCAACCACATACAC (SEQ ID NO:65) | 17 | 7536 8836 | 7536 8956 | + | SEPT | 657302 | 71.67 |
| 657302_29427306_SEPT.1 _6_Unmethyl | CCCTACACCCCTCAACTCTTAAACCCACAAACCAAAATCCTACCTACCAACCACATACACTACCATTTAA CCCTTACAAACACAAAACACACAACAACAATAACAAAAAACTTTATTTAA (SEQ ID NO:66) | 17 | 7536 8896 | 7536 9016 | + | SEPT | 657302 | 69.17 |
| 657302_29427306_SEPT.1 _7_Unmethyl | TACCATTTAACCCTTACAAACACAAAACACACAACAACAATAACAAAAAACTTTATTTAACTACCCAAA TACAACCTCCTACAAAAAAACCCTACACCCAAAAAAAAAAAAAAAATCTCTT (SEQ ID NO:67) | 17 | 7536 8956 | 7536 9076 | + | SEPT | 657302 | 61.67 |
| 557302_29427306_SEPT.1 _8_Unmethyl | CTACCCAAATACAACCTCCTACAAAAAAACCCTACACCCAAAAAAAAAAAAAATCTCTTCCCCTCTAA ACACCCACCCTCCTCACCATAACCCAACCTCCACATCCACCCACATCTAAC (SEQ ID NO:68) | 17 | 7536 9016 | 7536 9136 | + | SEPT | 657302 | 69.17 |
| 657302_29427306_SEPT.1 _9_Unmethyl | CCCCTCTAAACACCCACCCTCCTCACCATAACCCAACCTCCACATCCACCCACATCTAACCACAACAAAA CACCCAAAAAAAAAAACTAAAACCACATCTCTCACCATCCCCTAAACACA (SEQ ID NO:69) | 17 | 7536 9076 | 7536 9196 | + | SEPT | 657302 | 80.83 |
| 657302_29427306_SEPT.1 _10_Unmethyl | CACAACAAAACACCCAAAAAAAAAAACTAAAACCACATCTCTCACCATCCCCTAAACACAAACCAAACA AAAAAAAAAAAAACACTCCAATCATATACCCAAAACTATCCCCCAACAACC (SEQ ID NO:70) | 17 | 7536 9136 | 7536 9256 | + | SEPT | 657302 | 82.5 |
| 657302_29427306_SEPT.1 _11_Unmethyl | AACCAAACAAAAAAAAAAAAACACTCCAATCATATACCCAAAACTATCCCCCAACAACCACTCAAACC CCAACCCCCCAAACCTAACCTTAACAAACAAACAAAACAACCAATACAAAA (SEQ ID NO:71) | 17 | 7536 9196 | 7536 9316 | + | SEPT | 657302 | 77.5 |
| 657302_29427306_SEPT.1 _12_Unmethyl | ACTCAAACCCCAACCCCCCAAACCTAACCTTAACAAACAAACAAAACAACCAATACAAAACAAAAAAA CCAATACAAATACAAAAACCTAATCCACCCAAAAAACAAAAACAAAACAAA (SEQ ID NO:72) | 17 | 7536 9256 | 7536 9375 | + | SEPT | 657302 | 78.99 |
| 657302_29427307_SPINT 2.1_1_Unmethyl | ATCATACCCTAATTTTCTAACAACCCAACCTCTAATCCCTAAACTTAACCTTCCCCATCACAACTTTCATC ACTTTATACTAAACTTCCACTATCACTACTCTAAACCATTCCTATTTAA (SEQ ID NO:73) | 19 | 3875 4618 | 3875 4738 | + | SPINT2 | 657302 | 52.5 |
| 657302_29427307_SPINT 2.1_2_Unmethyl | ACCTCTAATCCCTAAACTTAACCTTCCCCATCACAACTTTCATCACTTTATACTAAACTTCCACTATCACT ACTCTAAACCATTCCTATTTAATAACTCAAAAACCAATCTAACATAACC (SEQ ID NO:74) | 19 | 3875 4645 | 3875 4765 | + | SPINT2 | 657302 | 55 |
| 657302_29427308_SPINT 2.2_1_Unmethyl | TCCCCAACATTAAACCCTAAAACATAAACCCAATAAACTTTAAAATTCAAAAAAAAATTCACAAAAAA CTACAACAAAACAAACAAAAAACCACAAACTTCCAAAAAACACATATCTAC (SEQ ID NO:75) | 19 | 3875 4774 | 3875 4894 | + | SPINT2 | 657302 | 54.17 |
| 657302_29427308_SPINT 2.2_2_Unmethyl | ACAAAAAAACTACAACAAAACAAACAAAAAACCACAAACTTCCAAAAAACACATATCTACCACCCCCTC CTCCCACCCTAAAACCAATCCTAAAACAAAAACCCTCCTCCAACATCATCA (SEQ ID NO:76) CACCCCCTCCTCCCACCCTAAAACCAATCCTAAAACAAAAACCCTCCTCCAACATCATCACCAAACCCAA AAAAAAAATAACAAATACTCAACAAACAAACACCCCACCCCACCCCACCA (SEQ ID NO:77) | 19 | 3875 4834 | 3875 4954 | + | SPINT2 | 657302 | 65 |
| 657302_29427308_SPINT 2.2_3_Unmethyl | CACCCCCTCCTCCCACCCTAAAACCAATCCTAAAACAAAAACCCTCCTCCAACATCATCACCAAACCCAA AAAAAAAATAACAAATACTCAACAAACAAACACCCCACCCCACCCCACCA (SEQ ID NO:77) | 19 | 3875 4894 | 3875 5014 | + | SPINT2 | 657302 | 73.33 |
| 657302_29427309_APC.1 _1_Unmethyl | CTTTTCTAATAACTATTTTTAATTCAAATATAATTCAAATAATCTATCTAACAAATCATCACTCTAACAAC TCAATAACTTATAATATAAAATTATTCATTATAATTCATTTAATATTAT (SEQ ID NO:78) | 5 | 1.12E +08 | 1.12E +08 | + | APC | 657302 | 25 |
| 657302_29427309_APC.1 _2_Unmethyl | ACTCTAACAACTCAATAACTTATAATATAAAATTATTCATTATAATTCATTTAATATTATTATTTCTCTAT ACTACAAAAATCATAACAATCAAAATATAATTTATTACTCTCCCTCCCA (SEQ ID NO:79) | 5 | 1.12E +08 | 1.12E +08 | + | APC | 657302 | 30.83 |
| 657302_29427309_APC.1 _3_Unmethyl | TATTTCTCTATACTACAAAAATCATAACAATCAAAATATAATTTATTACTCTCCCTCCCACCTCCAACATC TTATACTAATCCTTCTACCCTACAAACCTCCCCCAACTCTTTACTATAC (SEQ ID NO:80) | 5 | 1.12E +08 | 1.12E +08 | + | APC | 657302 | 48.33 |
| 657302_29427309_APC.1 _4_Unmethyl | CCTCCAACATCTTATACTAATCCTTCTACCCTACAAACCTCCCCCAACTCTTTACTATACATATCAACTAC CATCAACTTCCTTACTTACTAAAAACTAAAACCACAAAAACATACCCCC (SEQ ID NO:81) | 5 | 1.12E +08 | 1.12E +08 | + | APC | 657302 | 60.83 |
| 657302_29427309_APC.1 _5_Unmethyl | ATATCAACTACCATCAACTTCCTTACTTACTAAAAACTAAAACCACAAAAACATACCCCCAAAAAATAC AAAACTAAAACTAAACAAACTATACAATTAAACAAAACCCTATACCCCACT (SEQ ID NO:82) | 5 | 1.12E +08 | 1.12E +08 | + | APC | 657302 | 68.33 |
| 657302_29427309_APC.1 _6_Unmethyl | AAAAAATACAAAACTAAAACTAAACAAACTATACAATTAAACAAAACCCTATACCCCACTACAAAATAC AAATCAAAAAACAAAAAAAAAAACAACTATATAATCCACTAAATACAAACC (SEQ ID NO:83) | 5 | 1.12E +08 | 1.12E +08 | + | APC | 657302 | 69.17 |
| 657302_29427309_APC.1 _7_Unmethyl | ACAAAATACAAATCAAAAAACAAAAAAAAAAACAACTATATAATCCACTAAATACAAACCAAAACACT CCCCATTCCCATCAAAAACCCACCAATTAACTAAATATAAACACACATAACC (SEQ ID NO:84) | 5 | 1.12E +08 | 1.12E +08 | + | APC | 657302 | 69.17 |
| 657302_29427309_APC.1 _8Unmethyl | AAAACACTCCCCATTCCCATCAAAAACCCACCAATTAACTAAATATAAACACACATAACCAACATATAA CTATATTAATACAACCCACCAAAATATCACTAAAAACAAAATAAAAATACT (SEQ ID NO:85) | 5 | 1.12E +08 | 1.12E +08 | + | APC | 657302 | 65.83 |
| 657302_29427309_APC.1 _9_Unmethyl | AACATATAACTATATTAATACAACCCACCAAAATATCACTAAAAACAAAATAAAAATACTACCAAACTC AAAAATAAAATAAATACTAAAACCACCATAACCAAACTTACTACAAAAAAA (SEQ ID NO:86) | 5 | 1.12E +08 | 1.12E +08 | + | APC | 657302 | 63.33 |
| 657302_29427309_APC. 1 _10_Unmethyl | ACCAAACTCAAAAATAAAATAAATACTAAAACCACCATAACCAAACTTACTACAAAAAAAAAAAAAA AATAATTTTCCCTCACACTATCTTAAACCAATAACCTTTCCTTAACACAAAA (SEQ ID NO:87) | 5 | 1.12E +08 | 1.12E +08 | + | APC | 657302 | 63.33 |
| 657302_29427310_HOXA 1_1_Unmethyl | CAACTAAAAAATTATAATCCTATAATCCAAAAAATAAACTCAAACTAAACAAATCCCCAAATCACCACT ACTAAATATAAAATATTCCAAAAAAAAATTCATTCTTACATTATCCATCTA (SEQ ID NO:88) | 7 | 2713 5414 | 2713 5534 | + | HOXA1 | 657302 | 54.17 |
| 657302_29427310_HOXA 1_2_Unmethyl | ATCACCACTACTAAATATAAAATATTCCAAAAAAAAATTCATTCTTACATTATCCATCTATCACTAAATA ACCTAATCCTACAAAACCCAACATAACTATACCAACTTTCTCACTTCCTC (SEQ ID NO:89) | 7 | 2713 5474 | 2713 5594 | + | HOXA1 | 657302 | 53.33 |
| 657302_29427310_HOXA 1_3_Unmethyl | TCACTAAATAACCTAATCCTACAAAACCCAACATAACTATACCAACTTTCTCACTTCCTCCATAAAACCA AAAAAAAAAAATAATATAAATATACAATACACAAAAAAAAAAACAAAAAA (SEQ ID NO:90) | 7 | 2713 5534 | 2713 5654 | + | HOXA1 | 657302 | 57.5 |
| 657302_29427310_HOXA 1_4_Unmethyl | CATAAAACCAAAAAAAAAAAAATAATATAAATATACAATACACAAAAAAAAAAACAAAAAAACAAAAA ACACTAAAAAAAAAAACACATAACAATATCAACCAATAACTAAACCTCCTACAA (SEQ ID NO:91) | 7 | 2713 5594 | 2713 5714 | + | HOXA1 | 657302 | 61.67 |
| 657302_29427310_HOXA 1_5_Unmethyl | ACAAAAAACACTAAAAAAAAAACACATAACAATATCAACCAATAACTAAACCTCCTACAAAAATTTACC AACTTCCACAATAATAAATCACCATTTTAATAACATTTAAATCCCCAACAC (SEQ ID NO:92) | 7 | 2713 5654 | 2713 5774 | + | HOXA1 | 657302 | 60 |
| 657302_29427310_HOXA 1_6_Unmethyl | AAATTTACCAACTTCCACAATAATAAATCACCATTTTAATAACATTTAAATCCCCAACACTCCACCATCT AAATAACACACAATCACCCCCCCAAACAACCTAAACAACAACAACTACTA (SEQ ID NO:93) | 7 | 2713 5714 | 2713 5834 | + | HOXA1 | 657302 | 64.17 |
| 657302_29427310_HOXA 1_7_Unmethyl | TCCACCATCTAAATAACACACAATCACCCCCCAAACAACCTAAACAACAACAACTACTACAACAACTA CAAAAACCAATTTAAAATACTAAAACAAAAAAAAACAAAAACTACATTCTA (SEQ ID NO:94) | 7 | 2713 5774 | 2713 5894 | + | HOXA1 | 657302 | 65.83 |
| 657302_29427310_HOXA 1_8_Unmethyl | CAACAACTACAAAAACCAATTTAAAATACTAAAACAAAAAAAAACAAAAACTACATTCTACACACACCC AACTCCACTACCCACCCCACCAAACCTCCAAAAAATAAAAACTAAAAAACA (SEQ ID NO:95) | 7 | 2713 5834 | 2713 5954 | + | HOXA1 | 657302 | 70.83 |
| 657302_29427310_HOXA 1_9_Unmethyl | CACACACCCAACTCCACTACCCACCCCACCAAACCTCCAAAAAATAAAAACTAAAAAACATCCCCCACT CCCACCCCCTCCCCACCATTCAATAAAAAATAAACTAACAAAAAATAAAAA (SEQ ID NO:96) | 7 | 2713 5894 | 2713 6014 | + | HOXA1 | 657302 | 73.33 |
| 657302_29427310_HOXA 1_10_Unmethyl | TCCCCCACTCCCACCCCCTCCCCACCATTCAATAAAAATAAACTAACAAAAATAAAAAAAAAAAAA ACTCCCAACTCTCTCAAAACAAAAATCAATAAACCAAAACTCACCAAATAA (SEQ ID NO:97) | 7 | 2713 5954 | 2713 6074 | + | HOXA1 | 657302 | 67.5 |
| 657302_29427310_HOXA 1_11_Unmethyl | AAAAAAAAAACTCCCAACTCTCTCAAAACAAAAATCAATAAACCAAAACTCACCAAATAACCACAAATA CACCAACCCAACCCACAACACACCCAACCAAAAAACAAAAAATCCAACTAA (SEQ ID NO:98) | 7 | 2713 6014 | 2713 6134 | + | HOXA1 | 657302 | 75 |
| 657302_29427310_HOXA 1_12_Unmethyl | CCACAAATACACCAACCCAACCCACAACACACCCAACCAAAAAACAAAAAATCCAACTAACACCACAC CCCAAATTCCCAAACCACCTCCTCTATTCTAAAACTAAACTAAAAAACCATA (SEQ ID NO:99) | 7 | 2713 6074 | 2713 6194 | + | HOXA1 | 657302 | 74.17 |
| 657302_29427310_HOXA 1_13_Unmethyl | CACCACACCCCAAATTCCCAAACCACCTCCTCTATTCTAAAACTAAACTAAAAAACCATAAAACTATAAA AAACACATAAAACCATAATAAAAAACAAAACTAAACCACCAACTCTTCAA (SEQ ID NO:100) | 7 | 2713 6134 | 2713 6254 | + | HOXA1 | 657302 | 69.17 |
| 657302_29427310_HOXA 1_14_Unmethyl | AAACTATAAAAAACACATAAAACCATAATAAAAAACAAAACTAAACCACCAACTCTTCAAACTCAAAAT AAAAAAAAAAAAACACAAAAAACTAACTAAAAAAAAACTCAAATAAACATAA (SEQ ID NO:101) | 7 | 2713 6194 | 2713 6314 | + | HOXA1 | 657302 | 67.5 |
| 657302_29427310_HOXA 1_15_Unmethyl | ACTCAAAATAAAAAAAAAAAAACACAAAAAACTAACTAAAAAAAACTCAAATAAACATAAAAAAAAC AAAAACAAAAAAAAAAAACTTCCCTTCTTCCAAAAAAATCTTCAAAACCCTCTC (SEQ ID NO:102) | 7 | 2713 6254 | 2713 6374 | + | HOXA1 | 657302 | 60 |
| 657302_29427310_HOXA 1_16_Unmethyl | AAAAAACAAAAACAAAAAAAAAAACTTCCCTTCTTCCAAAAAAATCTTCAAAACCCTCTCCCCACAACC CCTCTCATCATTAACATAACAATAAAAAATTTCTATAATTCAACTTAAAAA (SEQ ID NO:103) | 7 | 2713 6314 | 2713 6434 | + | HOXA1 | 657302 | 51.67 |
| 657302_29427310_HOXA 1_17_Unmethyl | CCCACAACCCCTCTCATCATTAACATAACAATAAAAAATTTCTATAATTCAACTTAAAAAAACAAATAAA CCCTAAAAACTCAAAACTCACCAAAAAAAACCAAAAACAACCAAACTCTT (SEQ ID NO:104) | 7 | 2713 6374 | 2713 6494 | + | HOXA1 | 657302 | 60.83 |
| 657302_29427310_HOXA 1_18_Unmethyl | AACAAATAAACCCTAAAAACTCAAAACTCACCAAAAAAAACCAAAAACAACCAAACTCTTCTTCCCCAC CTTCCCTCTCTCATCACTCTCCACCCCTTTCTCTTTCCCACTCAATTTTAC (SEQ ID NO:105) | 7 | 2713 6434 | 2713 6554 | + | HOXA1 | 657302 | 64.17 |
| 657302_29427310_HOXA 1_19_Unmethyl | CTTCCCCACCTTCCCTCTCTCATCACTCTCCACCCCTTTCTCTTTCCCACTCAATTTTACACCAAAAACCCT CCAAAATACAAAACTACTCAACCACCAAATTTTTAAAAATAAAAAACA (SEQ ID NO:106) | 7 | 2713 6494 | 2713 6614 | + | HOXA1 | 657302 | 62.5 |
| 657302_29427310_HOXA 1_20_Unmethyl | ACCAAAAACCCTCCAAAATACAAAACTACTCAACCACCAAATTTTTAAAAATAAAAAACAAAAAAAA AAATAACACTAACAAACATAACCAACACAAAAACCAAACAATACACTACAAA (SEQ ID NO:107) | 7 | 2713 6554 | 2713 6674 | + | HOXA1 | 657302 | 72.5 |
| 657302_29427310_HOXA 1_21_Unmethyl | AAAAAAAAAAATAACACTAACAAACATAACCAACACAAAAACCAAACAATACACTACAAACCATCTAC CAACACCCTAAAACCCAAAAACCTCCACACTCCCACATAAACCTCACAAAAC (SEQ ID NO:108) | 7 | 2713 6614 | 2713 6734 | + | HOXA1 | 657302 | 77.5 |
| 657301_29427287_PRDM 2_1_Unmethyl | ATATCACCACCACCATCACCACCACACTCCTCAAAAAAAAAACCAACATCCCAACACAAACCCAAAAA CCACCCACCCACACCACTCCTTACCCACACCCACCACACCAACACCTCAAA (SEQ ID NO:109) | 1 | 1402 6768 | 1402 6888 | | PRDM 2 | 657301 | 81.67 |
| 657301_29427287_PRDM 2_2_Unmethyl | ACCCAAAAACCACCCACCCACACCACTCCTTACCCACACCCACCACACCAACACCTCAAAACACCAAAA ACCACCACCACCACCACTATTTCACCAACCCCAACACCCACAACCACACCA (SEQ ID NO:110) | 1 | 1402 6708 | 1402 6828 | - | PRDM 2 | 657301 | 85 |
| 65701_29427287_PRDM 2_3_Unmethyl | ACACCAAAAACCACCACCACCACCACTATTTCACCAACCCCAACACCCACAACCACACCACCACCATCTT AACTCCAATCAAAAATAACATCAAACAACAAAACAATAACCTACAAAACT (SEQ ID NO:111) | 1 | 1402 6648 | 1402 6768 | - | PRDM 2 | 657301 | 80 |
| 657301_29427287_PRDM 2_4_Unmethyl | CCACCATCTTAACTCCAATCAAAAATAACATCAAACAACAAAACAATAACCTACAAAACTAAAAAACTC CAAAACCCCCAATCTCCCACAATCCCAAAACCCAACCCTTAACCCTACAC (SEQ ID NO:112) | 1 | 1402 6588 | 1402 6708 | - | PRDM 2 | 657301 | 74.17 |
| 657301_29427287_PRDM 2_5_Unmethyl | AAAAAACTCCAAAACCCCCAATCTCCCCACAATCCCAAAACCCAACCCTTAACCCTACACCATCACCCA ATAACCACCACCCAACCACCCCTCATAAATCACCACAAATCCCATAACAAC (SEQ ID NO:113) | 1 | 1402 6528 | 1402 6648 | - | PRDM 2 | 657301 | 75.83 |
| 65701_29427287_PRDM 2_6_Unmethyl | CATCACCCAATAACCACCACCCAACCACCCCTCATAAATCACCACAAATCCCATAACAACACCTCAAAA AAAAACCCCAACAACTAACATCACAACAAATCCAACCACACCCTAAAACTA (SEQ ID NO:114) | 1 | 1402 6468 | 1402 6588 | - | PRDM 2 | 657301 | 73.33 |
| 657301_29427287_PRDM 2_7_Unmethyl | ACCTCAAAAAAAAACCCCAACAACTAACATCACAACAAATCCAACCACACCCTAAAACTAAAATCCTAC ATAATTCAAAAATTCTCAAAAAATCAAAAAAACTAAAAAAAATATTAAAAA (SEQ ID NO:115) | 1 | 1402 6408 | 1402 6528 | - | PRDM 2 | 657301 | 60 |
| 657301_29427287_PRDM 2_8_Unmethyl | AAATCCTACATAATTCAAAAATTCTCAAAAAATCAAAAAAACTAAAAAAAATATTAAAAAAACATATCT ATCATTAAAAATCATTAAATATCTAACAAATACATAAACATAAACCTTATA (SEQ ID NO:116) | 1 | 1402 6348 | 1402 6468 | - | PRDM 2 | 657301 | 41.67 |
| 657301_29427287_PRDM 2_9_Unmethyl | AACATATCTATCATTAAAAATCATTAAATATCTAACAAATACATAAACATAAACCTTATACTTCTACATT TAAATCTATACCAAAAAAATCCAACTCCAAACAAACAAACACAACTACTA (SEQ ID NO:117) | 1 | 1402 6288 | 1402 6408 | - | PRDM 2 | 657301 | 42.5 |
| 657301_29427288_RUNX 3_1_Unmethyl | AACCAAAACCACACAAAACTAAAAACAACAAAAACCACCAACCAAACATAAACAACACACAAAATCCC ATATAAAATAAAAACTCTTAAATCAAAATAATATACAAAACAAAAAAAATAA (SEQ ID NO:118) | 1 | 2525 7244 | 2525 7364 | - | RUNX3 | 657301 | 66.67 |
| 657301_29427288_RUNX 3_2_Unmethyl | AAAATCCCATATAAAATAAAAACTCTTAAATCAAAATAATATACAAAACAAAAAAAATAAATAACCTCT TTAAAACAACTCCCAATACAACATCACCAACCCTAAAACCCCACAACCCCC (SEQ ID NO:119) | 1 | 2525 7184 | 2525 7304 | - | RUNX3 | 657301 | 65.83 |
| 657301_29427288_RUNX 3_3_Unmethyl | ATAACCTCTTTAAAACAACTCCCAATACAACATCACCAACCCTAAAACCCCACAACCCCCAACCCAAAA TTACAAAAATCACAAACCCAAAACAACAAAAACTAAAAAAACCCAACCACA (SEQ ID NO:120) | 1 | 2525 7124 | 2525 7244 | - | RUNX3 | 657301 | 72.5 |
| 657301_29427288_RUNX 3_4_Unmethyl | AACCCAAAATTACAAAAATCACAAACCCAAAACAACAAAAACTAAAAAAACCCAACCACAACCAACAA AAAAAAAAAACAAAAAAATTACACCCCAACATCAAAAAACTACAACCCAAAA (SEQ ID NO:121) | 1 | 2525 7064 | 2525 7184 | - | RUNX3 | 657301 | 70.83 |
| 657301_29427288_RUNX 3_5_Unmethyl | ACCAACAAAAAAAAAAAACAAAAAAATTACACCCCAACATCAAAAAACTACAACCCAAAAAAAAACAA CAAAAACACCTTCCATAAAACCCAAACATTCTAAACAAATTTCTAACATTTA (SEQ ID NO:122) | 1 | 2525 7004 | 2525 7124 | - | RUNX3 | 657301 | 65 |
| 657301_29427288_RUNX 3_6_Unmethyl | AAAAACAACAAAAACACCTTCCATAAAACCCAAACATTCTAAACAAATTTCTAACATTTACCCCAAACT CCCAAAACTCTCAAAAACCCTAAACTATAACACTAAAACCTCCTCCACAAA (SEQ ID NO:123) | 1 | 2525 6944 | 2525 7064 | - | RUNX3 | 657301 | 69.17 |
| 657301_29427288_RUNX 3_7_Unmethyl | CCCCAAACTCCCAAAACTCTCAAAAACCCTAAACTATAACACTAAAACCTCCTCCACAAAATAACACCTT CCACCCCTCCCCATTAAACAACCTCCAACAAACCCCATTCCTCCCCACAA (SEQ ID NO:124) | 1 | 2525 6884 | 2525 7004 | - | RUNX3 | 657301 | 80 |
| 657301_29427288_RUNX 3_8_Unmethyl | ATAACACCTTCCACCCCTCCCCATTAAACAACCTCCAACAAACCCCATTCCTCCCCACAAACACCACCAA AATACCCACAATAAAAACTCCACCAATTAACTATACAACACATCACTCCA (SEQ ID NO:125) | 1 | 2525 6824 | 2525 6944 | - | RUNX3 | 657301 | 77.5 |
| 657301_29427288_RUNX 3_9_Unmethyl | ACACCACCAAAATACCCACAATAAAAACTCCACCAATTAACTATACAACACATCACTCCACCAACCCCA CCCCACAAACCCCAAAAATACTAACCCCACACAAACAACCACAACCCCACC (SEQ ID NO:126) | 1 | 2525 6764 | 2525 6884 | - | RUNX3 | 657301 | 82.5 |
| 657301_29427288_RUNX 3_10_Unmethyl | CCAACCCCACCCCACAAACCCCAAAAATACTAACCCCACACAAACAACCACAACCCCACCACTTAATTC TAAAAAATTTATTCTAAAACTACAACCACAAAATCAAAACAACCACAAACA (SEQ ID NO:127) | 1 | 2525 6704 | 2525 6824 | - | RUNX3 | 657301 | 80 |
| 657301_29427288_RUNX 3_11_Unmethyl | ACTTAATTCTAAAAAATTTATTCTAAAACTACAACCACAAAATCAAAACAACCACAAACAAACTTCAAA ACAAAAAACAACAACAACAACACAACCCCACACAAACCCCACCACAACCCA (SEQ ID NO:128) | 1 | 2525 6644 | 2525 6764 | - | RUNX3 | 657301 | 78.33 |
| 657301_29427289_PFKP.1 _1_Unmethyl | CCCCCACCCAACCCCAACACCAATAAACAATAACAAACAAAACCCAAACATATAAAAAAACTACAAA AAAAAAAACACAAACACAACTAAAAACAAAAACCAAAACTAAAACAAATATA (SEQ ID NO:129) | 10 | 3109 570 | 3109 690 | - | PFKP | 657301 | 84.17 |
| 657301_29427289_PFKP.1 _2_Unmethyl | ACTACAAAAAAAAAAACACAAACACAACTAAAAACAAAAACCAAAACTAAAACAAATATAAACAAAA ACATCTACACAAAAATCATCATAAATAAAAACCACACTAACAATACAAAAAAC (SEQ ID NO:130) | 10 | 3109 510 | 3109 630 | - | PFKP | 657301 | 80.83 |
| 657301_29427289_PFKP.1 _3_Unmethyl | AACAAAAACATCTACACAAAAATCATCATAAATAAAAACCACACTAACAATACAAAAAACAACAAAAA CAAAAATAACACCACAAATAAACAAATCCCTACTAATAAAACCACATCACAA (SEQ ID NO:131) | 10 | 3109 450 | 3109 570 | - | PFKP | 657301 | 74.17 |
| 657301_29427289_PFKP.1 _4_Unmethyl | AACAAAAACAAAAATAACACCACAAATAAACAAATCCCTACTAATAAAACCACATCACAAATACACAA ACCTCACAAATAAACTAAAAAACTCTAATTAAAAACTCTAAAAATAACAAAA (SEQ ID NO:132) | 10 | 3109 390 | 3109 510 | - | PFKP | 657301 | 70.83 |
| 657301_29427289_PFKP.1 _5_Unmethyl | ATACACAAACCTCACAAATAAACTAAAAAACTCTAATTAAAAACTCTAAAAATAACAAAAACACCACAA ATAAAACAAAAACAACATCCAAAAAAAAAAAAACCACAAAAAACCAAAATC (SEQ ID NO:133) | 10 | 3109 330 | 3109 450 | - | PFKP | 657301 | 74.17 |
| 657301_29427289_PFKP.1 _6_Unmethyl | ACACCACAAATAAACAAAAACAACATCCAAAAAAAAAAAAACCACAAAAAACCAAAATCACATCATT TACAAAACACACCAAAACAAAACAAAATAAAACACCAAAAACATCTCCCAAA (SEQ ID NO:134) | 10 | 3109 270 | 3109 390 | - | PFKP | 657301 | 76.67 |
| 657301_29427289_PFKP.1 _7_Unmethyl | ACATCATTTACAAAACACACCAAAACAAAACAAAATAAAACACCAAAAACATCTCCCAAAAAAACAAA AAAAACCATAAATAAACACAAACACCAAAACAAATAAAAACCCCACAATTAC (SEQ ID NO:135) | 10 | 3109 210 | 3109 330 | - | PFKP | 657301 | 75 |
| 657301_29427289_PFKP.1 _8_Unmethyl | AAAACAAAAAAAACCATAAATAAACACAAACACCAAAACAAATAAAAACCCCACAATTACAAAAAACA CCAATAACAAAAAAAAATAAAATAAAAACACAAAAACCCCACCTAAACACAA (SEQ ID NO:136) | 10 | 3109 150 | 3109 270 | - | PFKP | 657301 | 77.5 |
| 657301_29427289_PFKP.1 _9_Unmethyl | AAAAAACACCAATAACAAAAAAAAATAAAATAAAAACACAAAAACCCCACCTAAACACAAAAACTCAC AACAAACCCAACCACTCAAACCATTATAAAAACCAAACCCAACCACACACAC (SEQ ID NO:137) | 10 | 3109 090 | 3109 210 | - | PFKP | 657301 | 81.67 |
| 657301_29427289_PFKP.1 _10_Unmethyl | AAACTCACAACAAACCCAACCACTCAAACCATTATAAAAACCAAACCCAACCACACACACAACTTCTAA TAACCCACAAAATTCCTCCTAAAACAACACTAAAAACCTCAAAACTCAACT (SEQ ID NO:138) | 10 | 3109 030 | 3109 150 | - | PFKP | 657301 | 77.5 |
| 657301_29427290_GSTP1 .1_1_Unmethyl | CACAACCTCCAAACCTTATAAAAATAATCCCACCCCACTCCACCCCAATACTAAATCACAACACCAACCA CTCTTCTAAAAAATCCCACAAACTCCCACCAACCCCAACCCCAACAACCA (SEQ ID NO:139) | 11 | 6735 1154 | 6735 1274 | - | GSTP1 | 657301 | 78.33 |
| 657301_29427290_GSTP1 .1_2_Unmethyl | ACACCAACCACTCTTCTAAAAAATCCCACAAACTCCCACCAACCCCAACCCCAACAACCACTACACCCC AAACATCAACCACAAAAAAACACCCTAAAATCCCCAAAATCACCACACAAC (SEQ ID NO:140) | 11 | 6735 1094 | 6735 1214 | - | GSTP1 | 657301 | 83.33 |
| 657301_29427290_GSTP1 .1_3_Unmethyl | CTACACCCCAAACATCAACCACAAAAAAACACCCTAAAATCCCCAAAATCACCACACAACTAACCAAAA AAACCTTTCCCTCTTTCCCAAATCCCCAACAAAACCTAAAAAATAAACAAA (SEQ ID NO:141) | 11 | 6735 1034 | 6735 1154 | - | GSTP1 | 657301 | 72.5 |
| 657301_29427290_GSTP1 .1_4_Unmethyl | TAACCAAAAAAACCTTTCCCTCTTTCCCAAATCCCCAACAAAACCTAAAAAATAAACAAACAACAAAAA AAAAACCACAACAAAATATACACAACAAACTAACACACCAAAACATCACAA (SEQ ID NO:142) | 11 | 6735 0974 | 6735 1094 | - | GSTP1 | 657301 | 66.67 |
| 657301_29427290_GSTP1 .1_5_Unmethyl | CAACAAAAAAAAAACCACAACAAAATATACACAACAAACTAACACACCAAAACATCACAAAAAAAAT TCCCTAAAACCACTACAATCCCAAAACTTACACACCCACTTCACAAAACAAA (SEQ ID NO:143) | 11 | 6735 0914 | 6735 1034 | - | GSTP1 | 657301 | 66.67 |
| 657301_29427290_GSTP1 .1_6_Unmethyl | AAAAAAATTCCCTAAAACCACTACAATCCCAAAACTTACACACCCACTTCACAAAACAAAAAAAAAAA TAAAAACCACTTAAAAAAAAAAAAAATTACTTTATTTTATTTTATTTTATT (SEQ ID NO:144) | 11 | 6735 0855 | 6735 0974 | - | GSTP1 | 657301 | 50.42 |
| 657301_29427291_WIF1. 1_1_Unmethyl | ACCTACCCCCTCCTCCTACTCTCACAAACTCCTTAACACCCAAACCAAAAAACAACACACCCAACCATCT AAACAAAAACAACCCTAACTAAAAAAACTACAACACAACAAAATATCTAA (SEQ ID NO:145) | 12 | 6551 5035 | 6551 5155 | - | WIF1 | 657301 | 69.17 |
| 657301_29427291_WIF1. 1_2_Unmethyl | CCAACCATCTAAACAAAAACAACCCTAACTAAAAAAACTACAACACAACAAAATATCTAACAACACCAA ATTACATAAATACAACACAAAAAATTTTCCCAACAACAAAAAAATCCTAAA (SEQ ID NO:146) | 12 | 6551 4975 | 6551 5095 | - | WIF1 | 657301 | 65 |
| 657301_29427292_WIF1. 2_1_Unmethyl | TCTACTCTCCCCATTTCCCTCCCCCAAAACCTCCCTTAACCCAAAAAAATAACAAATAATATCCCAAAAA TCTCTAAATACCCTTCTCCAAATCCACCAACCCTACACACCCACTTCACA (SEQ ID NO:147) | 12 | 6551 5458 | 6551 5578 | - | WIF1 | 657301 | 67.5 |
| 657301_29427292_WIF1. 2_2_Unmethyl | ATCCCAAAAATCTCTAAATACCCTTCTCCAAATCCACCAACCCTACACACCCACTTCACAAACACTCCAC TAAACACACCACACTATAAATACAACCTCAAAAATCCCTCACAACCCCAC (SEQ ID NO:148) | 12 | 6551 5398 | 6551 5518 | - | WIF1 | 657301 | 73.33 |
| 657301_29427292_WIF1. 2_3_Unmethyl | AACACTCCACTAAACACACCACACTATAAATACAACCTCAAAAATCCCTCACAACCCCACCCCCAAAAA AACCCCACAACACCCCCAAATAACAACCACCCAAACCTCACAAACCCCACT (SEQ ID NO:149) | 12 | 6551 5338 | 6551 5458 | - | WIF1 | 657301 | 80.83 |
| 657301_29427292_WIF1. 2_4_Unmethyl | CCCCAAAAAAACCCCACAACACCCCCAAATAACAACCACCCAAACCTCACAAACCCCACTCCTCACTCA CACCTCACTCACACCAACCCTTCCCACTCTTCTATTCTCACTCTATTTACC (SEQ ID NO:150) | 12 | 6551 5278 | 6551 5398 | - | WIF1 | 657301 | 75 |
| 657301_29427292_WIF1. 2_5_Unmethyl | CCTCACTCACACCTCACTCACACCAACCCTTCCCACTCTTCTATTCTCACTCTATTTACCCCACTAACTACT AACCTCACCAACTTTACCAATCTTACATCTCTACCACCCCCACTCCCA (SEQ ID NO:151) | 12 | 6551 5218 | 6551 5338 | - | WIF1 | 657301 | 66.67 |
| 657301_29427292_WIF1. 2_6_Unmethyl | CCACTAACTACTAACCTCACCAACTTTACCAATCTTACATCTCTACCACCCCCACTCCCACCCACACCCCA TCTTCTTACACAACTCACACCCACTAATCCCCCCCTCCTCCTCCCACA (SEQ ID NO:152) | 12 | 6551 5159 | 6551 5278 | - | WIF1 | 657301 | 73.11 |
| 657301_29427293_AK055 957.1_1_Unmethyl | ACAAAAATCAACACAAAAACAATACTACAACCTCTAAACTTCCTAACAACCATATCCAAAACCAAACTC CTCCTCCAACAACAACCACCAAACTCACTTCAATACACTCAACTTCTCACA (SEQ ID NO:153) | 12 | 1.33E +08 | 1.33E +08 | - | AK055 957 | 657301 | 68.33 |
| 657301_29427293_AK055 957.1_2_Unmethyl | ACCAAACTCCTCCTCCAACAACAACCACCAAACTCACTTCAATACACTCAACTTCTCACAAAAACAAACA TCTAAACAAAAACAACCCAAAACAAAAATATAACAAAACTAAAAAACACT (SEQ ID NO:154) | 12 | 1.33E +08 | 1.33E +08 | - | AK055 957 | 657301 | 75 |
| 657301_29427293_AK055 957.1_3_Unmethyl | AAAACAAACATCTAAACAAAAACAACCCAAAACAAAAATATAACAAAACTAAAAAACACTAAAAACTA ATAAACTTAAAAAAACAAACAACACTCTAAAATTTAACTCCCAAATAATACA (SEQ ID NO:155) | 12 | 1.33E +08 | 1.33E +08 | - | AK055 957 | 657301 | 63.33 |
| 657301_29427293_AK055 957.1_4_Unmethyl | AAAAACTAATAAACTTAAAAAAACAAACAACACTCTAAAATTTAACTCCCAAATAATACATTCCAACAC TTCACAACAACTCAATCAACACTACTAAATTCCACCCCTCCTATACATTAC (SEQ ID NO:156) | 12 | 1.33E +08 | 1.33E +08 | - | AK055 957 | 657301 | 59.17 |
| 657301_29427293_AK055 957.1_5_Unmethyl | TTCCAACACTTCACAACAACTCAATCAACACTACTAAATTCCACCCCTCCTATACATTACTCAAAAACAA ATTTCTTAATAACAAACCCCTCACTATTCCCATTAACCAAAACACCCAAA (SEQ ID NO:157) | 12 | 1.33E +08 | 1.33E +08 | - | AK055 957 | 657301 | 61.67 |
| 657301_29427293_AK055 957.1_6_Unmethyl | TCAAAAACAAATTTCTTAATAACAAACCCCTCACTATTCCCATTAACCAAAACACCCAAAACCCACACAA CCATTAACTAAAATTATTATCTATTTCAAACACATTAACAAATTCCCCAC (SEQ ID NO:158) | 12 | 1.33E +08 | 1.33E +08 | - | AK055 957 | 657301 | 55.83 |
| 657301_29427293_AK055 957.1_7_Unmethyl | ACCCACACAACCATTAACTAAAATTATTATCTATTTCAAACACATTAACAAATTCCCCACCTCTACATTA CCAAAAAACAACATATTACCTAACAACATACAACTCCCATTACCTTTAAA (SEQ ID NO:159) | 12 | 1.33E +08 | 1.33E +08 | - | AK055 957 | 657301 | 58.33 |
| 657301_29427293_AK055 957.1_8_Unmethyl | CTCTACATTACCAAAAAACAACATATTACCTAACAACATACAACTCCCATTACCTTTAAACACAACTCTC CACCCCAACCCACCCCTCTAAAACCCATCCAAATCTACACCTCAACTAAA (SEQ ID NO:160) | 12 | 1.33E +08 | 1.33E +08 | - | AK055 957 | 657301 | 68.33 |
| 657301_29427293_AK055 957.1_9_Unmethyl | CACAACTCTCCACCCCAACCCACCCCTCTAAAACCCATCCAAATCTACACCTCAACTAAACAAAACAAAC TACAACACACAATCTTAAACATACACTTCAAAAAAAAAACCCTCACATAA (SEQ ID NO:161) | 12 | 1.33E +08 | 1.33E +08 | - | AK055 957 | 657301 | 75.83 |
| 657301_29427293_AK055 957.1_10_Unmethyl | CAAAACAAACTACAACACACAATCTTAAACATACACTTCAAAAAAAAACCCTCACATAAAAAAAACAC ATCTACAAAAAATACACCAACACAAACAAAACCCAAAACCACATAATCTCT (SEQ ID NO:162) | 12 | 1.33E +08 | 1.33E +08 | - | AK055 957 | 657301 | 75.83 |
| 657301_29427293_AK055 957.1_11_Unmethyl | AAAAAACACATCTACAAAAAATACACCAACACAAACAAAACCCAAAACCACATAATCTCTACACAATAC ATCATATAAAACAACCAACCCCAACCCAAATTTCCCTACTCATTTTCATCC (SEQ ID NO:163) | 12 | 1.33E +08 | 1.33E +08 | - | AK055 957 | 657301 | 74.17 |
| 657301_29427293_AK055 957.1_12_Unmethyl | ACACAATACATCATATAAAACAACCAACCCCAACCCAAATTTCCCTACTCATTTTCATCCAAACAAACAT CTTAATTCCCATTCCAAACCAACCCCATCCTAAATCACTACTTCATCCAA (SEQ ID NO:164) | 12 | 1.33E +08 | 1.33E +08 | - | AK055 957 | 657301 | 70 |
| 657301_29427293_AK055 957.1_13_Unmethyl | AAACAAACATCTTAATTCCCATTCCAAACCAACCCCATCCTAAATCACTACTTCATCCAACACTTAAAAC ATTTATATCATTAACATTATTTTCCTCTTAACTACAAACTTTAAATATAT (SEQ ID NO:165) | 12 | 1.33E +08 | 1.33E +08 | - | AK055 957 | 657301 | 54.17 |
| 657301_29427293_AK055 957.1_14_Unmethyl | CACTTAAAACATTTATATCATTAACATTATTTTCCTCTTAACTACAAACTTTAAATATATTCATCTACTCAT AACAACAAATTTAACAAACTTTCATTCCCAAAAAACATATCACACAAT (SEQ ID NO:166) | 12 | 1.33E +08 | 1.33E +08 | - | AK055 957 | 657301 | 45 |
| 657301_29427293_AK055 957.1_15_Unmethyl | TCATCTACTCATAACAACAAATTTAACAAACTTTCATTCCCAAAAAACATATCACACAATCTATATATTTT TCATACAAAAATTAACAAACATCATTACTTCTAAATTTCCACAAAAACT (SEQ ID NO:167) | 12 | 1.33E +08 | 1.33E +08 | - | AK055 957 | 657301 | 43.33 |
| 657301_29427293_AK055 957.1_16_Unmethyl | CTATATATTTTTCATACAAAAATTAACAAACATCATTACTTCTAAATTTCCACAAAAACTAATTCATCCCA CAACTTTACCTAAAAAAAAACATCTAAAACCAAACACAACAACTCCCA (SEQ ID NO:168) | 12 | 1.33E +08 | 1.33E +08 | - | AK055 957 | 657301 | 52.1 |
| 657301_29427294_SEPT.1 _1_Unmethyl | CCACCCCACCCCCACCTCCCAAACAAATCAAATTCCCACACCCACACCAACCTCCCTATCTCACACTAAC TACTCCACCCACCTATCAAAACCAAACCTAAAAAACTAAAACCCAAATAA (SEQ ID NO:169) | 17 | 7536 9254 | 7536 9374 | - | SEPT | 657301 | 79.17 |
| 657301_29427294_SEPT.1 _2_Unmethyl | TCACACTAACTACTCCACCCACCTATCAAAACCAAACCTAAAAAACTAAAACCCAAATAACCACTAAAA AACAATCCTAAACACACAACCAAAACACCCCCCTCCTCCCCACCTAACCCA (SEQ ID NO:170) | 17 | 7536 9194 | 7536 9314 | - | SEPT | 657301 | 78.33 |
| 657301_29427294_SEPT.1 _3_Unmethyl | CCACTAAAAAACAATCCTAAACACACAACCAAAACACCCCCCTCCTCCCCACCTAACCCACACCCAAAA AACAACAAAAAACACAACCTCAACCCCTCCCCCCAAACACCCCACTACAAC (SEQ ID NO:171) | 17 | 7536 9134 | 7536 9254 | - | SEPT | 657301 | 82.5 |
| 657301_29427294_SEPT.1 _4_Unmethyl | CACCCAAAAAACAACAAAAAACACAACCTCAACCCCTCCCCCCAAACACCCCACTACAACCAAATATAA ACAAATATAAAAACCAAACCATAACAAAAAAAACAAACACCCAAAAAAAAA (SEQ ID NO:172) | 17 | 7536 9074 | 7536 9194 | - | SEPT | 657301 | 79.17 |
| 657301_29427294_SEPT.1 _5_Unmethyl | CAAATATAAACAAATATAAAAACCAAACCATAACAAAAAAAACAAACACCCAAAAAAAAAAAAATTCC TCCCCTTCCCCAAACACAAAATCCTTCTACAAAAAACTATATTTAAACAACC (SEQ ID NO:173) | 17 | 7536 9014 | 7536 9134 | - | SEPT | 657301 | 69.17 |
| 657301_29427294_SEPT.1 6_Unmethyl | AAAATTCCTCCCCTTCCCCAAACACAAAATCCTTCTACAAAAAACTATATTTAAACAACCAAACAAAATT CTCTATCACCACCACCACACACTCTACACCTACAAAAATTAAACAACAAC (SEQ ID NO:174) | 17 | 7536 8954 | 7536 9074 | - | SEPT | 657301 | 63.33 |
| 657301_29427294_SEPT.1 _7_Unmethyl | AAACAAAATTCTCTATCACCACCACCACACACTCTACACCTACAAAAATTAAACAACAACACACACAAC TAACAAACAAAATCCCAACCTATAAACTCAAAAACCAAAAAACACAAAACA (SEQ ID NO:175) | 17 | 7536 8894 | 7536 9014 | - | SEPT | 657301 | 69.17 |
| 657301_29427294_SEPT.1 _8_Unmethyl | ACACACAACTAACAAACAAAATCCCAACCTATAAACTCAAAAACCAAAAAACACAAAACAAACATACA AATCCCATAACACCAACAAAAAACCACCAACTCCCACCCACCAAAAACACCC (SEQ ID NO:176) | 17 | 7536 8834 | 7536 8954 | - | SEPT | 657301 | 71.67 |
| 657301_29427294_SEPT.1 _9_Unmethyl | AACATACAAATCCCATAACACCAACAAAAAACCACCAACTCCCACCCACCAAAAACACCCCTACAACTA ACACCCCCACCACAACCACCACCCCAAAAACCACCACTACACCCTCCTAAA (SEQ ID NO:177) | 17 | 7536 8774 | 7536 8894 | - | SEPT | 657301 | 70.83 |
| 657301_29427294_SEPT.1 _10_Unmethyl | CTACAACTAACACCCCCACCACAACCACCACCCCAAAAACCACCACTACACCCTCCTAAATAAACCCTA AAAAAAACACTATCCAAAAAAAAAAACACAAAACAAAAATAAAACACAACT (SEQ ID NO:178) | 17 | 7536 8714 | 7536 8834 | - | SEPT | 657301 | 70.83 |
| 657301_29427294_SEPT.1 _11_Unmethyl | TAAACCCTAAAAAAAACACTATCCAAAAAAAAAAACACAAAACAAAAATAAAACACAACTATCAAAAA AACCAAAAACAAACAACACCTCCCTTCCTCCACAATAAAAACCTCCTAAAAA (SEQ ID NO:179) | 17 | 7536 8654 | 7536 8774 | - | SEPT | 657301 | 65 |
| 657301_29427294_SEPT.1 _12_Unmethyl | ATCAAAAAAACCAAAAACAAACAACACCTCCCTTCCTCCACAATAAAAACCTCCTAAAAACAAAAAATA AACCTTATCAAAAACAAACATTTCCAAAAAAAAATATAAAAAAAAAAACTA (SEQ ID NO:180) | 17 | 7536 8594 | 7536 8714 | - | SEPT | 657301 | 57.5 |
| 657301_29427295_SPINT 2.1_1_Unmethyl | ACCATACCAAACTAATCCCTAAATCACCAAACAAAAACAACCCAAAATAATAATAACAAAAACTTAACA CAAAATAATAAAAACTATAATAAAAAAAAACCAAACTTAAAAATCAAAAACT (SEQ ID NO:181) | 19 | 3875 4644 | 3875 4764 | | SPINT2 | 657301 | 54.17 |
| 657301_29427295_SPINT 2.1_2_Unmethyl | CAAACAAAAACAACCCAAAATAATAATAACAAAAACTTAACACAAAATAATAAAAACTATAATAAAAA AAACCAAACTTAAAAATCAAAAACTAAATCACTAAAAAACCAAAACATAATA (SEQ ID NO:182) | 19 | 3875 4617 | 3875 4737 | | SPINT2 | 657301 | 52.5 |
| 657301_29427296_SPINT 2.2_1_Unmethyl | ACAAAACAAAACAAAACATCTACCCACTAAACACCTACCACCCTTCCCTTAAACTTAATAACAACATCA AAAAAAAACCTTCACTCTAAAACTAACCTCAAAATAAAAAAAAAAAACAAC (SEQ ID NO:183) | 19 | 3875 4892 | 3875 5012 | | SPINT2 | 657301 | 74.17 |
| 657301_29427296_SPINT 2.2_2_Unmethyl | ACAACATCAAAAAAAAACCTTCACTCTAAAACTAACCTCAAAATAAAAAAAAAAAACAACAAACACAC ATTTCTTAAAAACCTATAATTTTCTACCCACCTCACCACAACTTTCCTATAA (SEQ ID NO:184) | 19 | 3875 4832 | 3875 4952 | - | SPINT2 | 657301 | 65 |
| 657301_29427296_SPINT 2.2_3_Unmethyl | AAACACACATTTCTTAAAAACCTATAATTTTCTACCCACCTCACCACAACTTTCCTATAAATTCCTTCCTA AATTTCAAAATTCACTAAACTCATACTCCAAAACTTAATACTAAAAAC (SEQ ID NO:185) | 19 | 3875 4773 | 3875 4892 | - | SPINT2 | 657301 | 53.78 |
| 657301_29427297_APC.1 _1_Unmethyl | CCCTATACCAAAAAAAAACCATCAATTTAAAACAATACAAAAAAAAACCACCTTCCCCCCTCCCCCCAC AACAAACCTAACCATAATAACTCCAACACCTACCCCATTTCCAAATCCAAC (SEQ ID NO:186) | 5 | 1.12E +08 | 1.12E +08 | - | APC | 657301 | 63.33 |
| 657301_29427297_APC.1 _2_Unmethyl | TCCCCCCACAACAAACCTAACCATAATAACTCCAACACCTACCCCATTTCCAAATCCAACAACACCTCCA TTCTATCTCCAATAACACCCTAACAAACTACACCAATACAACCACATATC (SEQ ID NO:187) | 5 | 1.12E +08 | 1.12E +08 | | APC | 657301 | 63.33 |
| 657301_29427297_APC.1 _3_Unmethyl | AACACCTCCATTCTATCTCCAATAACACCCTAACAAACTACACCAATACAACCACATATCAATCACATAC ACCCACACCCAACCAATCAACAAACTCCCAACAAAAATAAAAAACACCCT (SEQ ID NO:188) | 5 | 1.12E +08 | 1.12E +08 | | APC | 657301 | 65.83 |
| 657301_29427297_APC.1 _4_Unmethyl | AATCACATACACCCACACCCAACCAATCAACAAACTCCCAACAAAATAAAAAACACCCTAATCCACAT CCAACAAATTACACAACTACTTCTCTCTCCACTTCCCAACCCACACTCCAC (SEQ ID NO:189) | 5 | 1.12E +08 | 1.12E +08 | | APC | 657301 | 69.17 |
| 657301_29427297_APC.1 _5_Unmethyl | AATCCACATCCAACAAATTACACAACTACTTCTCTCTCCACTTCCCAACCCACACTCCACAATAAAACAC AAAACCCCACCCAACCACACAACCTACCTAACCCTAACCCCATACCCCTC (SEQ ID NO:190) | 5 | 1.12E +08 | 1.12E +08 | - | APC | 657301 | 69.17 |
| 657301_29427297_APC.1 _6_Unmethyl | AATAAAACACAAAACCCCACCCAACCACACAACCTACCTAACCCTAACCCCATACCCCTCAAAAATATA CCCTCACAACCCCAATCCCCAACAAACAAAAAAATTAATAACAATTAACAC (SEQ ID NO:191) | 5 | 1.12E +08 | 1.12E +08 | - | APC | 657301 | 68.33 |
| 657301_29427297_APC.1 _7_Unmethyl | AAAAATATACCCTCACAACCCCAATCCCCAACAAACAAAAAAATTAATAACAATTAACACACATAATAA AAAATCAAAAAAAATCCACAAAACAAAAAAATTAACACAAAATACCAAAAA (SEQ ID NO:192) | 5 | 1.12E +08 | 1.12E +08 | - | APC | 657301 | 60.83 |
| 657301_29427297_APC.1 _8_Unmethyl | AAAAATATACCCTCACAACCCCAATCCCCAACAAACAAAAAAATTAATAACAATTAACACACATAATAA AAAATCAAAAAAAAATCCACAAAACAAAAAAATTAACACAAAATACCAAAAA (SEQ ID NO:192) ACATAATAAAAAATCAAAAAAAAATCCACAAAACAAAAAAATTAACACAAAATACCAAAAATAAAAAA AAAATAATAAATTACATCTCAATTACTATAATTTTTACAACACAAAAAAACA (SEQ ID NO:193) | 5 | 1.12E +08 | 1.12E +08 | - | APC | 657301 | 48.33 |
| 657301_29427297_APC.1 _9_Unmethyl | TAAAAAAAAAAAATAATAAATTACATCTCAATTACTATAATTTTTACAACACAAAAAAACAATAATATTA AATAAATTACAATAAATAATTTTACATTACAAATCACTAAATTATCAAAAT (SEQ ID NO:194) | 5 | 1.12E +08 | 1.12E +08 | - | APC | 657301 | 30.83 |
| 657301_29427297_APC.1 _10_Unmethyl | ATAATATTAAATAAATTACAATAAATAATTTTACATTACAAATCACTAAATTATCAAAATAATAACTTAT TAAATAAATCACTCAAATTATATTTAAATTAAAAATAATTACCAAAAAAA (SEQ ID NO:195) | 5 | 1.12E +08 | 1.12E +08 | | APC | 657301 | 25 |
| 657301_29427298_HOXA 1_1_Unmethyl | AACCCTATAAAACCCACACAAAAACACAAAAACTCCTAAATCTCAAAACACCAACAAATAACCTACAAC ACACCACCCAACCCCCACACTAACCACATCCACCAACATCATCTCTCTCCC (SEQ ID NO:196) | 7 | 2713 6615 | 2713 6735 | - | HOXA1 | 657301 | 77.5 |
| 657301_29427298_HOXA 1_2_Unmethyl | ACCTACAACACACCACCCAACCCCCACACTAACCACATCCACCAACATCATCTCTCTCCCCCACCTCCTA CCCCTAAAAATCCAACAATCAAATAACTCCACATCCCAAAAAACTCCCAA (SEQ ID NO:197) | 7 | 2713 6555 | 2713 6675 | - | HOXA1 | 657301 | 73.33 |
| 657301_29427298_HOXA 1_3_Unmethyl | CCACCTCCTACCCCTAAAAATCCAACAATCAAATAACTCCACATCCCAAAAAACTCCCAATACAAAACT AAATAAAAAAAAAAAAAAAACAAAAAACAACAAAAAAAAAAAAAATAAAAAA (SEQ ID NO:198) | 7 | 2713 6495 | 2713 6615 | - | HOXA1 | 657301 | 62.5 |
| 657301_29427298_HOXA 1_4_Unmethyl | TACAAAACTAAATAAAAAAAAAAAAAAAACAAAAAACAACAAAAAAAAAAAAAATAAAAAAAAAAAAC CCAACCACCCCCAACCTTTTCCAACAAACTCTAAATTTCCAAAACTCATCCAC (SEQ ID NO:199) | 7 | 2713 6435 | 2713 6555 | - | HOXA1 | 657301 | 63.33 |
| 657301_29427298_HOXA 1_5_Unmethyl | AAAAAACCCAACCACCCCCAACCTTTTCCAACAAACTCTAAATTTCCAAAACTCATCCACCCCTCCAAAT CAAATTACAAAAACTCCTCATTACCATACTAATAACAAAAAAAACTATAA (SEQ ID NO:200) | 7 | 2713 6375 | 2713 6495 | - | HOXA1 | 657301 | 60.83 |
| 657301_29427298_HOXA 1_6_Unmethyl | CCCTCCAAATCAAATTACAAAAACTCCTCATTACCATACTAATAACAAAAAAAACTATAAAAAAAAAT TTCAAAAACCCTCCTAAAAAAAAAAAAATTTCTTTTCTTACCCTCATCTCC (SEQ ID NO:201) | 7 | 2713 6315 | 2713 6435 | - | HOXA1 | 657301 | 52.5 |
| 657301_29427298_HOXA 1_7_Unmethyl | AAAAAAAATTTCAAAAACCCTCCTAAAAAAAAAAAAATTTCTTTTCTTACCCTCATCTCCTTCACACCCA CCCAAATTCCTCCCAACCAACCCTCTACACTCTTCCCCCTCCATTCCAAA (SEQ ID NO:202) | 7 | 2713 6255 | 2713 6375 | - | HOXA1 | 657301 | 60 |
| 657301_29427298_HOXA 1_8_Unmethyl | TTCACACCCACCCAAATTCCTCCCAACCAACCCTCTACACTCTTCCCCCTCCATTCCAAACTTAAAAAACC AATAACCCAACCTCACCCTCCACCACAATTCTATACACTCCTCACAACC (SEQ ID NO:203) | 7 | 2713 6195 | 2713 6315 | - | HOXA1 | 657301 | 66.67 |
| 657301_29427298_HOXA 1_9_Unmethyl | CTTAAAAAACCAATAACCCAACCTCACCCTCCACCACAATTCTATACACTCCTCACAACCCCACAATCTC CCAACCCAACCTCAAAACAAAAAAAATAACCTAAAAACCCAAAACACAAT (SEQ ID NO:204) | 7 | 2713 6135 | 2713 6255 | - | HOXA1 | 657301 | 69.17 |
| 657301_29427298_HOXA 1_10_Unmethyl | CCACAATCTCCCAACCCAACCTCAAAACAAAAAAATAACCTAAAAACCCAAAACACAATATCAACCAA ATTCCCCACCTTCCAACTAAACACACTACAAACTAAACCAACATACTTACA (SEQ ID NO:205) | 7 | 2713 6075 | 2713 6195 | - | HOXA1 | 657301 | 74.17 |
| 657301_29427298_HOXA 1_11_Unmethyl | ATCAACCAAATTCCCCACCTTCCAACTAAACACACTACAAACTAAACCAACATACTTACAACCACCCAAC AAACTCTAACCCACTAATTCCCACCCCAAAAAAACCAAAAACCCTCTTCC (SEQ ID NO:206) | 7 | 2713 6015 | 2713 6135 | - | HOXA1 | 657301 | 75 |
| 657301_29427298_HOXA 1_12_Unmethyl | ACCACCCAACAAACTCTAACCCACTAATTCCCACCCCAAAAAAACCAAAAACCCTCTTCCCTTCTCACCT CTCACCAATTCATCTTTCATTAAACAATAAAAAAAAAACAAAAACAAAAA (SEQ ID NO:207) | 7 | 2713 5955 | 2713 6075 | - | HOXA1 | 657301 | 68.33 |
| 657301_29427298_HOXA 1_13_Unmethyl | CTTCTCACCTCTCACCAATTCATCTTTCATTAAACAATAAAAAAAAACAAAAACAAAAAACACCTCCCA ACCCCCACCTCCCAAAAACCTAACAAAACAAACAACAAAATCAAACACAC (SEQ ID NO:208) | 7 | 2713 5895 | 2713 6015 | - | HOXA1 | 657301 | 73.33 |
| 657301_29427298_HOXA 1_14_Unmethyl | ACACCTCCCAACCCCCACCTCCCAAAAACCTAACAAAACAAACAACAAAATCAAACACACACAAAACAC AACTTTTACTCTTCTTCACTCCAACACTCCAAATCAACCTTTACAATCACC (SEQ ID NO:209) | 7 | 2713 5835 | 2713 5955 | - | HOXA1 | 657301 | 70 |
| 657301_29427298_HOXA 1_15_Unmethyl | ACAAAACACAACTTTTACTCTTCTTCACTCCAACACTCCAAATCAACCTTTACAATCACCACAACAACTA CCACCACCTAAACTACCTAAAAAAACAACTACACATCACCTAAACAACAA (SEQ ID NO:210) | 7 | 2713 5775 | 2713 5895 | - | HOXA1 | 657301 | 66.67 |
| 657301_29427298_HOXA 1_16_Unmethyl | ACAACAACTACCACCACCTAAACTACCTAAAAAAACAACTACACATCACCTAAACAACAAAACACCAAA AATTTAAATACCACTAAAACAATAATCCATCACTACAAAAACCAACAAACT (SEQ ID NO:211) | 7 | 2713 5715 | 2713 5835 | - | HOXA1 | 657301 | 65 |
| 657301_29427298_HOXA 1_17_Unmethyl | AACACCAAAAATTTAAATACCACTAAAACAATAATCCATCACTACAAAAACCAACAAACTTTTACAAAA AACTCAACCATTAACTAACACCATCACATACCCCTCCTCCAACATCCTCCA (SEQ ID NO:212) | 7 | 2713 5655 | 2713 5775 | - | HOXA1 | 657301 | 59.17 |
| 657301_29427298_HOXA 1_18_Unmethyl | TTTACAAAAAACTCAACCATTAACTAACACCATCACATACCCCTCCTCCAACATCCTCCACCCTCCCACC CCCCCTCTTACACACTATACATTCATATCATTTTTCTTCTCCAACCCCAT (SEQ ID NO:213) | 7 | 2713 5595 | 2713 5715 | - | HOXA1 | 657301 | 60.83 |
| 657301_29427298_HOXA 1_19_Unmethyl | CCCTCCCACCCCCCCTCTTACACACTATACATTCATATCATTTTTCTTCTCCAACCCCATAAAAAAAATAA AAAAATTAACACAATCACACCAAACTTCACAAAACCAAATCACTCAATA (SEQ ID NO:214) | 7 | 2713 5535 | 2713 5655 | - | HOXA1 | 657301 | 58.33 |
| 657301_29427298_HOXA 1_20_Unmethyl | AAAAAAAATAAAAAAATTAACACAATCACACCAAACTTCACAAAACCAAATCACTCAATAACAAATAA ACAATACAAAAATAAACTCCTTCCTAAAATACCCCATACTTAACAATAACAA (SEQ ID NO:215) | 7 | 2713 5475 | 2713 5595 | - | HOXA1 | 657301 | 53.33 |
| 657301_29427298_HOXA 1_21_Unmethyl | ACAAATAAACAATACAAAAATAAACTCCTTCCTAAAATACCCCATACTTAACAATAACAACTCAAAAAC CTACTCAACCCAAACCTACCCCTCAAACCATAAAATTACAACTTTCCAATC (SEQ ID NO:216) CAACAACTACGTCTATCTACCTAAAATTAAATCTTTTTAATATAAATCCAAACGTAAAAATATAAAATCT ACGTTTATATACTTATCAAATATTTAATAACCTCCAACGATAAACATACC (SEQ ID NO:217) | 7 | 2713 5415 | 2713 5535 | | HOXA1 | 657301 | 53.33 |
| 657302_29427299_PRDM 2_1_Methyl | CAACAACTACGTCTATCTACCTAAAATTAAATCTTTTTAATATAAATCCAAACGTAAAAATATAAAATCT ACGTTTATATACTTATCAAATATTTAATAACCTCCAACGATAAACATACC (SEQ ID NO:217) | 1 | 1402 6288 | 1402 6408 | + | PRDM 2 | 657302 | 42.5 |
| 657302_29427299_PRDM 2_2_Methyl | TATAAAATCTACGTTTATATACTTATCAAATATTTAATAACCTCCAACGATAAACATACCCCCCCAACAC CTCCTTCAATCTTTTTAATTCTTTAAAAATCTTTAAACCACGCAAAATCC (SEQ ID NO:218) | 1 | 1402 6348 | 1402 6468 | + | PRDM 2 | 657302 | 41.67 |
| 657302_29427299_PRDM 2_3_Methyl | TATAAAATCTACGTTTATATACTTATCAAATATTTAATAACCTCCAACGATAAACATACCCCCCCAACAC CTCCTTCAATCTTTTTAATTCTTTAAAAATCTTTAAACCACGCAAAATCC (SEQ ID NO:218) CCCCCAACACCTCCTTCAATCTTTTTAATTCTTTAAAAATCTTTAAACCACGCAAAATCCTAACCCTAAAA TACGATCGAACTTACCGCGACGCCAACCGCCGAAATTCCTTCCCGAAAC (SEQ ID NO:219) | 1 | 1402 6408 | 1402 6528 | + | PRDM 2 | 657302 | 60 |
| 657302_29427299_PRDM 2_4_Methyl | TAACCCTAAAATACGATCGAACTTACCGCGACGCCAACCGCCGAAATTCCTTCCCGAAACGCCGCCAT AAAATCCGCGATAACCTACGAAAAACGACTAAATAATAACCATTAAACGACG (SEQ ID NO:220) | 1 | 1402 6468 | 1402 6588 | + | PRDM 2 | 657302 | 73.33 |
| 657302_29427299_PRDM 2_5_Methyl | ACCGCCATAAAATCCGCGATAACCTACGAAAAACGACTAAATAATAACCATTAAACGACGACGCAAAA TCAAAAACCGAACTCTAAAATCGTAAAAAAACCGAAAATCCCGAAACCCCCC (SEQ ID NO:221) | 1 | 1402 6528 | 1402 6648 | + | PRDM 2 | 657302 | 75.83 |
| 657302_29427299_PRDM 2_6_Methyl | ACGCAAAATCAAAAACCGAACTCTAAAATCGTAAAAAAACCGAAAATCCCGAAACCCCCCAACCCCGC AAACCACTACCTCGCCGCCCGACGTCACTTCCGACTAAAATCAAAATAACGA (SEQ ID NO:222) | 1 | 1402 6588 | 1402 6708 | + | PRDM 2 | 657302 | 74.17 |
| 657302_259427299_PRDM 2_7_Methyl | AACCCCGCAAACCACTACCTCGCCGCCCGACGTCACTTCCGACTAAAATCAAAATAACGACGACGCGA CCGCGAACGCCGAAACCGACGAAACAACGACGACGACGACGACCCTCGATAC (SEQ ID NO:223) | 1 | 1402 6648 | 1402 6768 | + | PROM 2 | 657302 | 80 |
| 657302_29427299_PRDM 2_8_Methyl | CGACGCGACCGCGAACGCCGAAACCGACGAAACAACGACGACGACGACGACCCTCGATACTCTAAAA CGCTAACGCGACGAACGCGAATAAAAAATAACGCGAATAAACGACCTCTAAAC (SEQ ID NO:224) | 1 | 1402 6708 | 1402 6828 | + | PRDM 2 | 657302 | 85 |
| 657302_29427299_PRDM 2_9_Methyl | TCTAAAACGCTAACGCGACGAACGCGAATAAAAAATAACGCGAATAAACGACCTCTAAACTCGCGCC GAAACGCTAATCCCTCCCCCCGAAAAACGCGACGACGACGACGACGACGACAC (SEQ ID NO:225) | 1 | 1402 6768 | 1402 6888 | + | PROM 2 | 657302 | 81.67 |
| 6S7302_29427300_RUNX 3_1_Methyl | TAAACCGCGACGAAACCCGCGCGAAACTATACCGCTACCGCCGCCTCCCGCCCCGAAACTCGCCCGCG ACCGCCCCGACTCCGCGACCGCAACCCCAAAACAAATCCTCCAAAATCAAAT (SEQ ID NO:226) | 1 | 2525 6644 | 2525 6764 | + | RUNX3 | 657302 | 78.33 |
| 657302_29427300_RUNX 3_2_Methyl | CGCCCGCGACCGCCCCGACTCCGCGACCGCAACCCCAAAACAAATCCTCCAAAATCAAATAACGAAAC CGCGACCGCCCGCGCGAAATTAATACCCCCGAAACCCGCGAAACGAAACTAA (SEQ ID NO:227) | 1 | 2525 6704 | 2525 6824 | + | RUNX3 | 657302 | 80 |
| 657302_29427300_RUNX 3_3_Methyl | AACGAAACCGCGACCGCCCGCGCGAAATTAATACCCCGAAACCCGCGAAACGAAACTAACGAAACG ACGCGTCGCACAACCAATCGACGAAACCCCCATCGCGAACACCTCGATAACGT (SEQ ID NO:228) | 1 | 2525 6764 | 2525 6884 | + | RUNX3 | 657302 | 82.5 |
| 657302_29427300_RUNX 3_4_Methyl | CGAAACGACGCGTCGCACAACCAATCGACGAAACCCCCATCGCGAACACCTCGATAACGTTCGCGAA AAAAAACGAAACCTACCGAAAACCGCCCAACGAAAAAAAACGAAAAACGCCAC (SEQ ID NO:229) | 1 | 2525 6824 | 2525 6944 | + | RUNX3 | 657302 | 77.5 |
| 657302_29427300_RUNX 3_5_Methyl | TCGCGAAAAAAAACGAAACCTACCGAAAACCGCCCAACGAAAAAAACGAAAAACGCCACCCCGCGA AAAAAACCCCAATACCACAACCCAAAACCCCCGAAAACTCTAAAAACCCGAAA (SEQ ID NO:230) | 1 | 2525 6884 | 2525 7004 | + | RUNX3 | 657302 | 80 |
| 657302_29427300_RUNX 3_6_Methyl | CCCGCGAAAAAAACCCCAATACCACAACCCAAAACCCCCGAAAACTCTAAAAACCCGAAACAAATACT AAAAATTTACTTAAAACGTCCGAATCCCACGAAAAACGCCCTTACCGCCCTC (SEQ ID NO:231) | 1 | 2525 6944 | 2525 7064 | + | RUNX3 | 657302 | 69.17 |
| 657302_29427300_RUNX 3_7_Methyl | CAAATACTAAAAATTTACTTAAAACGTCCGAATCCCACGAAAAACGCCCTTACCGCCCTCTCTCGAATC GTAACTCCCTAACGCTAAAACGCAACCCCTTCGCTCCTCCTCCCCGCTAAC (SEQ ID NO:232) | 1 | 2525 7004 | 2525 7124 | + | RUNX3 | 657302 | 65 |
| 657302_29427300_RUNX 3_8_Methyl | TCTCGAATCGTAACTCCCTAACGCTAAAACGCAACCCCTTCGCTCCTCCTCCCCGCTAACCGCGACCGA ACTTCCCCAACTCTTACTACTTCGAACCTATAACTTCTACAACCCCGAACT (SEQ ID NO:233) | 1 | 2525 7064 | 2525 7184 | + | RUNX3 | 657302 | 70.83 |
| 657302_29427300_RUNX 3_9_Methyl | CGCGACCGAACTTCCCCAACTCTTACTACTTCGAACCTATAACTTCTACAACCCCGAACTAAAAACCGC GAAATCTCAAAACCGATAACGCCGCACTAAAAACCGCCCCAAAAAAATTAC (SEQ ID NO:234) | 1 | 2525 7124 | 2525 7244 | + | RUNX3 | 657302 | 72.5 |
| 657302_29427300_RUNX 3_10_Methyl | AAAAACCGCGAAATCTCAAAACCGATAACGCCGCACTAAAAACCGCCCCAAAAAAATTACTCACCTCC CTCGTCCCGCACATTATTCTAACCCAAAAACCTCCACCCCACACGAAATTTT (SEQ ID NO:235) | 1 | 2525 7184 | 2525 7304 | + | RUNX3 | 657302 | 65.83 |
| 657302_29427300_RUNX 3_11_Methyl | TCACCTCCCTCGTCCCGCACATTATTCTAACCCAAAAACCTCCACCCCACACGAAATTTTACGCGTCGTC CACGCCCGACCGACGACCTTTACTACTCCCAACCCTACGCGACTTTAATC (SEQ ID NO:236) | 1 | 2525 7244 | 2525 7364 | + | RUNX3 | 657302 | 66.67 |
| 657302_29427301_PFKP.1 _1_Methyl | CCGAACCCCGAAACTCCCAACGCCGCCCCAAAAAAAATCTTACGAACCACTAAAAACTATACGCGCGA CCGAACTCGACCCCCACAACGACCCGAACGACCGAACCTACCGCGAACCTCC (SEQ ID NO:237) | 10 | 3109 032 | 3109 152 | + | PFKP | 657302 | 78.33 |
| 657302_29427301_PFKP.1 _2_Methyl | ACGCGCGACCGAACTCGACCCCCACAACGACCCGAACGACCGAACCTACCGCGAACCTCCGCGTCCAA ATAAAATCTCCGCGCCCCCATTCCACCCCTCCCCGCCATCGACGCTCCCCGC (SEQ ID NO:238) | 10 | 3109 092 | 3109 212 | + | PFKP | 657302 | 82.5 |
| 657302_29427301_PFKP.1 _3_Methyl | ACGTCCAAATAAAATCTCCGCGCCCCCATTCCACCCCTCCCCGCCATCGACGCTCCCCGCAACTACGAA ATTTCCACCCGCCCCGACGCTCGCGCCCACTCACGATCTCCCTCGCCCCCC (SEQ ID NO:239) | 10 | 3109 152 | 3109 272 | + | PFKP | 657302 | 77.5 |
| 657302_29427301_PFKP.1 _4_Methyl | AACTACGAAATTTCCACCCGCCCCGACGCTCGCGCCCACTCACGATCTCCCTCGCCCCCCCGAAAAATA CTCCCGACGTTCTATCCCGCCCCGCCCTAACGCGCCCCGCAAACGATATAA (SEQ ID NO:240) | 10 | 3109 *212* | 3109 332 | + | PFKP | 657302 | 74.17 |
| 657302_29427301_PFKP.1 _5_Methyl | CGAAAAATACTCCCGACGTTCTATCCCGCCCCGCCCTAACGCGCCCCGCAAACGATATAACCCCGACCC CCTACGATCTCCCCTCCTCCGAACGCTACCCCTACCCCACCTATAACGCTC (SEQ ID NO:241) | 10 | 3109 272 | 3109 392 | + | PFKP | 657302 | 75.83 |
| 657302_29427301_PFKP.1 _6_Methyl | CCCCGACCCCCTACGATCTCCCCTCCTCCGAACGCTACCCCTACCCCACCTATAACGCTCCCGTCATCTC TAAAACCCCCAACCAAAATCCTCCAACCCACCCGCGAAACTCGCGCACCT (SEQ ID NO:242) | 10 | 3109 332 | 3109 452 | + | PFKP | 657302 | 74.17 |
| 657302_29427301_PFKP.1 _7_Methyl | CCGTCATCTCTAAAACCCCCAACCAAAATCCTCCAACCCACCCGCGAAACTCGCGCACCTACGATACGA CCCCACTAACAAAAACCCGCCCACCTATAACGCTACCCCTACCTCTACCGC (SEQ ID NO:243) | 10 | 3109 392 | 3109 512 | + | PFKP | 657302 | 71.67 |
| 657302_29427301_PFKP.1 _8_Methyl | ACGATACGACCCCACTAACAAAAACCCGCCCACCTATAACGCTACCCCTACCTCTACCGCCTCCCGCAC CGCTAACGCGACCCCCACCTACGACGACCCCCGCGTAAATACTCCTACCCA (SEQ ID NO:244) | 10 | 3109 452 | 3109 572 | + | PFKP | 657302 | 74.17 |
| 657302_25427301_PFKP.1 _9_Methyl | CTCCCGCACCGCTAACGCGACCCCCACCTACGACGACCCCCGCGTAAATACTCCTACCCACACCCGCCC CAACCCCGACCCCTACCCCTAACCGCGCCCGCGCCCCCTCCCCCGCAACCC (SEQ ID NO:245) | 10 | 3109 512 | 3109 632 | + | PFKP | 657302 | 80.83 |
| 657302_29427301_PFKP.1 _10_Methyl | CACCCGCCCCAACCCCGACCCCTACCCCTAACCGCGCCCGCGCCCCCTCCCCCGCAACCCCTCCCATAC GCTCGAACCCCGCCCGTCACCGCCCATTAACGCTAAAACCGAACGAAAAC (SEQ ID NO:246) | 10 | 3109 572 | 3109 691 | + | PFKP | 657302 | 84.87 |
| 657302_29427302_GSTP1 .1_1_Methyl | ATAAAATAAAATAAAATAAAACAATTTCCTTTCCTCTAAACGACCTCCACCCCTCTCCCCTACCCTATAA AACGAATATACAAACTCCGAAATCGCAACGATCTTAAAAAATTTCCCCCC (SEQ ID NO:247) | 11 | 6735 0856 | 6735 0976 | + | GSTP1 | 657302 | 51.67 |
| 657302_29427302_GSTP1 .1_2_Methyl | TACCCTATAAAACGAATATACAAACTCCGAAATCGCAACGATCTTAAAAAATTTCCCCCCGCGATATCC CGACGCGCCAATTCGCTACGCACACTTCGCTACGATCCTCTTCCTACTATC (SEQ ID NO:248) | 11 | 6735 0916 | 6735 1036 | + | GSTP1 | 657302 | 65.83 |
| 657302_29427302_GSTP1 .1_3_Methyl | ACGATATCCCGACGCGCCAATTCGCTACGCACACTTCGCTACGATCCTCTTCCTACTATCTATTTACTCC CTAAACCCCGCTAAAAACCTAAAAAAAAAAAAAAAACTTCCCCGACCAAC (SEQ ID NO:249) | 11 | 6735 0976 | 6735 1096 | + | GSTP1 | 657302 | 66.67 |
| 657302_29427302_GSTP1 .1_4_Methyl | TATTTACTCCCTAAACCCCGCTAAAAACCTAAAAAAAAAAAAAAAACTTCCCCGACCAACTACGCGACG ACTCCGAAAACTCCAAAACGCCCCTCTACGACCGACGCCCGAAATACAACG (SEQ ID NO:250) | 11 | 6735 1036 | 6735 1156 | + | GSTP1 | 657302 | 73.33 |
| 657302_29427302_GSTP1 .1_5_Methyl | TACGCGACGACTCCGAAAACTCCAAAACGCCCCTCTACGACCGACGCCCGAAATACAACGACCGCCGA AACTAAAACCGACGAAAATCCGCGAAACCCTCCAAAAAAACGACCGACGCCG (SEQ ID NO:251) | 11 | 6735 1096 | 6735 1216 | + | GSTP1 | 657302 | 83.33 |
| 657302_29427302_GSTP1 .1_6_Methyl | ACCGCCGAAACTAAAACCGACGAAAATCCGCGAAACCCTCCAAAAAAACGACCGACGCCGTAACTCA ACACTAAAACGAAACGAAACGAAACCACCCTTATAAAACTCGAAAACCGCGAA (SEQ ID NO:252) | 11 | 6735 1156 | 6735 1276 | + | GSTP1 | 657302 | 77.5 |
| 657302_29427303_WIF1. 1_1_Methyl | CAAAACCTCCTCGCTACCGAAAAAACTCCTCGTACCGCACCTACGCAACCTAACGCCGTCAAATACTCT ACTACGCTACAACTCCCTCAACCAAAACTATTCCCGTTTAAACGACTAAAC (SEQ ID NO:253) | 12 | 6551 4977 | 6551 5097 | + | WIF1 | 657302 | 65.83 |
| 657302_29427303_WIF1. 1_2_Methyl | AAATACTCTACTACGCTACAACTCCCTCAACCAAAACTATTCCCGTTTAAACGACTAAACGCGTCGCCTC CCGACCTAAATACCAAAAAACCTACGAAAACAAAAAAAAAAAACAAACA (SEQ ID NO:254) | 12 | 6551 5037 | 6551 5156 | + | WIF1 | 657302 | 68.91 |
| 657302_29427304_WIF1. 2_1_Methyl | ACGAAAAAAAAAAAAAAAAACCAACGAACGCGAATCGCGCAAAAAAATAAAACGCGAACGAAAATA AAAACGACAAAAACGTAAAACTAACAAAACTAACGAAACCAACAATCAACGAAA (SEQ ID NO:255) | 12 | 6551 5160 | 6551 5280 | + | WIF1 | 657302 | 73.33 |
| 657302_29427304_WIF1. 2_2_Methyl | AAAATAAAAACGACAAAAACGTAAAACTAACAAAACTAACGAAACCAACAATCAACGAAACAAATAA AACGAAAACAAAAAAACGAAAAAAACTAACGCGAACGAAATACGAACGAAAAA (SEQ ID NO:256) | 12 | 6551 5220 | 6551 5340 | + | WIF1 | 657302 | 65.83 |
| 657302_29427304_WIF1. 2_3_Methyl | CAAATAAAACGAAAACAAAAAAACGAAAAAAACTAACGCGAACGAAATACGAACGAAAAATAAAACC CGCGAAACCTAAACGACCGCCACTTAAAAACGCTATAAAACCCCCCCGAAAAC (SEQ ID NO:257) | 12 | 6551 5280 | 6551 5400 | + | WIF1 | 657302 | 75 |
| 657302_29427304_WIF1. 2_4_Methyl | TAAAACCCGCGAAACCTAAACGACCGCCACTTAAAAACGCTATAAAACCCCCCCGAAAACGAAACCGC GAAAAACCCCCGAAACTACATTCACAATACGATACGCCCAATAAAACGCCCG (SEQ ID NO:258) | 12 | 6551 5340 | 6551 5460 | + | WIF1 | 657302 | 81.67 |
| 657302_29427304_WIF1. 2_5_Methyl | AAAACCGCGAAAAACCCCCGAAACTACATTCACAATACGATACGCCCAATAAAACGCCCGCGAAATAA ACGTATAAAACTAACGAACCCGAAAAAAAACACTCAAAAACCCCTAAAACAT (SEQ ID NO:259) | 12 | 6551 5400 | 6551 5520 | + | WIF1 | 657302 | 71.67 |
| 657302_29427304_WIF1. 2_6_Methyl | CGAAATAAACGTATAAAACTAACGAACCCGAAAAAAAACACTCAAAAACCCCTAAAACATCACTCGCC ACCTCCTCGAACCAAAAAAAACTTCGAAAAAAAAAAATAAAAAAAACAAACA (SEQ ID NO:260) | 12 | 6551 5460 | 6551 5580 | + | WIF1 | 657302 | 66.67 |
| 657302_29427305_AK055 957.1_1_Methyl | AAAAACCGCCGCGCCCGACCTCAAACGTTTTTCTCTAAATAAAACCGCGAAATAAACCAACCCCTATAA AAATTTAAAAATAACGACGTCCGTCAATTTCTATATAAAAAATATACAAAT (SEQ ID NO:261) | 12 | 1.33E +08 | 1.33E +08 | + | AK055 957 | 657302 | 50.83 |
| 657302_29427305_AK055 957.1_2_Methyl | CCCCTATAAAAATTTAAAAATAACGACGTCCGTCAATTTCTATATAAAAAATATACAAATCGTATAACA CGTCTTCTAAAAATAAAAATCTACTAAACTCGCCGTCACGAATAAATAAAC (SEQ ID NO:262) | 12 | 1.33E +08 | 1.33E +08 | + | AK055 957 | 657302 | 43.33 |
| 657302_29427305_AK055 957.1_3_Methyl | CGTATAACACGTCTTCTAAAAATAAAAATCTACTAAACTCGCCGTCACGAATAAATAAACACATTTAAA ATTTATAATTAAAAAAAAAACAACGCCAACGACACAAATACCCCAAACGCT {SEQ ID NO:263} | 12 | 1.33E +08 | 1.33E +08 | + | AK055 957 | 657302 | 45.83 |
| 657302_29427305_AK055 957.1_4_Methyl | ACATTTAAAATTTATAATTAAAAAAAAAACAACGCCAACGACACAAATACCCCAAACGCTAAATAAAA CAACGACCTAAAATAAAACTAACCTAAAACGAAAACCAAAACGCCCGCCCGA (SEQ ID NO:264) | 12 | 1.33E +08 | 1.33E +08 | + | AK055 957 | 657302 | 55 |
| 657302_29427305_AK055 957.1_5_Methyl | AAATAAAACAACGACCTAAAATAAAACTAACCTAAAACGAAAACCAAAACGCCCGCCCGAACGAAAA CGAACAAAAAAACCCGAACCGAAATCGACCGCCCCACACGACGCACTACGCAA (SEQ ID NO:265) | 12 | 1.33E +08 | 1.33E +08 | + | AK055 957 | 657302 | 70 |
| 657302_29427305_AK055 957.1_6_Methyl | ACGAAAACGAACAAAAAAACCCGAACCGAAATCGACCGCCCCACACGACGCACTACGCAAAAACCAC GCGACTCCGAATCTCGCCTACGCCGACGCATCCCTCGCAAACGCGCTCCTCCC (SEQ ID NO:266) | 12 | 1.33E +08 | 1.33E +08 | + | AK055 957 | 657302 | 74.17 |
| 657302_29427305_AK055 957.1_7_Methyl | AAACCACGCGACTCCGAATCTCGCCTACGCCGACGCATCCCTCGCAAACGCGCTCCTCCCACGCGAAA ACCCCTTCCCCGAAACGCACGCCTAAAACTACGCGCCGCAACCCGCCCTATC (SEQ ID NO:267) | 12 | 1.33E +08 | 1.33E +08 | + | AK055 957 | 657302 | 75.83 |
| 657302_29427305_AK055 957.1_8_Methyl | ACGCGAAAACCCCTTCCCCGAAACGCACGCCTAAAACTACGCGCCGCAACCCGCCCTATCTAACTAAA ACGCAAACCCGAATAAATCCCAAAAAAACGAATCGAAACGAAAAACCGCGCC (SEQ ID NO:268) | 12 | 1.33E +08 | 1.33E +08 | + | AK055 957 | 657302 | 75.83 |
| 657302_29427305_AK055 957.1_9_Methyl | TAACTAAAACGCAAACCCGAATAAATCCCAAAAAAACGAATCGAAACGAAAAACCGCGCCCAAAAAC AATAAAAACCGCACGCTACTAAACAACATACTATCTCCCGACAACGTAAAAAC (SEQ ID NO:269) | 12 | 1.33E +08 | 1.33E +08 | + | AK055 957 | 657302 | 69.17 |
| 657302_29427305_AK055 957.1_10_Methyl | CAAAAACAATAAAAACCGCACGCTACTAAACAACATACTATCTCCCGACAACGTAAAAACGAAAAATT CGCCAACGTATCTAAAACAAACAACAATTTTAACCAACGACTACGTAAACCT (SEQ ID NO:270) | 12 | 1.33E +08 | 1.33E +08 | + | AK055 957 | 657302 | 57.5 |
| 657302_29427305_AK055 957.1_11_Methyl | AAAAAATTCGCCAACGTATCTAAAACAAACAACAATTTTAACCAACGACTACGTAAACCTTAAACGTCC TAACCAATAAAAATAATAAAAAACCTATCACTAAAAAACCTATCCCTAAAC (SEQ ID NO:271) | 12 | 1.33E +08 | 1.33E +08 | + | AK055 957 | 657302 | 55.83 |
| 657302_29427305_AK055 957.1_12_Methyl | TAAACGTCCTAACCAATAAAAATAATAAAAAACCTATCACTAAAAAACCTATCCCTAAACAACGCATAA AAAAAATAAAACCCAACAACGCTAACTAAACCGCCGCGAAATACCGAAACG (SEQ ID NO:272) | 12 | 1.33E +08 | 1.33E +08 | + | AK055 957 | 657302 | 62.5 |
| 657302_29427305_AK055 957.1_13_Methyl | AACGCATAAAAAAAATAAAACCCAACAACGCTAACTAAACCGCCGCGAAATACCGAAACGTATTACCT AAAAACCAAATTTCAAAACGTCGTTTATCTTCCCAAACCCACCAACTTTCAA (SEQ ID NO:273) | 12 | 1.33E +08 | 1.33E +08 | + | AK055 957 | 657302 | 58.33 |
| 657302_29427305_AK055 957.1_14_Methyl | TATTACCTAAAAACCAAATTTCAAAACGTCGTTTATCTTCCCAAACCCACCAACTTTCAACGTTCTCCAA CCTCGCCACACCCCCGCCTCGAACCGTCCCCGCCCAAACGCTTATCCCCG (SEQ ID NO:274) | 12 | 1.33E +08 | 1.33E +08 | + | AK055 957 | 657302 | 63.33 |
| 657302_29427305_AK055 957.1_15_Methyl | TATTACCTAAAAACCAAATTTCAAAACGTCGTTTATCTTCCCAAACCCACCAACTTTCAACGTTCTCCAA CCTCGCCACACCCCCGCCTCGAACCGTCCCCGCCCAAACGCTTATCCCCG (SEQ ID NO:274) CGTTCTCCAACCTCGCCACACCCCCGCCTCGAACCGTCCCCGCCCAAACGCTTATCCCCGCGAAAAACT AAACGCACTAAAATAAACCCGACGACTACCGCCGAAAAAAAAACCCGACTC (SEQ ID NO:275) | 12 | 1.33E +08 | 1.33E +08 | + | AK055 957 | 657302 | 75 |
| 657302_29427305_AK055 957.1_16_Methyl | CGAAAAACTAAACGCACTAAAATAAACCCGACGACTACCGCCGAAAAAAAAACCCGACTCTAAACAC GACCGTCAAAAAACTCAAAAACTACAACACCGCTTCTACGTTAATTTCCGCTT (SEQ ID NO:276) | 12 | 1.33E +08 | 1.33E +08 | + | AK055 957 | 657302 | 66.67 |
| 657302_29427306_SEPT.1 _1_Methyl | ACTCCTTCCTTCACACCTTCCTTCGAAAACGTCTACTCCTAACAAAATCTACTTCCTACTCTCAAAAAACC CTTATTATAAAAAAAAAAAAACGTCGCCCGTCCCTAACTTCTCTAACAA (SEQ ID NO:277) | 17 | 7536 8596 | 7536 8716 | + | SEPT | 657302 | 58.33 |
| 657302_29427306_SEPT.1 _2_Methyl | CTCAAAAAACCCTTATTATAAAAAAAAAAAAAACGTCGCCCGTCCCTAACTTCTCTAACAACCGTATTCC ATCCCCGCCCTATACCCCTTCTCCCGAACAATACCTTCTCCAAAACTCACC (SEQ ID NO:278) | 17 | 7536 8656 | 7536 8776 | + | SEPT | 657302 | 65.83 |
| 657302_29427306_SEPT.1 _3_Methyl | CCGTATTCCATCCCCGCCCTATACCCCTTCTCCCGAACAATACCTTCTCCAAAACTCACCCAAAAAAATA CAACGATAACCCCCGAAACGATAATCGTAATAAAAATATTAACTACAAAA (SEQ ID NO:279) | 17 | 7536 8716 | 7536 8836 | + | SEPT | 657302 | 71.67 |
| 657302_29427306_SEPT.1 _4_Methyl | CAAAAAAATACAACGATAACCCCCGAAACGATAATCGTAATAAAAATATTAACTACAAAAATACCCTC GATAAATAAAAATTAATAACCTCTCGCTAATACCATAAAACTCGCATATTCG (SEQ ID NO:280) | 17 | 7536 8776 | 7536 8896 | + | SEPT | 657302 | 70.83 |
| 657302_29427306_SEPT.1 _5_Methyl | ATACCCTCGATAAATAAAAATTAATAACCTCTCGCTAATACCATAAAACTCGCATATTCGCCCTACGCCC CTCGACTCTTAAACCCACAAACCGAAATCCTACCTACCAACCGCGTACGC (SEQ ID NO:281) | 17 | 7536 8836 | 7536 8956 | + | SEPT | 657302 | 71.67 |
| 657302_29427306_SEPT.1 _6_Methyl | CCCTACGCCCCTCGACTCTTAAACCCACAAACCGAAATCCTACCTACCAACCGCGTACGCTACCGTTTA ACCCTTACAAACGCAAAACGCGCGACGACGATAACAAAAAACTTTATTTAA (SEQ ID NO:282) | 17 | 7536 8896 | 7536 9016 | + | SEPT | 657302 | 69.17 |
| 6S7302_29427306_SEPT.1 _7_Methyl | TACCGTTTAACCCTTACAAACGCAAAACGCGCGACGACGATAACAAAAACTTTATTTAACTACCCAAA TACAACCTCCTACAAAAAAACCCTACGCCCGAAAAAAAAAAAAAATCTCTT (SEQ ID NO:283) | 17 | 7536 8956 | 7536 9076 | + | SEPT | 657302 | 61.67 |
| 657302_29427306_SEPT.1 _8_Methyl | CTACCCAAATACAACCTCCTACAAAAAAACCCTACGCCCGAAAAAAAAAAAAAATCTCTTCCCCTCTAA ACGCCCGCCCTCCTCGCCATAACCCGACCTCCACATCCGCCCACATCTAAC (SEQ ID NO:284) | 17 | 7536 9016 | 7536 9136 | + | SEPT | 657302 | 69.17 |
| 657302_29427306_SEPT.1 _9_Methyl | CCCCTCTAAACGCCCGCCCTCCTCGCCATAACCCGACCTCCACATCCGCCCACATCTAACCGCAACGAA ACGCCCGAAAAAAAAAACTAAAACCGCGTCTCTCGCCGTCCCCTAAACGCG (SEQ ID NO:285) | 17 | 7536 9076 | 7536 9196 | + | SEPT | 657302 | 80.83 |
| 657302_29427306_SEPT.1 10_Methyl | CGCAACGAAACGCCCGAAAAAAAAAACTAAAACCGCGTCTCTCGCCGTCCCCTAAACGCGAACCAAAC GAAAAAAAAAAAAACGCTCCGATCGTATACCCAAAACTATCCCCCAACGACC (SEQ ID NO:286) | 17 | 7536 9136 | 7536 9256 | + | SEPT | 657302 | 82.5 |
| 657302_29427306_SEPT.1 _11_Methyl | AACCAAACGAAAAAAAAAAAAACGCTCCGATCGTATACCCAAAACTATCCCCCAACGACCACTCGAAC CCCAACCCCCCAAACCTAACCTTAACAAACGAACGAAACAACCAATACGAAA (SEQ ID NO:287) | 17 | 7536 9196 | 7536 9316 | + | SEPT | 657302 | 77.5 |
| 657302_29427306_SEPT.1 _12_Methyl | ACTCGAACCCCAACCCCCCAAACCTAACCTTAACAAACGAACGAAACAACCAATACGAAACAAAAAA CCGATACGAATACGAAAACCTAATCCGCCCGAAAAACGAAAACGAAACGAA (SEQ ID NO:288) | 17 | 7536 9256 | 7536 9375 | + | SEPT | 657302 | 78.99 |
| 657302_29427307_5PINT 2.1_1_Methyl | ATCATACCCTAATTTTCTAACGACCCAACCTCTAATCCCTAAACTTAACCTTCCCCATCACAACTTTCATC ACTTTATACTAAACTTCCGCTATCACTACTCTAAACCGTTCCTATTTAA (SEQ ID NO:289) | 19 | 3875 4618 | 3875 4738 | + | SPINT2 | 657302 | 52.5 |
| 657302_29427307_SPINT 2.1_2_Methyl | ACCTCTAATCCCTAAACTTAACCTTCCCCATCACAACTTTCATCACTTTATACTAAACTTCCGCTATCACT ACTCTAAACCGTTCCTATTTAATAACTCAAAAACCAATCTAACATAACC (SEQ ID NO:290) | 19 | 3875 4645 | 3875 4765 | + | SPINT2 | 657302 | 55 |
| 657302_29427308_SPINT 2.2_1_Methyl | TCCCCAACATTAAACCCTAAAACATAAACCCAATAAACTTTAAAATTCAAAAAAAAATTCACAAAAAA CTACGACGAAACGAACAAAAAACCACAAACTTCCAAAAAACGCGTATCTAC (SEQ ID NO:291) | 19 | 3875 4774 | 3875 4894 | + | SPINT2 | 657302 | 54.17 |
| 657302_29427308_SPINT 2.2_2_Methyl | ACAAAAAAACTACGACGAAACGAACAAAAAACCACAAACTTCCAAAAAACGCGTATCTACCGCCCCCT CCTCCCACCCTAAAACCAATCCTAAAACGAAAACCCTCCTCCGACGTCGTCA (SEQ ID NO:292) | 19 | 3875 4834 | 3875 4954 | + | SPINT2 | 657302 | 65 |
| 657302_29427308_SPINT 2.2_3_Methyl | CGCCCCCTCCTCCCACCCTAAAACCAATCCTAAAACGAAAACCCTCCTCCGACGTCGTCACCAAACCCA AAAAAAAAATAACAAATACTCAACGAACAAACGCCCCGCCCCGCCCCGCCA (SEQ ID NO:293) | 19 | 3875 4894 | 3875 5014 | + | SPINT2 | 657302 | 73.33 |
| 657302_29427309_APC.1 _1_Methyl | CTTTTCTAATAACTATTTTTAATTCAAATATAATTCGAATAATCTATCTAACAAATCATCACTCTAACAAC TCAATAACTTATAATATAAAATTATTCATTATAATTCATTTAATATTAT (SEQ ID NO:294) | 5 | 1.12E +08 | 1.12E +08 | + | APC | 657302 | 25 |
| 657302_29427309_APC.1 _2_Methyl | ACTCTAACAACTCAATAACTTATAATATAAAATTATTCATTATAATTCATTTAATATTATTATTTCTCTAT ACTACAAAAATCATAACAATCGAAATATAATTTATTACTCTCCCTCCCA (SEQ ID NO:295) | 5 | 1.12E +08 | 1.12E +08 | + | APC | 657302 | 30.83 |
| 657302_29427309_APC.1 _3_Methyl | TATTTCTCTATACTACAAAAATCATAACAATCGAAATATAATTTATTACTCTCCCTCCCACCTCCGACATC TTATACTAATCCTTCTACCCTACGAACCTCCCCCGACTCTTTACTATAC (SEQ ID NO:296) | 5 | 1.12E +08 | 1.12E +08 | + | APC | 657302 | 48.33 |
| 657302_29427309_APC.1 _4_Methyl | CCTCCGACATCTTATACTAATCCTTCTACCCTACGAACCTCCCCCGACTCTTTACTATACGTATCAACTAC CATCAACTTCCTTACTTACTAAAAACTAAAACCGCGAAAACATACCCCC (SEQ ID NO:297) | 5 | 1.12E +08 | 1.12E +08 | + | APC | 657302 | 60.83 |
| 657302_29427309_APC.1 _5_Methyl | ATATCAACTACCATCAACTTCCTTACTTACTAAAAACTAAAACCGCGAAAACATACCCCCGAAAAATAC GAAACTAAAACTAAACAAACTATACGATTAAACGAAACCCTATACCCCACT (SEQ ID NO:298) | 5 | 1.12E +08 | 1.12E +08 | + | APC | 657302 | 68.33 |
| 657302_29427309_APC. 1 _6_Methyl | AAAAAATACGAAACTAAAACTAAACAAACTATACGATTAAACGAAACCCTATACCCCACTACGAAATA CGAATCGAAAAACGAAAAAAAAAACAACTATATAATCCGCTAAATACGAACC (SEQ ID NO:299) | 5 | 1.12E +08 | 1.12E +08 | + | APC | 657302 | 69.17 |
| 657302_29427309_APC.1 _7_Methyl | ACGAAATACGAATCGAAAAACGAAAAAAAAAACAACTATATAATCCGCTAAATACGAACCAAAACGC TCCCCATTCCCGTCGAAAACCCGCCGATTAACTAAATATAAACGCACGTAACC (SEQ ID NO:300) | 5 | 1.12E +08 | 1.12E +08 | + | APC | 657302 | 69.17 |
| 657302_29427309_APC.1 _8_Methyl | AAAACGCTCCCCATTCCCGTCGAAAACCCGCCGATTAACTAAATATAAACGCACGTAACCGACATATAA CTATATTAATACAACCCGCCAAAATATCACTAAAAACAAAATAAAAATACT (SEQ ID NO:301) | 5 | 1.12E +08 | 1.12E +08 | + | APC | 657302 | 65.83 |
| 657302_29427309_APC.1 _9_Methyl | AACATATAACTATATTAATACAACCCGCCAAAATATCACTAAAAACAAAATAAAAATACTACCGAACTC GAAAATAAAATAAATACTAAAACCACCATAACCAAACTTACTACGAAAAAA (SEQ ID NO:302) | 5 | 1.12E +08 | 1.12E +08 | + | APC | 657302 | 63.33 |
| 657302_29427309_APC.1 _10_Methyl | ACCGAACTCGAAAATAAAATAAATACTAAAACCACCATAACCAAACTTACTACGAAAAAAAAAAAAA AATAATTTTCCCTCGCACTATCTTAAACCGATAACCTTTCCTTAACACAAAA (SEQ ID NO:303) | 5 | 1.12E +08 | 1.12E +08 | + | APC | 657302 | 63.33 |
| 657302_29427310_HOXA 1_1_Methyl | CGACTAAAAAATTATAATCCTATAATCCGAAAAATAAACTCGAACTAAACAAATCCCCGAATCGCCACT ACTAAATATAAAATATTCCAAAAAAAAATTCATTCTTACATTATCCATCTA (SEQ ID NO:304) | 7 | 2713 5414 | 2713 5534 | + | HOXA1 | 657302 | 54.17 |
| 657302_29427310_HOXA 1_2_Methyl | ATCGCCACTACTAAATATAAAATATTCCAAAAAAAAATTCATTCTTACATTATCCATCTATCACTAAATA ACCTAATCCTACGAAACCCGACGTAACTATACCAACTTTCTCACTTCCTC (SEQ ID NO:305) | 7 | 2713 5474 | 2713 5594 | + | HOXA1 | 657302 | 53.33 |
| 657302_29427310_HOXA 1_3_Methyl | TCACTAAATAACCTAATCCTACGAAACCCGACGTAACTATACCAACTTTCTCACTTCCTCCATAAAACCG AAAAAAAAAAATAATATAAATATACAATACGCAAAAAAAAAAACGAAAAA (SEQ ID NO:306) | 7 | 2713 5534 | 2713 5654 | + | HOXA1 | 657302 | 57.5 |
| 657302_29427310_HOXA 1_4_Methyl | CATAAAACCGAAAAAAAAAAATAATATAAATATACAATACGCAAAAAAAAAACGAAAAAACGAAAA ACGCTAAAAAAAAAACACGTAACGATATCAACCAATAACTAAACCTCCTACAA (SEQ ID NO:307) | 7 | 2713 5594 | 2713 5714 | + | HOXA1 | 657302 | 61.67 |
| 657302_29427310_HOXA 1_5_Methyl | ACGAAAAACGCTAAAAAAAAAACACGTAACGATATCAACCAATAACTAAACCTCCTACAAAAATTTAC CGACTTCCGCAATAATAAATCACCGTTTTAATAACATTTAAATCCCCGACGC (SEQ ID NO:308) | 7 | 2713 5654 | 2713 5774 | + | HOXA1 | 657302 | 60 |
| 657302_29427310_HOXA 1_6_Methyl | AAATTTACCGACTTCCGCAATAATAAATCACCGTTTTAATAACATTTAAATCCCCGACGCTCCGCCGTCT AAATAACGCGCAATCGCCCCCCCAAACAACCTAAACGACGACAACTACTA (SEQ ID NO:309) | 7 | 2713 5714 | 2713 5834 | + | HOXA1 | 657302 | 64.17 |
| 657302_29427310_HOXA 1_7_Methyl | TCCGCCGTCTAAATAACGCGCAATCGCCCCCCAAACAACCTAAACGACGACAACTACTACGACGACT ACAAAAACCGATTTAAAATACTAAAACGAAAAAAAACAAAAACTACGTTCTA (SEQ ID NO:310) | 7 | 2713 5774 | 2713 5894 | + | HOXA1 | 657302 | 65.83 |
| 657302_29427310_HOXA 1_8_Methyl | CGACGACTACAAAAACCGATTTAAAATACTAAAACGAAAAAAAACAAAAACTACGTTCTACGCGCGCC CGACTCCGCTACCCGCCCCGCCAAACCTCCGAAAAATAAAAACTAAAAAACG (SEQ ID NO:311) | 7 | 2713 5834 | 2713 5954 | + | HOXA1 | 657302 | 70.83 |
| 657302_29427310_HOXA 1_9_Methyl | CGCGCGCCCGACTCCGCTACCCGCCCCGCCAAACCTCCGAAAAATAAAAACTAAAAAACGTCCCCCGC TCCCGCCCCCTCCCCACCGTTCAATAAAAAATAAACTAACGAAAAATAAAAA (SEQ ID NO:312) | 7 | 2713 5894 | 2713 6014 | + | HOXA1 | 657302 | 73.33 |
| 657302_29427310_HOXA 1_10_Methyl | TCCCCCGCTCCCGCCCCCTCCCCACCGTTCAATAAAAAATAAACTAACGAAAAATAAAAAAAAAAAA AACTCCCGACTCTCTCGAAACGAAAATCAATAAACCAAAACTCGCCGAATAA (SEQ ID NO:313) | 7 | 2713 5954 | 2713 6074 | + | HOXA1 | 657302 | 67.5 |
| 657302_29427310_HOXA 1_11_Methyl | AAAAAAAAAACTCCCGACTCTCTCGAAACGAAAATCAATAAACCAAAACTCGCCGAATAACCGCAAAT ACGCCGACCCAACCCGCAACGCGCCCAACCGAAAAACGAAAAATCCGACTAA (SEQ ID NO:314) | 7 | 2713 6014 | 2713 6134 | + | HOXA1 | 657302 | 75 |
| 657302_29427310_HOXA 1_12_Methyl | CCGCAAATACGCCGACCCAACCCGCAACGCGCCCAACCGAAAAACGAAAAATCCGACTAACACCGCG CCCCGAATTCCCAAACCACCTCCTCTATTCTAAAACTAAACTAAAAAACCGTA (SEQ ID NO:315) | 7 | 2713 6074 | 2713 6194 | + | HOXA1 | 657302 | 74.17 |
| 657302_29427310_HOXA 1_13_Methyl | CACCGCGCCCCGAATTCCCAAACCACCTCCTCTATTCTAAAACTAAACTAAAAAACCGTAAAACTATAA AAAACGCATAAAACCGTAATAAAAAACGAAACTAAACCACCGACTCTTCAA (SEQ ID NO:316) | 7 | 2713 6134 | 2713 6254 | + | HOXA1 | 657302 | 69.17 |
| 657302_29427310_HOXA 1_14_Methyl | AAACTATAAAAAACGCATAAAACCGTAATAAAAAACGAAACTAAACCACCGACTCTTCAAACTCGAAA TAAAAAAAAAAAAACGCAAAAAACTAACTAAAAAAAAACTCGAATAAACGTAA (SEQ ID NO:317) | 7 | 2713 6194 | 2713 6314 | + | HOXA1 | 657302 | 67.5 |
| 657302_29427310_HOXA 1_15_Methyl | ACTCGAAATAAAAAAAAAAAAACGCAAAAAACTAACTAAAAAAAACTCGAATAAACGTAAAAAAAC GAAAACAAAAAAAAAAAACTTCCCTTCTTCCAAAAAAATCTTCGAAACCCTCTC (SEQ ID NO:318) | 7 | 2713 6254 | 2713 6374 | + | HOXA1 | 657302 | 60 |
| 657302_29427310_HOXA 1_16_Methyl | AAAAAAACGAAAACAAAAAAAAAAACTTCCCTTCTTCCAAAAAAATCTTCGAAACCCTCTCCCCACAACC CCTCTCGTCATTAACATAACAATAAAAAATTTCTATAATTCGACTTAAAAA (SEQ ID NO:319) | 7 | 2713 6314 | 2713 6434 | + | HOXA1 | 657302 | 51.67 |
| 657302_29427310_HOXA 1_17_Methyl | CCCACAACCCCTCTCGTCATTAACATAACAATAAAAAATTTCTATAATTCGACTTAAAAAAACGAATAA ACCCTAAAAACTCAAAACTCGCCGAAAAAAACCGAAAACGACCGAACTCTT (SEQ ID NO:320) | 7 | 2713 6374 | 2713 6494 | + | HOXA1 | 657302 | 60.83 |
| 657302_29427310_HOXA 1_18_Methyl | AACGAATAAACCCTAAAAACTCAAAACTCGCCGAAAAAAACCGAAAACGACCGAACTCTTCTTCCCCA CCTTCCCTCTCTCGTCGCTCTCCGCCCCTTTCTCTTTCCCACTCAATTTTAC (SEQ ID NO:321) | 7 | 2713 6434 | 2713 6554 | + | HOXA1 | 657302 | 64.17 |
| 657302_29427310_HOXA 1_19_Methyl | CTTCCCCACCTTCCCTCTCTCGTCGCTCTCCGCCCCTTTCTCTTTCCCACTCAATTTTACACCGAAAACCCT CCGAAATACGAAACTACTCGACCGCCGAATTTTTAAAAATAAAAAACG (SEQ ID NO:322) | 7 | 2713 6494 | 2713 6614 | + | HOXA1 | 657302 | 62.5 |
| 657302_29427310_HOXA 1_20_Methyl | ACCGAAAACCCTCCGAAATACGAAACTACTCGACCGCCGAATTTTTAAAAATAAAAAACGAAAAAAA AAATAACGCTAACGAACGTAACCAACGCGAAAACCGAACGATACGCTACAAA (SEQ ID NO:323) | 7 | 2713 6554 | 2713 6674 | + | HOXA1 | 657302 | 72.5 |
| 657302_29427310_HOXA 1_21_Methyl | AAAAAAAAAAATAACGCTAACGAACGTAACCAACGCGAAAACCGAACGATACGCTACAAACCATCTA CCGACGCCCTAAAACCCAAAAACCTCCGCGCTCCCGCGTAAACCTCACAAAAC (SEQ ID NO:324) | 7 | 2713 6614 | 2713 6734 | + | HOXA1 | 657302 | 77.5 |
| 657301_29427287_PRDM 2_1_Methyl | ATATCGCCGCCGCCGTCGCCGCCGCGCTCCTCGAAAAAAAAACCAACGTCCCGACGCGAACCCAAAA ACCGCCCACCCGCGCCACTCCTTACCCGCGCCCGCCGCGCCAACGCCTCAAA (SEQ ID NO:325) | 1 | 1402 6768 | 1402 6888 | - | PRDM 2 | 657301 | 81.67 |
| 657301_29427287_PRDM 2_2_Methyl | ACCCAAAAACCGCCCACCCGCGCCACTCCTTACCCGCGCCCGCCGCGCCAACGCCTCAAAACACCGAA AACCGCCGCCGCCGCCGCTATTTCGCCGACCCCGACGCCCGCGACCGCGCCG (SEQ ID NO:326) | 1 | 1402 6708 | 1402 6828 | - | PRDM 2 | 657301 | 85 |
| 657301_29427287_PRDM 2_3_Methyl | ACACCGAAAACCGCCGCCGCCGCCGCTATTTCGCCGACCCCGACGCCCGCGACCGCGCCGCCGCCATC TTAACTCCAATCGAAAATAACGTCGAACGACGAAACAATAACCTACGAAACT (SEQ ID NO:327) | 1 | 1402 6648 | 1402 6768 | - | PRDM 2 | 657301 | 80 |
| 657301_29427287_PRDM 2_4_Methyl | CCGCCATCTTAACTCCAATCGAAAATAACGTCGAACGACGAAACAATAACCTACGAAACTAAAAAACT CCGAAACCCCCGATCTCCCCACGATCCCAAAACCCGACCCTTAACCCTACGC (SEQ ID NO:328) | 1 | 1402 6588 | 1402 6708 | - | PRDM 2 | 657301 | 74.17 |
| 657301_29427287_PRDM 2_5_Methyl | AAAAAACTCCGAAACCCCCGATCTCCCCACGATCCCAAAACCCGACCCTTAACCCTACGCCGTCGCCCA ATAACCACCACCCAACCGCCCCTCGTAAATCACCGCGAATCCCATAACGAC (SEQ ID NO:329) | 1 | 1402 6528 | 1402 6648 | - | PRDM 2 | 657301 | 75.83 |
| 657301_29427287_PRDM 2_6_Methyl | CGTCGCCCAATAACCACCACCCAACCGCCCCTCGTAAATCACCGCGAATCCCATAACGACGCCTCGAA AAAAAACCCCGACGACTAACGTCGCGACAAATCCGACCGCACCCTAAAACTA (SEQ ID NO:330) | 1 | 1402 6468 | 1402 6588 | - | PRDM 2 | 657301 | 73.33 |
| 65701_29427287_PRDM 2_7_Methyl | ACCTCGAAAAAAAACCCCGACGACTAACGTCGCGACAAATCCGACCGCACCCTAAAACTAAAATCCTA CGTAATTCAAAAATTCTCAAAAAATCAAAAAAACTAAAAAAAATATTAAAAA (SEQ ID NO:331) | 1 | 1402 6408 | 1402 6528 | - | PRDM 2 | 657301 | 60 |
| 657301_29427287_PRDM 2_8_Methyl | AAATCCTACGTAATTCAAAAATTCTCAAAAAATCAAAAAAACTAAAAAAAATATTAAAAAAACATATCT ATCGTTAAAAATCATTAAATATCTAACAAATACATAAACGTAAACCTTATA (SEQ ID NO:332) | 1 | 1402 6348 | 1402 6468 | - | PRDM 2 | 657301 | 41.67 |
| 65701_29427287_PRDM 2_9_Methyl | AACATATCTATCGTTAAAAATCATTAAATATCTAACAAATACATAAACGTAAACCTTATACTTCTACGTT TAAATCTATACCAAAAAAATCCAACTCCAAACAAACAAACGCAACTACTA (SEQ ID NO:333) | 1 | 1402 6288 | 1402 6408 | - | PRDM 2 | 657301 | 42.5 |
| 657301_29427288_RUNX 3_1_Methyl | AACCAAAACCGCGCAAAACTAAAAACAACAAAAACCGCCGACCGAACGTAAACGACGCGCAAAATCC CGTATAAAATAAAAACTCTTAAATCAAAATAATATACGAAACGAAAAAAATAA (SEQ ID NO:334) | 1 | 2525 7244 | 2525 7364 | - | RUNX3 | 657301 | 66.67 |
| 657301_29427288_RUNX 3_2_Methyl | AAAATCCCGTATAAAATAAAAACTCTTAAATCAAAATAATATACGAAACGAAAAAAATAAATAACCTCT TTAAAACGACTCCCAATACGACGTCACCGACCCTAAAACCCCGCGACCCCC (SEQ ID NO:335) | 1 | 2525 7184 | 2525 7304 | - | RUNX3 | 657301 | 65.83 |
| 657301_29427288_RUNX 3_3_Methyl | ATAACCTCTTTAAAACGACTCCCAATACGACGTCACCGACCCTAAAACCCCGCGACCCCCAACCCGAAA TTACAAAAATCACAAACCCGAAACAACAAAAACTAAAAAAACCCGACCGCG (SEQ ID NO:336) | 1 | 2525 7124 | 2525 7244 | - | RUNX3 | 657301 | 72.5 |
| 657301_29427288_RUNX 3_4_Methyl | AACCCGAAATTACAAAAATCACAAACCCGAAACAACAAAAACTAAAAAAACCCGACCGCGACCAACG AAAAAAAAAAACGAAAAAATTACGCCCCAACGTCAAAAAACTACGACCCGAAA (SEQ ID NO:337) | 1 | 2525 7064 | 2525 7184 | - | RUNX3 | 657301 | 70.83 |
| 657301_29427288 _RUNX 3_5_Methyl | ACCAACGAAAAAAAAAAACGAAAAAATTACGCCCCAACGTCAAAAAACTACGACCCGAAAAAAAACG ACAAAAACGCCTTCCGTAAAACCCGAACGTTCTAAACAAATTTCTAACATTTA (SEQ ID NO:338) | 1 | 2525 7004 | 2525 7124 | - | RUNX3 | 657301 | 65 |
| 657301_29427288_RUNX 3_6_Methyl | AAAAACGACAAAAACGCCTTCCGTAAAACCCGAACGTTCTAAACAAATTTCTAACATTTACCCCGAACT CCCAAAACTCTCGAAAACCCTAAACTATAACACTAAAACCTCCTCCGCGAA (SEQ ID NO:339) | 1 | 2525 6944 | 2525 7064 | - | RUNX3 | 657301 | 69.17 |
| 657301_29427288_RUNX 3_7_Methyl | CCCCGAACTCCCAAAACTCTCGAAAACCCTAAACTATAACACTAAAACCTCCTCCGCGAAATAACGCCT TCCGCCCCTCCCCGTTAAACGACCTCCGACAAACCCCGTTCCTCCCCGCGA (SEQ ID NO:340) | 1 | 2525 6884 | 2525 7004 | - | RUNX3 | 657301 | 80 |
| 657301_29427288_RUNX 3_8_Methyl | ATAACGCCTTCCGCCCCTCCCCGTTAAACGACCTCCGACAAACCCCGTTCCTCCCCGCGAACGCCACCG AAATACCCGCGATAAAAACTCCGCCGATTAACTATACGACGCGTCGCTCCG (SEQ ID NO:341) | 1 | 2525 6824 | 2525 6944 | - | RUNX3 | 657301 | 77.5 |
| 657301_29427288_RUNX 3_9_Methyl | ACGCCACCGAAATACCCGCGATAAAAACTCCGCCGATTAACTATACGACGCGTCGCTCCGCCAACCCC GCCCCGCGAACCCCGAAAATACTAACCCCGCGCGAACGACCGCGACCCCGCC (SEQ ID NO:342) | 1 | 2525 6764 | 2525 6884 | - | RUNX3 | 657301 | 82.5 |
| 657301_29427288_RUNX 3_10_Methyl | CCAACCCCGCCCCGCGAACCCCGAAAATACTAACCCCGCGCGAACGACCGCGACCCCGCCACTTAATT CTAAAAAATTTATTCTAAAACTACGACCGCGAAATCGAAACGACCGCGAACG (SEQ ID NO:343) | 1 | 2525 6704 | 2525 6824 | - | RUNX3 | 657301 | 80 |
| 657301_29427288_RUNX 3_11_Methyl | ACTTAATTCTAAAAAATTTATTCTAAAACTACGACCGCGAAATCGAAACGACCGCGAACGAACTTCGA AACGAAAAACGACGACAACGACACAACCCCGCGCGAACCCCGCCGCGACCCA (SEQ ID NO:344) | 1 | 2525 6644 | 2525 6764 | - | RUNX3 | 657301 | 78.33 |
| 657301_29427289_PFKP.1 _1_Methyl | CCCCCGCCCGACCCCAACGCCAATAAACGATAACGAACGAAACCCGAACGTATAAAAAAACTACGA AAAAAAAAACGCGAACGCGACTAAAAACAAAAACCGAAACTAAAACGAATATA (SEQ ID NO:345) | 10 | 3109 570 | 3109 690 | - | PFKP | 657301 | 84.17 |
| 657301_29427289_PFKP.1 _2_Methyl | ACTACGAAAAAAAAAACGCGAACGCGACTAAAAACAAAAACCGAAACTAAAACGAATATAAACAAAA ACATCTACGCGAAAATCGTCGTAAATAAAAACCGCGCTAACGATACGAAAAAC (SEQ ID NO:346) | 10 | 3109 510 | 3109 630 | - | PFKP | 657301 | 80.83 |
| 657301_29427289_PFKP.1 _3_Methyl | AACAAAAACATCTACGCGAAAATCGTCGTAAATAAAAACCGCGCTAACGATACGAAAAACGACAAAA ACAAAAATAACGCCACAAATAAACGAATCCCTACTAATAAAACCGCATCGCAA (SEQ ID NO:347) | 10 | 3109 450 | 3109 570 | - | PFKP | 657301 | 74.17 |
| 657301_29427289_PFKP.1 _4_Methyl | AACAAAAACAAAAATAACGCCACAAATAAACGAATCCCTACTAATAAAACCGCATCGCAAATACGCGA ACCTCGCGAATAAACTAAAAAACTCTAATTAAAAACTCTAAAAATAACGAAA (SEQ ID NO:348) | 10 | 3109 390 | 3109 510 | - | PFKP | 657301 | 70.83 |
| 657301_29427289_PFKP.1 _5_Methyl | ATACGCGAACCTCGCGAATAAACTAAAAAACTCTAATTAAAAACTCTAAAAATAACGAAAACGCCACA AATAAAACAAAAACAACGTCCGAAAAAAAAAAAACCGCAAAAAACCGAAATC (SEQ ID NO:349) | 10 | 3109 330 | 3109 450 | - | PFKP | 657301 | 74.17 |
| 657301_29427289_PFKP.1 _6_Methyl | ACGCCACAAATAAAACAAAAACAACGTCCGAAAAAAAAAAACCGCAAAAAACCGAAATCACATCGT TTACGAAACGCGCCAAAACGAAACGAAATAAAACGCCGAAAACATCTCCCGAA (SEQ ID NO:350) | 10 | 3109 270 | 3109 390 | - | PFKP | 657301 | 76.67 |
| 657301_29427289_PFKP.1 _7_Methyl | ACATCGTTTACGAAACGCGCCAAAACGAAACGAAATAAAACGCCGAAAACATCTCCCGAAAAAACGA AAAAAACCGTAAATAAACGCGAACGCCGAAACGAATAAAAACCCCGCAATTAC (SEQ ID NO:351) | 10 | 3109 210 | 3109 330 | - | PFKP | 657301 | 75 |
| 657301_29427289_PFKP.1 _8_Methyl | AAAACGAAAAAAACCGTAAATAAACGCGAACGCCGAAACGAATAAAAACCCCGCAATTACGAAAAAC GCCGATAACGAAAAAAAAATAAAATAAAAACGCGAAAACCCCACCTAAACGCGA (SEQ ID NO:352) | 10 | 3109 150 | 3109 270 | - | PFKP | 657301 | 77.5 |
| 657301_29427289_PFKP.1 _9_Methyl | AAAAAACGCCGATAACGAAAAAAAATAAAATAAAAACGCGAAAACCCCACCTAAACGCGAAAACTCG CGACAAACCCGACCGCTCGAACCGTTATAAAAACCGAACCCGACCGCGCGCAC (SEQ ID NO:353) | 10 | 3109 090 | 3109 210 | - | PFKP | 657301 | 81.67 |
| 657301_29427289_PFKP.1 _10_Methyl | AAACTCGCGACAAACCCGACCGCTCGAACCGTTATAAAAACCGAACCCGACCGCGCGCACAACTTCTA ATAACCCGCAAAATTCCTCCTAAAACGACGCTAAAAACCTCGAAACTCGACT (SEQ ID NO:354) | 10 | 3109 030 | 3109 150 | - | PFKP | 657301 | 77.5 |
| 657301_29427290_GSTP1 .1_1_Methyl | CGCGACCTCCGAACCTTATAAAAATAATCCCGCCCCGCTCCGCCCCAATACTAAATCACGACGCCGACC GCTCTTCTAAAAAATCCCGCGAACTCCCGCCGACCCCAACCCCGACGACCG (SEQ ID NO:355) | 11 | 6735 1154 | 6735 1274 | - | GSTP1 | 657301 | 78.33 |
| 657301_29427290_GSTP1 .1_2_Methyl | ACGCCGACCGCTCTTCTAAAAAATCCCGCGAACTCCCGCCGACCCCAACCCCGACGACCGCTACACCCC GAACGTCGACCGCAAAAAAACGCCCTAAAATCCCCGAAATCGCCGCGCAAC (SEQ ID NO:356) | 11 | 6735 1094 | 6735 1214 | - | GSTP1 | 657301 | 83.33 |
| 657301_29427290_GSTP1 .1_3_Methyl | CTACACCCCGAACGTCGACCGCAAAAAACGCCCTAAAATCCCCGAAATCGCCGCGCAACTAACCGAA AAAACCTTTCCCTCTTTCCCAAATCCCCAACGAAACCTAAAAAATAAACAAA (SEQ ID NO:357) | 11 | 6735 1034 | 6735 1154 | - | GSTP1 | 657301 | 72.5 |
| 657301_29427290_GSTP1 .1_4_Methyl | TAACCGAAAAAACCTTTCCCTCTTTCCCAAATCCCCAACGAAACCTAAAAAATAAACAAACAACAAAAA AAAAACCGCAACGAAATATACGCAACGAACTAACGCGCCGAAACATCGCGA (SEQ ID NO:358) | 11 | 6735 0974 | 6735 1094 | - | GSTP1 | 657301 | 66.67 |
| 657301_29427290_GSTP1 .1_5_Methyl | CAACAAAAAAAAAACCGCAACGAAATATACGCAACGAACTAACGCGCCGAAACATCGCGAAAAAAA TTCCCTAAAACCGCTACGATCCCGAAACTTACACACCCGCTTCACAAAACAAA (SEQ ID NO:359) | 11 | 6735 0914 | 6735 1034 | - | GSTP1 | 657301 | 66.67 |
| 657301_29427290_GSTP1 .1_6_Methyl | AAAAAAATTCCCTAAAACCGCTACGATCCCGAAACTTACACACCCGCTTCACAAAACAAAAAAAAAA ATAAAAACCGCTTAAAAAAAAAAAAATTACTTTATTTTATTTTATTTTATT (SEQ ID NO:360) | 11 | 6735 0855 | 6735 0974 | - | GSTP1 | 657301 | 50.42 |
| 657301_29427291_WIF1. 1_1_Methyl | ACCTACCCCCTCCTCCTACTCTCGCAAACTCCTTAACACCCAAACCGAAAAACGACGCGCCCAACCGTC TAAACGAAAACAACCCTAACTAAAAAAACTACAACGCAACAAAATATCTAA (SEQ ID NO:361) | 12 | 6551 5035 | 6551 5155 | - | WIF1 | 657301 | 69.17 |
| 657301_29427291_WIF1. 1_2_Methyl | CCAACCGTCTAAACGAAAACAACCCTAACTAAAAAAACTACAACGCAACAAAATATCTAACGACGCCA AATTACGTAAATACGACACGAAAAATTTTCCCGACAACGAAAAAATCCTAAA (SEQ ID NO:362) | 12 | 6551 4975 | 6551 5095 | - | WIF1 | 657301 | 65 |
| 657301_29427292_WIF1. 2_1_Methyl | TCTACTCTCCCCATTTCCCTCCCCCGAAACCTCCCTTAACCCGAAAAAATAACGAATAATATCCCAAAAA TCTCTAAATACCCTTCTCCGAATCCGCCAACCCTACACGCCCACTTCGCG (SEQ ID NO:363) | 12 | 6551 5458 | 6551 5578 | - | WIF1 | 657301 | 67.5 |
| 657301_29427292_WIF1. 2_2_Methyl | ATCCCAAAAATCTCTAAATACCCTTCTCCGAATCCGCCAACCCTACACGCCCACTTCGCGAACGCTCCAC TAAACGCACCGCACTATAAATACAACCTCGAAAATCCCTCGCGACCCCGC (SEQ ID NO:364) | 12 | 6551 5398 | 6551 5518 | - | WIF1 | 657301 | 73.33 |
| 657301_29427292_WIF1. 2_3_Methyl | AACGCTCCACTAAACGCACCGCACTATAAATACAACCTCGAAAATCCCTCGCGACCCCGCCCCCGAAA AAACCCCACAACGCCCCCAAATAACGACCGCCCAAACCTCGCGAACCCCACT (SEQ ID NO:365) | 12 | 6551 5338 | 6551 5458 | - | WIF1 | 657301 | 80.83 |
| 657301_29427292_WIF1. 2_4_Methyl | CCCCGAAAAAACCCCACAACGCCCCCAAATAACGACCGCCCAAACCTCGCGAACCCCACTCCTCGCTC GCACCTCGCTCGCGCCAACCCTTCCCGCTCTTCTATTCTCGCTCTATTTACC (SEQ ID NO:366) | 12 | 6551 5278 | 6551 5398 | - | WIF1 | 657301 | 75 |
| 657301_29427292_WIF1. 2_5_Methyl | CCTCGCTCGCACCTCGCTCGCGCCAACCCTTCCCGCTCTTCTATTCTCGCTCTATTTACCCCGCTAACTAC TAACCTCGCCAACTTTACCAATCTTACGTCTCTACCGCCCCCACTCCCG (SEQ ID NO:367) | 12 | 6551 5218 | 6551 5338 | - | WIF1 | 657301 | 66.67 |
| 657301_29427292_WIF1. 2_6_Methyl | CCGCTAACTACTAACCTCGCCAACTTTACCAATCTTACGTCTCTACCGCCCCCACTCCCGCCCGCGCCCC ATCTTCTTACGCGACTCGCGCCCGCTAATCCCCCCCTCCTCCTCCCGCG (SEQ ID NO:368) | 12 | 6551 5159 | 6551 5278 | - | WIF1 | 657301 | 73.11 |
| 657301_29427293_AK055 957.1_1_Methyl | ACGAAAATCAACGCAAAAACGATACTACAACCTCTAAACTTCCTAACGACCGTATCCAAAACCGAACTC CTCCTCCGACGACAACCGCCGAACTCACTTCAATACGCTCAACTTCTCGCG (SEQ ID NO:369) | 12 | 1.33E +08 | 1.33E +08 | - | AK055 957 | 657301 | 68.33 |
| 657301_29427293_AK055 957.1_2_Methyl | ACCGAACTCCTCCTCCGACGACAACCGCCGAACTCACTTCAATACGCTCAACTTCTCGCGAAAACAAAC GTCTAAACGAAAACGACCCGAAACGAAAATATAACGAAACTAAAAAACGCT (SEQ ID NO:370) | 12 | 1.33E +08 | 1.33E +08 | - | AK055 957 | 657301 | 75 |
| 657301_29427293_AK055 957.1_3_Methyl | AAAACAAACGTCTAAACGAAAACGACCCGAAACGAAAATATAACGAAACTAAAAAACGCTAAAAACT AATAAACTTAAAAAAACAAACGACGCTCTAAAATTTAACTCCCAAATAATACG (SEQ ID NO:371) | 12 | 1.33E +08 | 1.33E +08 | - | AK055 957 | 657301 | 63.33 |
| 657301_29427293_AK055 957.1_4_Methyl | AAAAACTAATAAACTTAAAAAAACAAACGACGCTCTAAAATTTAACTCCCAAATAATACGTTCCGACAC TTCGCGACGACTCAATCAACGCTACTAAATTCCACCCCTCCTATACGTTAC (SEQ ID NO:372) TTCCGACACTTCGCGACGACTCAATCAACGCTACTAAATTCCACCCCTCCTATACGTTACTCAAAAACAA ATTTCTTAATAACAAACCCCTCACTATTCCCATTAACCAAAACGCCCAAA (SEQ ID NO:373) | 12 | 1.33E +08 | 1.33E +08 | - | AK055 957 | 657301 | 59.17 |
| 657301_29427293_AK055 957.1_5_Methyl | TTCCGACACTTCGCGACGACTCAATCAACGCTACTAAATTCCACCCCTCCTATACGTTACTCAAAAACAA ATTTCTTAATAACAAACCCCTCACTATTCCCATTAACCAAAACGCCCAAA (SEQ ID NO:373) | 12 | 1.33E +08 | 1.33E +08 | - | AK055 957 | 657301 | 61.67 |
| 657301_29427293_AK055 957.1_6_Methyl | TCAAAAACAAATTTCTTAATAACAAACCCCTCACTATTCCCATTAACCAAAACGCCCAAAACCCACGCA ACCGTTAACTAAAATTATTATCTATTTCAAACACGTTAACGAATTCCCCGC (SEQ ID NO:374) | 12 | 1.33E +08 | 1.33E +08 | - | AK055 957 | 657301 | 55.83 |
| 657301_29427293_AK055 957.1_7_Methyl | ACCCACGCAACCGTTAACTAAAATTATTATCTATTTCAAACACGTTAACGAATTCCCCGCCTCTACGTTA CCGAAAAACAACATATTACCTAACAACGTACGACTCCCATTACCTTTAAA (SEQ ID NO:375) | 12 | 1.33E +08 | 1.33E +08 | - | AK055 957 | 657301 | 58.33 |
| 657301_29427293_AK055 957.1_8_Methyl | CTCTACGTTACCGAAAAACAACATATTACCTAACAACGTACGACTCCCATTACCTTTAAACGCGACTCTC CGCCCCGACCCGCCCCTCTAAAACCCATCCGAATCTACGCCTCAACTAAA (SEQ ID NO:376) | 12 | 1.33E +08 | 1.33E +08 | - | AK055 957 | 657301 | 68.33 |
| 657301_29427293_AK055 957.1_9_Methyl | CGCGACTCTCCGCCCCGACCCGCCCCTCTAAAACCCATCCGAATCTACGCCTCAACTAAACAAAACGAA CTACGACGCGCAATCTTAAACGTACGCTTCGAAAAAAAAACCCTCGCGTAA (SEQ ID NO:377) | 12 | 1.33E +08 | 1.33E +08 | - | AK055 957 | 657301 | 75.83 |
| 657301_29427293_AK055 957.1_10_Methyl | CAAAACGAACTACGACGCGCAATCTTAAACGTACGCTTCGAAAAAAAACCCTCGCGTAAAAAAACG CGTCTACGAAAAATACGCCGACGCAAACGAAACCCGAAACCGCGTAATCTCT (SEQ ID NO:378) | 12 | 1.33E +08 | 1.33E +08 | - | AK055 957 | 657301 | 75.83 |
| 657301_29427293_AK055 957.1_11_Methyl | AAAAAACGCGTCTACGAAAAATACGCCGACGCAAACGAAACCCGAAACCGCGTAATCTCTACGCAAT ACGTCGTATAAAACGACCGACCCCGACCCGAATTTCCCTACTCGTTTTCGTCC (SEQ ID NO:379) | 12 | 1.33E +08 | 1.33E +08 | - | AK055 957 | 657301 | 74.17 |
| 657301_29427293_AK055 957.1_12_Methyl | ACGCAATACGTCGTATAAAACGACCGACCCCGACCCGAATTTCCCTACTCGTTTTCGTCCGAACGAACG TCTTAATTCCCGTTCCAAACCAACCCCATCCTAAATCGCTACTTCATCCAA (SEQ ID NO:380) | 12 | 1.33E +08 | 1.33E +08 | - | AK055 957 | 657301 | 70 |
| 657301_29427293_AK055 957.1_13_Methyl | AAACGAACGTCTTAATTCCCGTTCCAAACCAACCCCATCCTAAATCGCTACTTCATCCAACGCTTAAAAC ATTTATATCGTTAACGTTATTTTCCTCTTAACTACAAACTTTAAATATAT (SEQ ID NO:381) | 12 | 1.33E +08 | 1.33E +08 | - | AK055 957 | 657301 | 54.17 |
| 657301_29427293_AK055 957.1_14_Methyl | CGCTTAAAACATTTATATCGTTAACGTTATTTTCCTCTTAACTACAAACTTTAAATATATTCATCTACTCG TAACGACGAATTTAACAAACTTTCATTCCCAAAAAACGTATCACACGAT (SEQ ID NO:382) | 12 | 1.33E +08 | 1.33E +08 | - | AK055 957 | 657301 | 45 |
| 657301_29427293_AK055 957.1_15_Methyl | TCATCTACTCGTAACGACGAATTTAACAAACTTTCATTCCCAAAAAACGTATCACACGATCTATATATTT TTCATACAAAAATTAACGAACGTCGTTACTTCTAAATTTCCACAAAAACT (SEQ ID NO:383) | 12 | 1.33E +08 | 1.33E +08 | - | AK055 957 | 657301 | 43.33 |
| 657301_29427293_AK055 957.1_16_Methyl | CTATATATTTTTCATACAAAAATTAACGAACGTCGTTACTTCTAAATTTCCACAAAAACTAATTCATCCC GCGACTTTACCTAAAAAAAAACGTCTAAAACCGAACGCGACGACTCCCG (SEQ ID NO:384) | 12 | 1.33E +08 | 1.33E +08 | - | AK055 957 | 657301 | 52.1 |
| 657301_29427294_SEPT.1 _1_Methyl | CCGCCCCGCCCCCGCCTCCCGAACGAATCAAATTCCCGCACCCGCACCGACCTCCCTATCTCGCACTAA CTACTCCGCCCGCCTATCAAAACCAAACCTAAAAAACTAAAACCCGAATAA (SEQ ID NO:385) | 17 | 7536 9254 | 7536 9374 | - | SEPT | 657301 | 79.17 |
| 657301_29427294_SEPT.1 _2_Methyl | TCGCACTAACTACTCCGCCCGCCTATCAAAACCAAACCTAAAAAACTAAAACCCGAATAACCGCTAAAA AACAATCCTAAACACACGACCGAAACGCCCCCCTCCTCCCCGCCTAACCCG (SEQ ID NO:386) | 17 | 7536 9194 | 7536 9314 | - | SEPT | 657301 | 78.33 |
| 657301_29427294_SEPT.1 _3_Methyl | CCGCTAAAAAACAATCCTAAACACACGACCGAAACGCCCCCCTCCTCCCCGCCTAACCCGCGCCCAAAA AACGACGAAAAACGCGACCTCAACCCCTCCCCCCGAACGCCCCGCTACGAC (SEQ ID NO:387) | 17 | 7536 9134 | 7536 9254 | - | SEPT | 657301 | 82.5 |
| 657301_29427294_SEPT.1 _4_Methyl | CGCCCAAAAAACGACGAAAAACGCGACCTCAACCCCTCCCCCGAACGCCCCGCTACGACCAAATATA AACGAATATAAAAACCGAACCATAACGAAAAAAACGAACGCCCAAAAAAAAA (SEQ ID NO:388) | 17 | 7536 9074 | 7536 9194 | - | SEPT | 657301 | 79.17 |
| 657301_29427294_SEPT.1 _5_Methyl | CAAATATAAACGAATATAAAAACCGAACCATAACGAAAAAAACGAACGCCCAAAAAAAAAAAAATTC CTCCCCTTCCCCGAACGCAAAATCCTTCTACAAAAAACTATATTTAAACAACC (SEQ ID NO:389) | 17 | 7536 9014 | 7536 9134 | - | SEPT | 657301 | 69.17 |
| 657301_29427294_SEPT.1 _6_Methyl | AAAATTCCTCCCCTTCCCCGAACGCAAAATCCTTCTACAAAAAACTATATTTAAACAACCAAACAAAATT CTCTATCACCGCCGCCGCGCGCTCTACGCCTACAAAAATTAAACGACAAC (SEQ ID NO:390) | 17 | 7536 8954 | 7536 9074 | - | SEPT | 657301 | 63.33 |
| 657301_29427294_SEPT.1 _7_Methyl | AAACAAAATTCTCTATCACCGCCGCCGCGCGCTCTACGCCTACAAAAATTAAACGACAACGCACGCGA CTAACAAACAAAATCCCGACCTATAAACTCAAAAACCGAAAAACGCAAAACG (SEQ ID NO:391) | 17 | 7536 8894 | 7536 9014 | - | SEPT | 657301 | 69.17 |
| 657301_29427294_SEPT.1 _8_Methyl | ACACGCGACTAACAAACAAAATCCCGACCTATAAACTCAAAAACCGAAAAACGCAAAACGAACATAC GAATCCCATAACACCAACGAAAAACCACCAACTCCCACCCACCGAAAACACCC (SEQ ID NO:392) | 17 | 7536 8834 | 7536 8954 | - | SEPT | 657301 | 71.67 |
| 657301_29427294_SEPT.1 _9_Methyl | AACATACGAATCCCATAACACCAACGAAAAACCACCAACTCCCACCCACCGAAAACACCCCTACAACTA ACACCCCCACCACGACCACCGCCCCGAAAACCACCGCTACACCCTCCTAAA (SEQ ID NO:393) | 17 | 7536 8774 | 7536 8894 | - | SEPT | 657301 | 70.83 |
| 657301_29427294_SEPT.1 _10_Methyl | CTACAACTAACACCCCCACCACGACCACCGCCCCGAAAACCACCGCTACACCCTCCTAAATAAACCCTA AAAAAAACACTATCCGAAAAAAAAAACACAAAACGAAAATAAAACACGACT (SEQ ID NO:394) | 17 | 7536 8714 | 7536 8834 | - | SEPT | 657301 | 70.83 |
| 657301_29427294_SEPT.1 _11_Methyl | TAAACCCTAAAAAAAACACTATCCGAAAAAAAAAACACAAAACGAAAATAAAACACGACTATCAAAAA AACCAAAAACGAACGACGCCTCCCTTCCTCCACAATAAAAACCTCCTAAAAA (SEQ ID NO:395) | 17 | 7536 8654 | 7536 8774 | - | SEPT | 657301 | 65 |
| 657301_29427294_SEPT.1 _12_Methyl | ATCAAAAAAACCAAAAACGAACGACGCCTCCCTTCCTCCACAATAAAAACCTCCTAAAAACAAAAAAT AAACCTTATCAAAAACAAACGTTTCCGAAAAAAAATATAAAAAAAAAAACTA (SEQ ID NO:396) | 17 | 7536 8594 | 7536 8714 | - | SEPT | 657301 | 57.5 |
| 657301_29427295_SPINT 2.1_1_Methyl | ACCATACCAAACTAATCCCTAAATCACCAAACAAAAACGACCCAAAATAATAATAACGAAAACTTAAC ACAAAATAATAAAAACTATAATAAAAAAAACCAAACTTAAAAATCAAAAACT (SEQ ID NO:397) | 19 | 3875 4644 | 3875 4764 | - | SPINT2 | 657301 | 54.17 |
| 657301_29427295_SPINT 2.1_2_Methyl | CAAACAAAAACGACCCAAAATAATAATAACGAAAACTTAACACAAAATAATAAAAACTATAATAAAAA AAACCAAACTTAAAAATCAAAAACTAAATCGCTAAAAAACCAAAACATAATA (SEQ ID NO:398) | 19 | 3875 4617 | 3875 4737 | - | SPINT2 | 657301 | 52.5 |
| 657301_29427296_SPINT 2.2_1_Methyl | ACGAAACGAAACGAAACGTCTACCCGCTAAACACCTACCACCCTTCCCTTAAACTTAATAACGACGTCG AAAAAAAACCTTCGCTCTAAAACTAACCTCAAAATAAAAAAAAAAAACGAC (SEQ ID NO:399) | 19 | 3875 4892 | 3875 5012 | - | SPINT2 | 657301 | 74.17 |
| 657301_29427296_SPINT 2.2__2__Methyl | ACGACGTCGAAAAAAAACCTTCGCTCTAAAACTAACCTCAAAATAAAAAAAAAAAACGACAAACACGC GTTTCTTAAAAACCTATAATTTTCTACCCGCCTCGCCGCAACTTTCCTATAA (SEQ ID NO:400) | 19 | 3875 4832 | 3875 4952 | - | SPINT2 | 657301 | 65 |
| 657301_29427296_SPINT 2.2_3_Methyl | AAACACGCGTTTCTTAAAAACCTATAATTTTCTACCCGCCTCGCCGCAACTTTCCTATAAATTCCTTCCTA AATTTCAAAATTCACTAAACTCATACTCCAAAACTTAATACTAAAAAC (SEQ ID NO:401) | 19 | 3875 4773 | 3875 4892 | - | SPINT2 | 657301 | 53.78 |
| 657301_29427297_APC.1 _1_Methyl | CCCTATACCAAAAAAAAACCATCGATTTAAAACAATACGAAAAAAAACCACCTTCCCCCCTCCCCCCGC AACAAACCTAACCATAATAACTCCAACACCTACCCCATTTCCGAATCCGAC (SEQ ID NO:402) | 5 | 1.12E +08 | 1.12E +08 | - | APC | 657301 | 63.33 |
| 657301_29427297_APC.1 _2_Methyl | TCCCCCCGCAACAAACCTAACCATAATAACTCCAACACCTACCCCATTTCCGAATCCGACAACACCTCCA TTCTATCTCCAATAACACCCTAACGAACTACACCAATACAACCACATATC (SEQ ID NO:403) | 5 | 1.12E +08 | 1.12E +08 | - | APC | 657301 | 63.33 |
| 657301_29427297_APC.1 _3_Methyl | AACACCTCCATTCTATCTCCAATAACACCCTAACGAACTACACCAATACAACCACATATCGATCACGTAC GCCCACACCCAACCAATCGACGAACTCCCGACGAAAATAAAAAACGCCCT (SEQ ID NO:404) | 5 | 1.12E +08 | 1.12E +08 | -- | APC | 657301 | 65.83 |
| 657301_29427297_APC.1 _4_Methyl | AATCACGTACGCCCACACCCAACCAATCGACGAACTCCCGACGAAAATAAAAAACGCCCTAATCCGCA TCCAACGAATTACACAACTACTTCTCTCTCCGCTTCCCGACCCGCACTCCGC (SEQ ID NO:405) | 5 | 1.12E +08 | 1.12E +08 | - | APC | 657301 | 69.17 |
| 657301_29427297_APC.1 _5_Methyl | AATCCGCATCCAACGAATTACACAACTACTTCTCTCTCCGCTTCCCGACCCGCACTCCGCAATAAAACAC AAAACCCCGCCCAACCGCACAACCTACCTAACCCTAACCCCGTACCCCTC (SEQ ID NO:406) | 5 | 1.12E +08 | 1.12E +08 | - | APC | 657301 | 69.17 |
| 657301_29427297_APC.1 _6_Methyl | AATAAAACACAAAACCCCGCCCAACCGCACAACCTACCTAACCCTAACCCCGTACCCCTCGAAAATATA CCCTCGCGACCCCAATCCCCAACAAACAAAAAAATTAATAACAATTAACAC (SEQ ID NO:407) | 5 | 1.12E +08 | 1.12E +08 | - | APC | 657301 | 68.33 |
| 657301_29427297_APC.1 _7_Methyl | AAAAATATACCCTCGCGACCCCAATCCCCAACAAACAAAAAAATTAATAACAATTAACACGCATAATAA AAAATCGAAAAAAATCCGCAAAACAAAAAAATTAACACAAAATACCGAAAA (SEQ ID NO:408) | 5 | 1.12E +08 | 1.12E +08 | - | APC | 657301 | 60.83 |
| 657301_29427297_APC.1 _8_Methyl | ACATAATAAAAAATCGAAAAAAATCCGCAAAACAAAAAAATTAACACAAAATACCGAAAATAAAAAA AAAAATAATAAATTACATCTCGATTACTATAATTTTTACAACACAAAAAAACA (SEQ ID NO:409) | 5 | 1.12E +08 | 1.12E +08 | - | APC | 657301 | 48.33 |
| 657301_29427297_APC.1 _9_Methyl | TAAAAAAAAAAATAATAAATTACATCTCGATTACTATAATTTTTACAACACAAAAAAACAATAATATTA AATAAATTACAATAAATAATTTTACATTACAAATCACTAAATTATCAAAAT (SEQ ID NO:410) | 5 | 1.12E +08 | 1.12E +08 | - | APC | 657301 | 30.83 |
| 657301_29427297_APC.1 _10_Methyl | ATAATATTAAATAAATTACAATAAATAATTTTACATTACAAATCACTAAATTATCAAAATAATAACTTAT TAAATAAATCACTCGAATTATATTTAAATTAAAAATAATTACCAAAAAAA (SEQ ID NO:411) | 5 | 1.12E +08 | 1.12E +08 | - | APC | 657301 | 25 |
| 657301_29427298_HOXA 1_1_Methyl | AACCCTATAAAACCCACGCGAAAACGCGAAAACTCCTAAATCTCAAAACGCCGACAAATAACCTACAA CGCACCGCCCGACCCCCGCGCTAACCACGTCCGCCAACGTCATCTCTCTCCC (SEQ ID NO:412) | 7 | 2713 6615 | 2713 6735 | - | HOXA1 | 657301 | 77.5 |
| 657301_29427298_HOXA 1_2_Methyl | ACCTACAACGCACCGCCCGACCCCCGCGCTAACCACGTCCGCCAACGTCATCTCTCTCCCCCGCCTCCT ACCCCTAAAAATCCGACGATCGAATAACTCCGCATCCCGAAAAACTCCCGA (SEQ ID NO:413) | 7 | 2713 6555 | 2713 6675 | - | HOXA1 | 657301 | 73.33 |
| 657301_29427298_HOXA 1_3_Methyl | CCGCCTCCTACCCCTAAAAATCCGACGATCGAATAACTCCGCATCCCGAAAAACTCCCGATACAAAACT AAATAAAAAAAAAAAAAAAACGAAAAACGACGAAAAAAAAAAAAATAAAAAA (SEQ ID NO:414) | 7 | 2713 6495 | 2713 6615 | - | HOXA1 | 657301 | 62.5 |
| 657301_29427298_HOXA 1_4_Methyl | TACAAAACTAAATAAAAAAAAAAAAAAAACGAAAAACGACGAAAAAAAAAAAAATAAAAAAAAAAAA CCCGACCGCCCCCGACCTTTTCCGACGAACTCTAAATTTCCAAAACTCATCCGC (SEQ ID NO:415) | 7 | 2713 6435 | 2713 6555 | - | HOXA1 | 657301 | 63.33 |
| 657301_29427298_HOXA 1_5_Methyl | AAAAAACCCGACCGCCCCCGACCTTTTCCGACGAACTCTAAATTTCCAAAACTCATCCGCCCCTCCAAA TCGAATTACAAAAACTCCTCATTACCATACTAATAACGAAAAAAACTATAA (SEQ ID NO:416) | 7 | 2713 6375 | 2713 6495 | - | HOXA1 | 657301 | 60.83 |
| 657301_29427298_HOXA 1_6_Methyl | CCCTCCAAATCGAATTACAAAAACTCCTCATTACCATACTAATAACGAAAAAAACTATAAAAAAAAAT TTCGAAAACCCTCCTAAAAAAAAAAAAATTTCTTTTCTTACCCTCGTCTCC (SEQ ID NO:417) | 7 | 2713 6315 | 2713 6435 | - | HOXA1 | 657301 | 52.5 |
| 657301_29427298_HOXA 1_7_Methyl | AAAAAAAATTTCGAAAACCCTCCTAAAAAAAAAAAAATTTCTTTTCTTACCCTCGTCTCCTTCACGCCCA CCCGAATTCCTCCCAACCAACCCTCTACGCTCTTCCCCCTCCATTCCGAA (SEQ ID NO:418) | 7 | 2713 6255 | 2713 6375 | - | HOXA1 | 657301 | 60 |
| 657301_29427298_HOXA 1_8_Methyl | TTCACGCCCACCCGAATTCCTCCCAACCAACCCTCTACGCTCTTCCCCCTCCATTCCGAACTTAAAAAAC CGATAACCCAACCTCGCCCTCCACCACGATTCTATACGCTCCTCACAACC (SEQ ID NO:419) | 7 | 2713 6195 | 2713 6315 | - | HOXA1 | 657301 | 66.67 |
| 657301_29427298_HOXA 1_9_Methyl | CTTAAAAAACCGATAACCCAACCTCGCCCTCCACCACGATTCTATACGCTCCTCACAACCCCACGATCTC CCAACCCAACCTCAAAACAAAAAAATAACCTAAAAACCCGAAACGCGAT (SEQ ID NO:420) | 7 | 2713 6135 | 2713 6255 | - | HOXA1 | 657301 | 69.17 |
| 657301_29427298_HOXA 1_10_Methyl | CCACGATCTCCCAACCCAACCTCAAAACAAAAAAAATAACCTAAAAACCCGAAACGCGATATCAACCG AATTCCCCGCCTTCCGACTAAACGCGCTACGAACTAAACCGACGTACTTACG (SEQ ID NO:421) | 7 | 2713 6075 | 2713 6195 | - | HOXA1 | 657301 | 74.17 |
| 657301_29427298_HOXA 1_11_Methyl | ATCAACCGAATTCCCCGCCTTCCGACTAAACGCGCTACGAACTAAACCGACGTACTTACGACCACCCGA CGAACTCTAACCCACTAATTCCCGCCCCGAAAAAACCGAAAACCCTCTTCC (SEQ ID NO:422) | 7 | 2713 6015 | 2713 6135 | - | HOXA1 | 657301 | 75 |
| 657301_29427298_HOXA 1_12_Methyl | ACCACCCGACGAACTCTAACCCACTAATTCCCGCCCCGAAAAAACCGAAAACCCTCTTCCCTTCTCACCT CTCGCCAATTCATCTTTCATTAAACGATAAAAAAAAAACGAAAACGAAAA (SEQ ID NO:423) | 7 | 2713 5955 | 2713 6075 | - | HOXA1 | 657301 | 68.33 |
| 657301_29427298_HOXA 1_13_Methyl | CTTCTCACCTCTCGCCAATTCATCTTTCATTAAACGATAAAAAAAAAACGAAAACGAAAAACGCCTCCC AACCCCCACCTCCCGAAAACCTAACGAAACGAACAACGAAATCGAACGCGC (SEQ ID NO:424) | 7 | 2713 5895 | 2713 6015 | - | HOXA1 | 657301 | 73.33 |
| 657301_29427298_HOXA 1_14_Methyl | ACGCCTCCCAACCCCCACCTCCCGAAAACCTAACGAAACGAACAACGAAATCGAACGCGCGCAAAACG CAACTTTTACTCTTCTTCGCTCCAACACTCCAAATCGACCTTTACAATCGCC (SEQ ID NO:425) | 7 | 2713 5835 | 2713 5955 | - | HOXA1 | 657301 | 70 |
| 657301_29427298_HOXA 1_15_Methyl | ACAAAACGCAACTTTTACTCTTCTTCGCTCCAACACTCCAAATCGACCTTTACAATCGCCGCAACAACTA CCGCCGCCTAAACTACCTAAAAAAACGACTACGCGTCACCTAAACGACGA (SEQ ID NO:426) | 7 | 2713 5775 | 2713 5895 | - | HOXA1 | 657301 | 66.67 |
| 657301_29427298_HOXA 1_16_Methyl | ACAACAACTACCGCCGCCTAAACTACCTAAAAAAACGACTACGCGTCACCTAAACGACGAAACGCCGA AAATTTAAATACCACTAAAACGATAATCCATCACTACGAAAACCGACAAACT (SEQ ID NO:427) | 7 | 2713 5715 | 2713 5835 | - | HOXA1 | 657301 | 65 |
| 657301_29427298_HOXA 1_17_Methyl | AACGCCGAAAATTTAAATACCACTAAAACGATAATCCATCACTACGAAAACCGACAAACTTTTACAAAA AACTCAACCATTAACTAACACCGTCACGTACCCCTCCTCCAACGTCCTCCG (SEQ ID NO:428) | 7 | 2713 5655 | 2713 5775 | - | HOXA1 | 657301 | 59.17 |
| 657301_29427298_HOXA 1_18_Methyl | TTTACAAAAAACTCAACCATTAACTAACACCGTCACGTACCCCTCCTCCAACGTCCTCCGCCCTCCCGCC CCCCCTCTTACGCACTATACATTCATATCATTTTTCTTCTCCGACCCCAT (SEQ ID NO:429) | 7 | 2713 5595 | 2713 5715 | - | HOXA1 | 657301 | 60.83 |
| 657301_29427298_HOXA 1_19_Methyl | CCCTCCCGCCCCCCCTCTTACGCACTATACATTCATATCATTTTTCTTCTCCGACCCCATAAAAAAAATAA AAAAATTAACACAATCACGCCGAACTTCGCAAAACCAAATCACTCAATA (SEQ ID NO:430) | 7 | 2713 5535 | 2713 5655 | - | HOXA1 | 657301 | 58.33 |
| 657301_29427298_HOXA 1_20_Methyl | AAAAAAAATAAAAAAATTAACACAATCACGCCGAACTTCGCAAAACCAAATCACTCAATAACAAATAA ACAATACAAAAATAAACTCCTTCCTAAAATACCCCATACTTAACAATAACGA (SEQ ID NO:431) | 7 | 2713 5475 | 2713 5595 | - | HOXA1 | 657301 | 53.33 |
| 657301_29427298_HOXA 1_21_Methyl | ACAAATAAACAATACAAAAATAAACTCCTTCCTAAAATACCCCATACTTAACAATAACGACTCGAAAAC CTACTCAACCCGAACCTACCCCTCGAACCATAAAATTACAACTTTCCAATC (SEQ ID NO:432) | 7 | 2713 5415 | 2713 5535 | - | HOXA1 | 657301 | 53.33 |

**TABLE 4.**

| CpG Site Identifier | Genomic Location (GRCh37/hg19 Human Genome Assembly) |
|---|---|
| cg15607538 | chr12:133,484,854 |
| cg08572734 | chr12:133,485,299 |
| cg00577935 | chr5:112,073,349 |
| cg03667968 | chr5:112,073,439 |
| cg08571859 | chr5:112,073,351 |
| cg02659086 | chr11:67,350,977 |
| cg04673590 | chr7:27,136,192 |
| cg09420439 | chr7:27,136,425 |
| cg26744375 | chr7:27, 135, 724 |
| cg08465862 | chr10:3,109,459 |
| cg14250130 | chr10:3,109,361 |
| cg00922376 | chr1:14,026,585 |
| cg05346841 | chr1:14,026,591 |
| cg26421310 | chr1:25,257,059 |
| cg13629563 | chr1:25,256,949 |
| cg06848185 | chr17:75,368,903 |
| cg17300544 | chr17:75,369,091 |
| cg22522066 | chr19:38,754,727 |
| cg24166864 | chr12:65,515,291 |
| cg26397188 | chr12:65,515,277 |

## Claims

1. A method of diagnosing hepatocellular carcinoma (HCC) in a patient, the method comprising:
detecting methylation at one or more CpG sites in each of the following genes from circulating free DNA (cfDNA) in a sample: SPINT2, RUNX3, PRDM2, APC, GSTP1, WIF1, SEPT9, HOXA1, PFKP, and AK055957, wherein increased frequency of methylation at the one or more CpG sites in the genes consisting of SPINT2, RUNX3, PRDM2, APC, GSTP1, WIF1, SEPT9, HOXA1, PFKP, and AK055957 in the cfDNA sample from the patient compared to reference value ranges for frequency of methylation at the one or more CpG sites in a control cfDNA sample indicates that the patient has a positive diagnosis for the HCC, wherein the one or more CpG sites are selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof.

2. The method of claim 1, wherein said detecting methylation comprises measuring frequency of methylation at the cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188 CpG sites in the cfDNA.

3. The method of claim 1 or claim 2, wherein the reference value ranges for frequency of methylation at the one or more CpG sites are obtained from cfDNA from one or more blood samples from one or more control subjects not having HCC.

4. The method of any one of claims 1 to 3, further comprising calculating an HCC risk score based on the methylation frequency at the CpG sites in the SPINT2, RUNX3, PRDM2, APC, GSTP1, WIF1, SEPT9, HOXA1, PFKP, and AK055957 genes of the cfDNA using one or more algorithms.

5. The method of any one of claims 1 to 4, wherein said positive diagnosis enables the patient to be selected for treatment comprising surgical resection of an HCC tumor, radiofrequency ablation (RFA) of an HCC tumor, cryoablation of an HCC tumor, percutaneous injection of an HCC tumor with ethanol or acetic acid, transcatheter arterial chemoembolization (TACE), selective internal radiation therapy (SIRT), liver transplantation, high intensity focused ultrasound, external beam therapy, portal vein embolization, radionuclide therapy, chemotherapy, targeted therapy, immunotherapy, or biologic therapy.

6. The method of any one of claims 1 to 5, wherein said detecting the methylation of CpG sites in the cfDNA comprises performing methylation-sensitive arbitrarily-primed polymerase chain reaction (MS AP-PCR), methylation-sensitive single nucleotide primer extension (Ms-SNuPE), methylation-specific PCR (MSP), methylation-sensitive DNA restriction enzyme analysis, restriction enzyme-based sequencing, restriction enzyme-based microarray analysis, combined bisulfite restriction analysis (COBRA), methylated CpG island amplification (MCA), methylated CpG island amplification and microarray (MCAM), HpaII tiny fragment enrichment by ligation-mediated PCR (HELP), bisulfite sequencing, bisulfite microarray analysis, methylation-specific pyrosequencing, HELP-sequencing (HELP-seq), TET-assisted pyridine borane sequencing (TAPS), Glal hydrolysis and ligation adapter dependent PCR (GLAD-PCR), methylated DNA immunoprecipitation-sequencing (MeDIP-Seq), or methylated DNA immunoprecipitation-microarray analysis (MeDIP-chip), Southern blotting with methyl-sensitive restriction enzymes, or methylation-specific giant magnetoresistive sensor-based microarray analysis.

7. The method of any one of claims 1 to 6, wherein said detecting the methylation of CpG sites in the cfDNA comprises using at least one probe comprising a sequence selected from the group consisting of SEQ ID NOS:1-432.

8. The method of any one of claims 1 to 7, further comprising measuring blood levels of alpha-fetoprotein (AFP), wherein detection of increased blood levels of AFP in combination with increased frequency of methylation at the one or more CpG sites in the genes consisting of SPINT2, RUNX3, PRDM2, APC, GSTP1, WIF1, SEPT9, HOXA1, PFKP, and AK055957 compared to reference value ranges for blood levels of AFP and frequency of methylation at the one or more CpG sites in the genes consisting of SPINT2, RUNX3, PRDM2, APC, GSTP1, WIF1, SEPT9, HOXA1, PFKP, and AK055957 for a control subject indicate that the patient has a positive diagnosis for the HCC.

9. A method of monitoring hepatocellular carcinoma (HCC) in a patient, the method comprising:
detecting methylation at one or more CpG sites in each of the following genes from circulating free DNA (cfDNA) in a first blood sample at a first time point and a second blood sample at a second time point: SPINT2, RUNX3, PRDM2, APC, GSTP1, WIF1, SEPT9, HOXA1, PFKP, and AK055957, wherein detection of increased frequency of methylation of the CpG sites in the genes consisting of SPINT2, RUNX3, PRDM2, APC, GSTP1, WIF1, SEPT9, HOXA1, PFKP, and AK055957 in the cfDNA of the second blood sample compared to the cfDNA of the first blood sample indicate that the HCC is progressing, and detection of decreased frequency of methylation of the CpG sites in the genes consisting of SPINT2, RUNX3, PRDM2, APC, GSTP1, WIF1, SEPT9, HOXA1, PFKP, and AK055957 in the cfDNA of the second blood sample compared to the cfDNA of the first blood sample indicate that the HCC is not progressing, wherein the one or more CpG sites are selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof.

10. A method of monitoring for a recurrence of hepatocellular carcinoma (HCC) in a patient, the method comprising:
a) detecting methylation at one or more CpG sites in promoter regions of biomarker genes in circulating free DNA (cfDNA) from a first cfDNA sample after treatment for a previous occurrence at a first time point when the patient is characterized as cancer-free from imaging or other diagnostic modalities, wherein the biomarker genes comprise each of AK055957, APC, GSTP1, HOXA1, PFKP, PRDM2, RUNX3, SEPTIN9, SPINT2, and WIF1;
b) detecting methylation at the one or more CpG sites in the promoter regions of the biomarker genes in cfDNA from a second cfDNA sample at a second time point during a period of monitoring for the recurrence, wherein the biomarker genes comprise each of AK055957, APC, GSTP1, HOXA1, PFKP, PRDM2, RUNX3, SEPTIN9, SPINT2, and WIF1, wherein increased frequency of methylation at the one or more CpG sites in the promoter regions of the biomarker genes comprising each of AK055957, APC, GSTP1, HOXA1, PFKP, PRDM2, RUNX3, SEPTIN9, SPINT2, and WIF1 in the cfDNA of the second cfDNA sample compared to the cfDNA of the first cfDNA sample indicates that the HCC has recurred; and
c) repeating step b subsequently during the period of monitoring for the recurrence, and
wherein the one or more CpG sites are selected from cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, and cg26397188, and CpG sites located within 200 nucleotides thereof.

11. A kit comprising primers and/or probes selected from the group consisting of:
(i) PCR primers that selectively amplify DNA regions that contain CpG dinucleotides;
(ii) methylation-specific primers;
and/or
(iii) at least one probe comprising a sequence selected from the group consisting of SEQ ID NOS:1-432 for detecting methylation of CpG sites in each of SPINT2, RUNX3, PRDM2, APC, GSTP1, WIFI, SEPT9, HOXA1, PFKP, and AK055957 genes in cfDNA.

12. The kit of claim 11, further comprising a bisulfite reagent, reagents for measuring AFP, instructions for using the kit for diagnosis of hepatocellular carcinoma (HCC), detecting recurrence of HCC, or monitoring treatment of HCC, or any combination thereof.

## Patentansprüche

1. Verfahren zur Diagnose eines hepatozellulären Karzinoms (HCC) bei einem Patienten, wobei das Verfahren Folgendes umfasst:
Nachweisen von Methylierung an einer oder mehreren CpG-Stellen in jedem der folgenden Gene aus cfDNA (circulating free DNA) in einer Probe: SPINT2, RUNX3, PRDM2, APC, GSTP1, WIF1, SEPT9, HOXA1, PFKP und AK055957, wobei eine erhöhte Methylierungshäufigkeit an der einen oder den mehreren CpG-Stellen in den Genen bestehend aus SPINT2, RUNX3, PRDM2, APC, GSTP1, WIF1, SEPT9, HOXA1, PFKP und AK055957 in der cfDNA-Probe von dem Patienten im Vergleich zu Referenzwertbereichen für die Methylierungshäufigkeit an der einen oder den mehreren CpG-Stellen in einer cfDNA-Kontrollprobe anzeigt, dass bei dem Patienten eine positive Diagnose für das HCC vorliegt,
wobei die eine oder die mehreren CpG-Stellen aus cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864 und cg26397188 sowie weniger als jeweils 200 Nukleotide davon entfernt liegenden CpG-Stellen ausgewählt sind.

2. Verfahren nach Anspruch 1, wobei das Nachweisen von Methylierung Messen der Methylierungshäufigkeit an den CpG-Stellen cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864 und cg26397188 in der cfDNA umfasst.

3. Verfahren nach Anspruch 1 oder Anspruch 2, wobei die Referenzwertbereiche für die Methylierungshäufigkeit an der einen oder den mehreren CpG-Stellen aus cfDNA aus einer oder mehreren Blutproben von einer oder mehreren Kontrollpersonen ohne HCC erhalten werden.

4. Verfahren nach einem der Ansprüche 1 bis 3, ferner umfassend Berechnen einer HCC-Risikobewertung anhand der Methylierungshäufigkeit an den CpG-Stellen in den Genen SPINT2, RUNX3, PRDM2, APC, GSTP1, WIF1, SEPT9, HOXA1, PFKP und AK055957 der cfDNA unter Verwendung eines oder mehrerer Algorithmen.

5. Verfahren nach einem der Ansprüche 1 bis 4, wobei die positive Diagnose die Auswahl des Patienten für eine Behandlung ermöglicht, die chirurgische Resektion eines HCC-Tumors, Radiofrequenzablation (RFA) eines HCC-Tumors, Kryoablation eines HCC-Tumors, perkutane Injektion eines HCC-Tumors mit Ethanol oder Essigsäure, transarterielle Chemoembolisation (TACE), selektive interne Radiotherapie (SIRT), Lebertransplantation, hochintensiven fokussierten Ultraschall, externe Strahlentherapie, Pfortaderembolisation, Radionuklidtherapie, Chemotherapie, zielgerichtete Therapie, Immuntherapie oder biologische Therapie umfasst.

6. Verfahren nach einem der Ansprüche 1 bis 5, wobei das Nachweisen der Methylierung von CpG-Stellen in der cfDNA Durchführen von methylierungsempfindlicher Polymerase-Kettenreaktion mit willkürlichen Primern (methylation-sensitive arbitrarily-primed polymerase chain reaction, MS AP-PCR), methylierungsempfindlicher Einzelnukleotid-Primerverlängerung (methylation-sensitive single nucleotide primer extension, MS-SNuPE), methylierungsspezifischer PCR (MSP), methylierungsempfindlicher DNA-Restriktionsenzymanalyse, Sequenzierung auf Restriktionsenzymbasis, Microarray-Analyse auf Restriktionsenzymbasis, kombinierter Hydrogensulfit-Restriktionsanalyse (combined bisulfite restriction analysis, COBRA), Amplifikation methylierter CpG-Inseln (MCA), Amplifikation methylierter CpG-Inseln und Microarray (MCAM), Anreicherung kleiner HpaII-Fragmente durch ligationsvermittelte PCR (HELP), Hydrogensulfit-Sequenzierung, Hydrogensulfit-Microarray-Analyse, methylierungsspezifischer Pyrosequenzierung, HELP-Sequenzierung (HELP-seq), TET-unterstützter Pyridinboran-Sequenzierung (TAPS), Glal-Hydrolyse und ligationsadapterabhängiger PCR (GLAD-PCR), Methylierte-DNA-Immunpräzipitation-Sequenzierung (MeDIP-Seq) oder Methylierte-DNA-Immunpräzipitation-Mikroarray-Analyse (MeDIP-Chip), Southern Blot mit methylempfindlichen Restriktionsenzymen oder methylierungsspezifischer Microarray-Analyse auf GMR(Giant Magnetoresistive)-Sensor-Basis umfasst.

7. Verfahren nach einem der Ansprüche 1 bis 6, wobei das Nachweisen der Methylierung von CpG-Stellen in der cfDNA Verwenden mindestens einer Sonde umfasst, die eine Sequenz umfasst, die aus der Gruppe bestehend aus SEQ ID NO:1-432 ausgewählt ist.

8. Verfahren nach einem der Ansprüche 1 bis 7, ferner umfassend Messen der Blutspiegel von Alpha-Fetoprotein (AFP), wobei ein Nachweis erhöhter AFP-Blutspiegel in Kombination mit erhöhter Methylierungshäufigkeit an der einen oder den mehreren CpG-Stellen in den Genen bestehend aus SPINT2, RUNX3, PRDM2, APC, GSTP1, WIF1, SEPT9, HOXA1, PFKP und AK055957 im Vergleich zu Referenzwertbereichen für AFP-Blutspiegel bzw. Methylierungshäufigkeit an der einen oder den mehreren CpG-Stellen in den Genen bestehend aus SPINT2, RUNX3, PRDM2, APC, GSTP1, WIF1, SEPT9, HOXA1, PFKP und AK055957 bei einer Kontrollperson anzeigt, dass bei dem Patienten eine positive Diagnose für das HCC vorliegt.

9. Verfahren zur Überwachung eines hepatozellulären Karzinoms (HCC) bei einem Patienten, wobei das Verfahren Folgendes umfasst:
Nachweisen von Methylierung an einer oder mehreren CpG-Stellen in jedem der folgenden Gene aus cfDNA (circulating free DNA) in einer ersten Blutprobe zu einem ersten Zeitpunkt und einer zweiten Blutprobe zu einem zweiten Zeitpunkt: SPINT2, RUNX3, PRDM2, APC, GSTP1, WIF1, SEPT9, HOXA1, PFKP UND AK055957, wobei der Nachweis einer erhöhten Methylierungshäufigkeit bei den CpG-Stellen in den Genen bestehend aus SPINT2, RUNX3, PRDM2, APC, GSTP1, WIF1, SEPT9, HOXA1, PFKP und AK055957 in der cfDNA der zweiten Blutprobe im Vergleich zur cfDNA der ersten Blutprobe anzeigt, dass das HCC fortschreitet, und der Nachweis einer verringerten Methylierungshäufigkeit bei den CpG-Stellen in den Genen bestehend aus SPINT2, RUNX3, PRDM2, APC, GSTP1, WIF1, SEPT9, HOXA1, PFKP und AK055957 in der cfDNA der zweiten Blutprobe im Vergleich zur cfDNA der ersten Blutprobe anzeigt, dass das HCC nicht fortschreitet,
wobei die eine oder die mehreren CpG-Stellen aus cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864 und cg26397188 sowie weniger als jeweils 200 Nukleotide davon entfernt liegenden CpG-Stellen ausgewählt sind.

10. Verfahren zur Überwachung auf ein Rezidiv eines hepatozellulären Karzinoms (HCC) bei einem Patienten, wobei das Verfahren Folgendes umfasst:
a) Nachweisen von Methylierung an einer oder mehreren CpG-Stellen in Promotorregionen von Biomarkergenen in cfDNA (circulating free DNA) aus einer ersten cfDNA-Probe nach Behandlung eines vorherigen Auftretens zu einem ersten Zeitpunkt, an dem der Patient anhand bildgebender oder anderer diagnostischer Modalitäten als krebsfrei charakterisiert ist, wobei die Biomarkergene jeweils AK055957, APC, GSTP1, HOXA1, PFKP, PRDM2, RUNX3, SEPTIN9, SPINT2 und WIF1 umfassen;
b) Nachweisen von Methylierung an der einen oder den mehreren CpG-Stellen in den Promotorregionen der Biomarkergene in cfDNA aus einer zweiten cfDNA-Probe zu einem zweiten Zeitpunkt während einer Periode der Rezidivüberwachung, wobei die Biomarkergene jeweils AK055957, APC, GSTP1, HOXA1, PFKP, PRDM2, RUNX3, SEPTIN9, SPINT2 und WIF1 umfassen, wobei eine erhöhte Methylierungshäufigkeit an der einen oder den mehreren CpG-Stellen in den Promotorregionen der Biomarkergene, die jeweils AK055957, APC, GSTP1, HOXA1, PFKP, PRDM2, RUNX3, SEPTIN9, SPINT2 und WIF1 umfassen, in der cfDNA der zweiten cfDNA-Probe im Vergleich zur cfDNA der ersten cfDNA-Probe anzeigt, dass das HCC rezidiviert ist; und
c) anschließendes Wiederholen von Schritt b während der Periode der Rezidivüberwachung, und
wobei die eine oder die mehreren CpG-Stellen aus cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864 und cg26397188 sowie weniger als jeweils 200 Nukleotide davon entfernt liegenden CpG-Stellen ausgewählt sind.

11. Kit, umfassend Primer und/oder Sonden, die ausgewählt sind aus der Gruppe bestehend aus:
(i) PCR-Primern, die DNA-Regionen, die CpG-Dinukleotide enthalten, selektiv amplifizieren;
(ii) methylierungsspezifischen Primern;
und/oder
(iii) mindestens einer Sonde, die eine Sequenz umfasst, die aus der Gruppe bestehend aus SEQ ID NO:1-432 ausgewählt ist,
zum Nachweisen der Methylierung von CpG-Stellen in jedem der Gene SPINT2, RUNX3, PRDM2, APC, GSTP1, WIF1, SEPT9, HOXA1, PFKP und AK055957 in cfDNA.

12. Kit nach Anspruch 11, ferner umfassend ein Hydrogensulfitreagenz, Reagenzien zur Messung von AFP, Anweisungen zur Verwendung des Kits bei der Diagnose von hepatozellulärem Karzinom (HCC), dem Nachweis eines HCC-Rezidivs oder der Überwachung der Behandlung von HCC oder einer beliebigen Kombination davon.

## Revendications

1. Procédé de diagnostic d'un carcinome hépatocellulaire (HCC) chez un patient, le procédé comprenant :
la détection d'une méthylation au niveau d'un ou plusieurs sites CpG dans chacun des gènes suivants à partir d'ADN libre circulant (ADNcf) dans un échantillon : SPINT2, RUNX3, PRDM2, APC, GSTP1, WIF1, SEPT9, HOXA1, PFKP, et AK055957 dans lequel la fréquence accrue de méthylation au niveau des un ou plusieurs sites CpG dans les gènes constitués par SPINT2, RUNX3, PRDM2, APC, GSTP1, WIF1, SEPT9, HOXA1, PFKP, et AK055957 dans l'échantillon d'ADNcf du patient comparé à des plages de valeurs de référence pour la fréquence de méthylation au niveau des un ou plusieurs sites CpG dans un échantillon d'ADNcf témoin indique que le patient a un diagnostic positif pour le HCC,
dans lequel le ou les sites CpG sont choisis parmi cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, et cg26397188, et des sites CpG situés à moins de 200 nucléotides de ceux-ci.

2. Procédé selon la revendication 1, dans lequel ladite détection d'une méthylation comprend la mesure de la fréquence de méthylation aux niveaux des sites CpG cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, et cg26397188 dans l'ADNcf.

3. Procédé selon la revendication 1 ou la revendication 2, dans lequel les plages de valeurs de référence pour la fréquence de méthylation au niveau des un ou plusieurs sites CpG sont obtenues à partir d'ADNcf provenant d'un ou plusieurs échantillons de sang provenant d'un ou plusieurs sujets témoins ne présentant pas de HCC.

4. Procédé selon l'une quelconque des revendications 1 à 3, comprenant en outre le calcul d'un score de risque d'HCC basé sur la fréquence de méthylation au niveau des sites CpG dans les gènes SPINT2, RUNX3, PRDM2, APC, GSTP1, WIF1, SEPT9, HOXA1, PFKP, et AK055957 de l'ADNcf en utilisant un ou plusieurs algorithmes.

5. Procédé selon l'une quelconque des revendications 1 à 4, dans lequel ledit diagnostic positif permet au patient d'être sélectionné pour un traitement comprenant une résection chirurgicale d'une tumeur HCC, une ablation par radiofréquence (RFA) d'une tumeur HCC, une cryoablation d'une tumeur HCC, une injection percutanée d'éthanol ou d'acide acétique dans une tumeur HCC, une chimioembolisation artérielle transcathéter (TACE), une radiothérapie interne sélective (SIRT), une transplantation hépatique, des ultrasons focalisés de haute intensité, une radiothérapie externe, une embolisation de la veine porte, une thérapie par radionucléides, une chimiothérapie, une thérapie ciblée, une immunothérapie ou une thérapie biologique.

6. Procédé selon l'une quelconque des revendications 1 à 5, dans lequel ladite détection d'une méthylation de sites CpG dans l'ADNcf comprend la réalisation d'une réaction en chaîne par polymérase à amorçage arbitraire sensible à la méthylation (MS AP-PCR), d'une extension d'amorce mononucléotidique sensible à la méthylation (Ms-SNuPE), d'une PCR spécifique de la méthylation (MSP), d'une analyse par enzyme de restriction de l'ADN sensible à la méthylation, d'un séquençage basé sur des enzymes de restriction, d'une analyse par microréseau basée sur des enzymes de restriction, d'une analyse combinée de restriction au bisulfite (COBRA), d'une amplification d'îlots CpG méthylés (MCA), d'une amplification d'îlots CpG méthylés et d'un microréseau (MCAM), d'un enrichissement de petits fragments HpaII par PCR médiée par ligation (HELP), d'un séquençage au bisulfite, d'une analyse par microréseau au bisulfite, d'un pyroséquençage spécifique de la méthylation, d'un séquençage HELP (HELP-seq), d'un séquençage au pyridine borane assisté par TET (TAPS), d'une PCR dépendante de l'hydrolyse de Glal et de l'adaptateur de ligation (GLAD-PCR), d'un séquençage par immunoprécipitation de l'ADN méthylé (MeDIP-Seq), ou d'une analyse par microréseau d'immunoprécipitation d'ADN méthylé (MeDIP-chip), d'un transfert de Southern avec des enzymes de restriction sensibles à la méthylation, ou d'une analyse par microréseau basée sur un capteur magnétorésistif géant spécifique de la méthylation.

7. Procédé selon l'une quelconque des revendications 1 à 6, dans lequel ladite détection de la méthylation de sites CpG dans l'ADNcf comprend l'utilisation d'au moins une sonde comprenant une séquence choisie dans le groupe constitué par les SEQ ID NO: 1-432.

8. Procédé selon l'une quelconque des revendications 1 à 7, comprenant en outre la mesure de taux sanguins d'alpha-foetoprotéine (AFP), dans lequel la détection de taux sanguins accrus d'AFP en combinaison avec une fréquence accrue de méthylation au niveau des un ou plusieurs sites CpG dans les gènes constitués par SPINT2, RUNX3, PRDM2, APC, GSTP1, WIF1, SEPT9, HOXA1, PFKP, et AK055957 comparés à des plages de valeurs de référence pour les taux sanguins d'AFP et la fréquence de méthylation au niveau des un ou plusieurs sites CpG dans les gènes constitués par SPINT2, RUNX3, PRDM2, APC, GSTP1, WIF1, SEPT9, HOXA1, PFKP, et AK055957 pour un sujet témoin indiquent que le patient a un diagnostic positif de HCC.

9. Procédé de surveillance d'un carcinome hépatocellulaire (HCC) chez un patient, le procédé comprenant :
la détection d'une méthylation au niveau d'un ou de plusieurs sites CpG dans chacun des gènes suivants à partir d'ADN circulant libre (ADNcf) dans un premier échantillon de sang à un premier instant et d'un deuxième échantillon de sang à un deuxième instant : SPINT2, RUNX3, PRDM2, APC, GSTP1, WIF1, SEPT9, HOXA1, PFKP, et AK055957 où la détection d'une fréquence accrue de méthylation des sites CpG dans les gènes constitués par SPINT2, RUNX3, PRDM2, APC, GSTP1, WIF1, SEPT9, HOXA1, PFKP, et AK055957 dans l'ADNcf du deuxième échantillon de sang par rapport à l'ADNcf du premier échantillon de sang indiquent que l'HCC progresse, et la détection d'une diminution de la fréquence de méthylation des sites CpG dans les gènes constitués par SPINT2, RUNX3, PRDM2, APC, GSTP1, WIF1, SEPT9, HOXA1, PFKP, et AK055957 dans l'ADNcf du deuxième échantillon de sang comparé à l'ADNcf du premier échantillon de sang indiquent que l'HCC ne progresse pas,
dans lequel le ou les sites CpG sont choisis parmi cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, et cg26397188, et des sites CpG situés à moins de 200 nucléotides de ceux-ci.

10. Procédé de surveillance d'une récurrence d'un carcinome hépatocellulaire (HCC) chez un patient, le procédé comprenant :
a) la détection d'une méthylation au niveau d'un ou plusieurs sites CpG dans des régions promotrices de gènes de biomarqueur dans de l'ADN libre circulant (ADNcf) à partir d'un premier échantillon d'ADNcf après traitement pour une occurrence précédente à un premier instant lorsque le patient est caractérisé comme étant exempt d'un cancer à partir d'une imagerie ou d'autres modalités de diagnostic, où les gènes de biomarqueur comprennent chacun parmi AK055957, APC, GSTP1, HOXA1, PFKP, PRDM2, RUNX3, SEPTIN9, SPINT2, et WIF1 ;
b) la détection d'une méthylation au niveau des un ou plusieurs sites CpG dans les régions promotrices des gènes de biomarqueur dans l'ADNcf à partir d'un deuxième échantillon d'ADNcf à un deuxième instant pendant une période de surveillance de la récurrence, les gènes de biomarqueur comprenant chacun parmi AK055957, APC, GSTP1, HOXA1, PFKP, PRDM2, RUNX3, SEPTIN9, SPINT2, et WIF1, où une fréquence augmentée de méthylation au niveau des un ou plusieurs sites CpG dans les régions promotrices des gènes de biomarqueur comprenant chacun parmi AK055957, APC, GSTP1, HOXA1, PFKP, PRDM2, RUNX3, SEPTIN9, SPINT2, et WIF1 dans l'ADNcf du deuxième échantillon d'ADNcf comparé à l'ADNcf du premier échantillon d'ADNcf indique que l'HCC a récidivé ; et
c) la répétition de l'étape b ultérieurement pendant la période de surveillance de la récurrence, et
dans lequel le ou les sites CpG sont choisis parmi cg15607538, cg08572734, cg00577935, cg03667968, cg08571859, cg02659086, cg04673590, cg09420439, cg26744375, cg08465862, cg14250130, cg00922376, cg05346841, cg26421310, cg13629563, cg06848185, cg17300544, cg22522066, cg24166864, et cg26397188, et des sites CpG situés à moins de 200 nucléotides de ceux-ci.

11. Kit comprenant des amorces et/ou des sondes choisies dans le groupe constitué par :
(i) des amorces de PCR qui amplifient sélectivement des régions d'ADN qui contiennent des dinucléotides CpG ;
(ii) des amorces spécifiques d'une méthylation ;
et/ou
(iii) au moins une sonde comprenant une séquence choisie dans le groupe constitué par les SEQ ID NO: 1-432
pour la détection d'une méthylation de sites CpG dans chacun des gènes SPINT2, RUNX3, PRDM2, APC, GSTP1, WIF1, SEPT9, HOXA1, PFKP, et AK055957 dans l'ADNcf.

12. Kit selon la revendication 11, comprenant en outre un réactif de bisulfite, des réactifs pour mesurer l'AFP, des instructions pour l'utilisation du kit pour le diagnostic d'un carcinome hépatocellulaire (HCC), la détection de la récurrence d'HCC, ou la surveillance du traitement de l'HCC, ou toute combinaison de ceux-ci.
